(19) Europäisches Patentamt
European Patent Office
Office européen des brevets

(11) Publication number: 0 273 426 B1

(12) # EUROPEAN PATENT SPECIFICATION

(45) Date of publication of patent specification: **17.03.93**

(51) Int. Cl.5: **C07C 211/48**, C07C 217/94, C07D 209/86, C07D 215/38, C07D 263/48, C07D 215/02, C07D 209/88, G03G 5/06

(21) Application number: **87119260.5**

(22) Date of filing: **28.12.87**

(54) **Enamine derivatives, production thereof and electrophotographic plates containing the same.**

(30) Priority: **29.12.86 JP 309380/86**
**21.10.87 JP 265834/87**

(43) Date of publication of application:
**06.07.88 Bulletin 88/27**

(45) Publication of the grant of the patent:
**17.03.93 Bulletin 93/11**

(84) Designated Contracting States:
**DE FR GB**

(56) References cited:
**DE-A- 2 064 481**
**DE-A- 3 414 141**

**JOURNAL OF IMAGING SCIENCE, vol. 29, no. 1, 1985, Springfield S.L. RICE et al. "A new class of photoconductive transport materials: substituted phenyl and diphenyl acetaldehyde enamines" pages 7-10**

(73) Proprietor: **Hitachi Chemical Co., Ltd.**
**1-1, Nishishinjuku 2-chome**
**Shinjuku-ku, Tokyo 160(JP)**

(72) Inventor: **Katsuya, Yasuo**
**21-2-302, Nishinarusawacho-3-chome**
**Hitachi-shi(JP)**
Inventor: **Akimoto, Takayuki**
**Yamazakiryo 2-9, Higashicho-4-chome**
**Hitachi-shi(JP)**
Inventor: **Morishita, Yoshii**
**Yamazakiryo 2-9, Higashicho-4-chome**
**Hitachi-shi(JP)**
Inventor: **Shinbo, Yasushi**
**Yamazakiryo 2-9, Higashicho-4-chome**
**Hitachi-shi(JP)**
Inventor: **Kageyama, Akira**
**3-6, Takasuzucho-5-chome**
**Hitachi-shi(JP)**
Inventor: **Hayashida, Shigeru**
**16-5-402, Sukegawacho-5-chome**
**Hitachi-shi(JP)**

(74) Representative: **Kraus, Walter, Dr. et al**
**Patentanwälte Kraus, Weisert & Partner**
**Thomas-Wimmer-Ring 15**
**W-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

This invention relates to enamine derivatives, processes for producing the same and electrophotographic plates containing the same as a charge transport material and excellent in sensitivity, photoresponse and durability.

In electrophotographic plates using photoconductive substances as a photosensitive material, there have been used mainly inorganic photoconductive substances such as selenium, zinc oxide, titanium oxide, cadmium sulfide, etc. But since most of these substances are generally strongly toxic, there is a problem in their disposal, otherwise they have low durability, e.g. in the case of zinc oxide.

On the other hand, photosensitive materials using organic photoconductive compounds are widely studied recently, since they are generally weak in toxicity and advantageous in transparency, flexibility, light-weight, cost, and the like comparing with the case of using the inorganic photoconductive substances.

Particularly in the case of two layers type electrophotographic plates wherein functions of generation and transport of charges are separated, the sensitivity which was a large defect of one layer type electrophotographic plates using organic photoconductive compounds can be improved greatly, so that the two layers type electrophotographic plates are rapidly progressing recently. These two layers type electrophotographic plates are applied to electrophotographic apparatuses by the Carlson method, e.g., copying machines, laser beam printers, facsimile machines, etc. With recent progress in miniaturization of electrophotographic apparatuses and high speed in printing speed, there have been demanded for electrophotographic plates so-called high speed photoresponse, wherein a surface potential at the time of exposing to a light image decays in a short time, and a longer life of printed copies.

The high speed photoresponse and the longer life are explained in detail below. With miniaturization of the electrophotographic apparatus, the diameter of a photoreceptive drum used in the apparatus becomes smaller. For example, the diameter of photosensitive drum in a large-size apparatus is 100 to 300 mm, while that in a small-size apparatus is 30 to 60 mm. Thus, in the small-size apparatus, individual parts such as a charging device, a developing device, etc. around the photoreceptive drum are installed in a narrow space and a time required for from the exposure to light image to the development is shortened compared with middle-size and large-size machines. Further, when the drum diameter is small, it is necessary to increase the revolving rate of drum in order to copy or print on sheets with the same rate. Therefore, in an electrophotographic process the time required for each step in an electrophotographic process of charging → exposing to a light image → developing → transferring → destaticizing becomes shorter and shorter. Further, among required performance for the electrophotographic plates, it becomes important to decay the surface potential rapidly after exposure to a light image. In other words, it is necessary to obtain electrophotographic plates good in photoresponse.

Further, since the drum diameter is reduced and the revolving rate is increased, it is necessary to repeat the above-mentioned electrophotographic process much more times compared with the case of using a larger drum diameter in order to obtain the same number of sheets by electrophotography. Thus, it is necessary to improve durability for repeated use of the electrophotographic plates.

The organic photosensitive body generally comprises a charge generating material which generates charges by absorbing light, a charge transport material which transports the charges, a binder and a small amount of additives depending on necessity. The photoresponse is mainly controlled by the charge transport material. In order to obtain high speed photoresponse, it is known to select suitable charge transport materials, or to increase the compounding ratio of the charge transport material to the binder.

As the charge transport materials, there are known pyrazoline derivatives disclosed in e.g. J. Photographic Science and Engineering vol. 21(2), p.73 (1977), etc.; oxazole derivatives disclosed in e.g. Japanese Patent Unexamined Publication No. 58-87557, U.S. Patent Nos. 4,346,157; 4,619,879; 4,150,987; 4,278,747; 4,367,273; 4,365,014 and 4,454,212; hydrazone derivatives disclosed in e.g. Japanese Patent Unexamined Publication Nos. 54-59143, 54-150128 and 55-46760; enamine derivatives disclosed in e.g. J. Imaging Science vol. 29(1), p. 7 (1985); etc.

As to the method for enlarging the compounding ratio of the charge transport material to the binder in order to obtain high speed photoresponse, there are problems in known charge transport materials. For example, in the case of using pyrazoline derivatives and oxazole derivatives, the photoresponse is improved by increasing the compounding ratio of the charge transport material to the binder, but durability for repeated electrophotographic process is lowered, and there take place phenomena that an image fine line is broadened in the case of regular development and an image fine line is thinned in the case of reverse development. Such phenomena are called as lowering in print resolution. In the case of using hydrazone derivatives as the charge transport material, the photoresponse is undesirably slow, and there take place undesirably image fogging (toners are adhered to a white ground to make black) in the case of regular

development and lowering in print density in the case of reverse development, due to an increase of potential after exposure to a light image, that is, an increase of residual potential, when electrophotography is repeated.

On the other hand, according to the above-mentioned J. Imaging Science, the following enamine derivatives are disclosed:

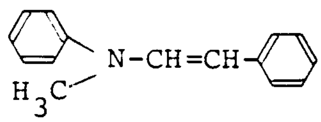
(A)

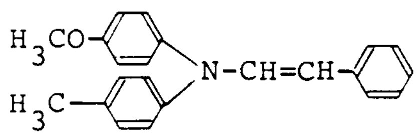
(B)

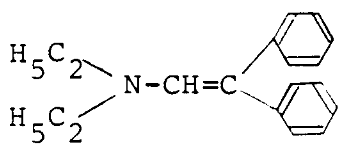
(C)

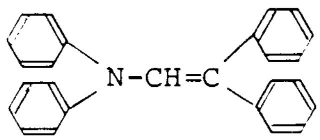
(D)

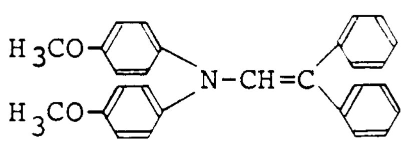
(E)

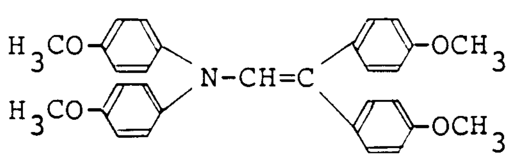
(F)

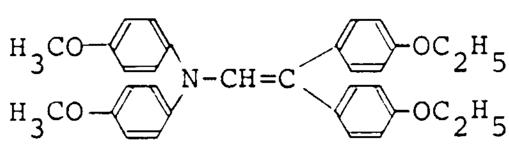
(G)

Among the compounds, it is described that the compounds (A), (B) and (C) are lacking in photoconductivity, the compound (D) is poor in photoconductivity and the compounds (E), (F) and (G) have good or excellent photoconductivity. But when the compounds (E), (F) and (G) are used as a charge transport material, they can withstand the repeated use but are insufficient in the sensitivity and photoresponse. The improvement in these points has been desired.

It is an object of this invention to provide enamine derivatives usable as a charge transport material excellent in photoresponse and durability in repeated use. It is another object of this invention to provide processes for producing these enamine derivatives. It is a further object of this invention to provide an electrophotographic plate containing such enamine derivatives as a charge transport material.

This invention provides an enamine derivative of the formula:

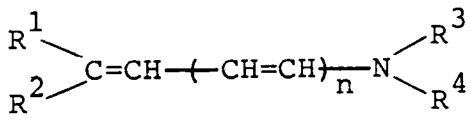

(I)

wherein

(i) in the case of n being zero,

(a) $R^1$ is a group of the formula:

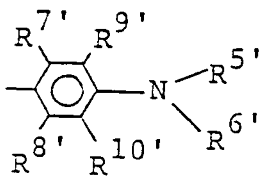

or

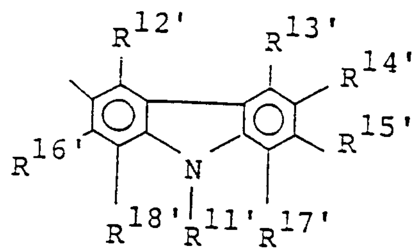

in which $R^{5'}$ and $R^{6'}$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{7'}$ and $R^{8'}$ are independently hydrogen, an alkyl group having 1 to 9 carbon atoms, or an alkoxy group having 1 to 4 carbon atoms; $R^{9'}$ and $R^{10'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group, or a cyano group; $R^{11'}$ is an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{12'}$, $R^{13'}$, $R^{14'}$, $R^{15'}$ and $R^{16'}$ are independently hydrogen, an alkyl group having 1 to 9 carbon atoms, or an alkoxy group having 1 to 4 carbon atoms; $R^{17'}$ and $R^{18'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group; $R^2$, $R^3$ and $R^4$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups,alkyl groups, alkoxy groups, acetyl groups, acetoxy groups, arylamino groups, aralkylamino groups, aryl groups, and aryloxy groups, or a

heterocyclic ring group, and further $R^2$ may be hydrogen, and $R^3$ and $R^4$ may together form a group of the formula:

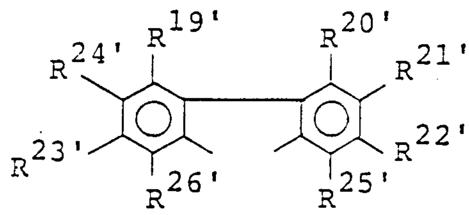

in which $R^{19'}$, $R^{20'}$, $R^{21'}$, $R^{22'}$, $R^{23'}$ and $R^{24'}$ are independently hydrogen, an alkyl group having 1 to 9 carbon atoms or an alkoxy group having 1 to 4 carbon atoms; $R^{25'}$ and $R^{26'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group, or

(b) $R^1$ is a group of the formula:

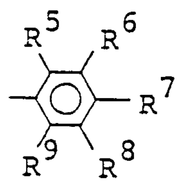

in which $R^5$, $R^6$, $R^7$, $R^6$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain non-substituted or substituted alkyl groups having 1 to 9 carbon atoms, said substituted alkyl groups including alkyl groups substituted with one or more halogen atoms, hydroxyl groups, cyano groups, nitro groups, alkylamino groups, arylamino groups, aryl groups, and aryloxy groups, an amino group substituted with one or two non-substituted or substituted aralkyl groups, said substituted aralkyl groups including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two non-substituted or substituted heterocyclic ring groups, said substituted heterocyclic ring groups including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, a hydroxyl group, a nitro group or a cyano group; $R^2$ is hydrogen, an alkyl group having 1 to 9 carbon atoms, an

aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; and $R^3$ and $R^4$ together form a group of the formula:

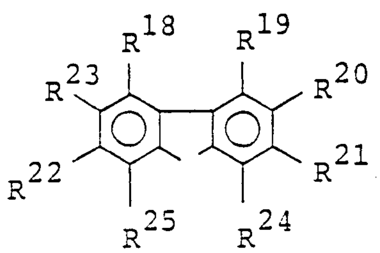

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group; and

(ii) in the case of n being an integer of 1 or 2, $R^1$ is a group of the formula:

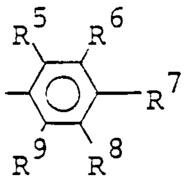

or

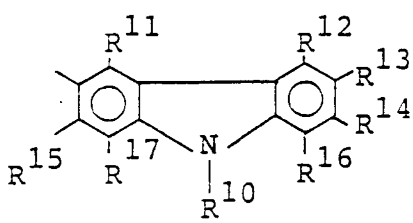

in which $R^5$ through $R^9$ are as defined above; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocylclic ring

group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, an alkoxy group having 1 to 4 carbon atoms, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups,; $R^{16}$ and $R^{17}$ are independently hydrogen, a halogen atom, a straight-or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, and further $R^2$ may be a hydrogen atom, and $R^3$ and $R^4$ may together form a group of the formula:

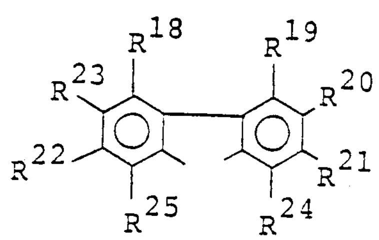

(II)

in which $R^{18}$ through $R^{25}$ are as defined above.

This invention further provides an electrophotographic plate characterized by using the enamine derivative of the formula (I) as a charge transport material.

Figs. 1 to 3 are graphs showing surface potential decaying curves obtained in the photoresponse test of electrophotographic plates of Examples and Comparative Examples.

Figs. 4, 5, 7, 8, 10, 12, 13, 14 and 15 are NMR spectra of enamine derivatives obtained in Examples of this invention.

Figs. 6, 9 and 11 are infrared spectra of enamine derivatives obtained in Examples of this invention.

The enamine derivative of this invention is represented by the formula:

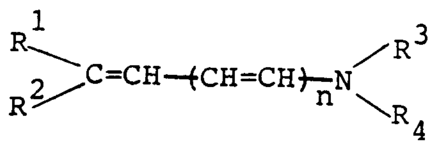

(I)

wherein

(i) in the case of n being zero,

(a) $R^1$ is a group of the formula:

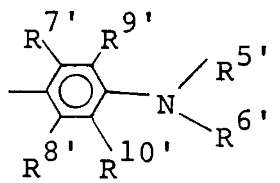

or

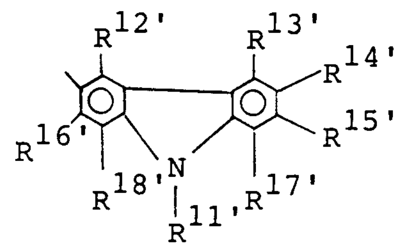

in which $R^{5'}$ and $R^{6'}$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{7'}$ and $R^{8'}$ are independently hydrogen, an alkyl group having 1 to 9 carbon atoms, or an alkoxy group having 1 to 4 carbon atoms; $R^{9'}$ and $R^{10'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group, or a cyano group; $R^{11'}$ is an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{12'}$, $R^{13'}$, $R^{14'}$, $R^{15'}$ and $R^{16'}$ are independently hydrogen, an alkyl group having 1 to 9 carbon atoms, or an alkoxy group having 1 to 4 carbon atoms; $R^{17'}$ and $R^{18'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group; $R^2$, $R^3$ and $R^4$ are independently an alkyl group having 1 to 9 carbon atoms, an aryl group which may have one

or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group, and further $R^2$ may be hydrogen, and $R^3$ and $R^4$ may together form a group of the formula:

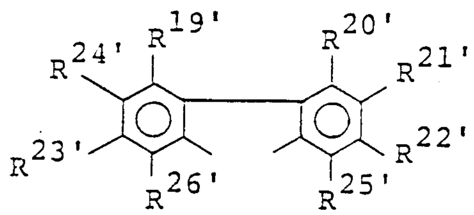

in which $R^{19'}$, $R^{20'}$, $R^{21'}$, $R^{22'}$, $R^{23'}$ and $R^{24'}$ are independently hydrogen, an alkyl group having 1 to 9 carbon atoms or an alkoxy group having 1 to 4 carbon atoms; $R^{25'}$ and $R^{26'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group, or

(b) $R^1$ is a group of the formula:

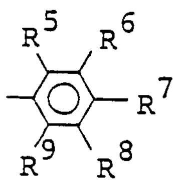

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain non-substituted or substituted alkyl groups having 1 to 9 carbon atoms, said substituted alkyl groups including alkyl groups substituted with one or more halogen atoms, hydroxyl groups, cyano groups, nitro groups, alkylamino groups, arylamino groups, aryl groups, and aryloxy groups, an amino group substituted with one or two non-substituted or substituted aralkyl groups, said substituted aralkyl groups including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an amino group substituted with

one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two non-substituted or substituted heterocyclic ring groups, said substituted heterocyclic ring groups including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, a hydroxyl group, a nitro group or a cyano group; $R^2$ is hydrogen, an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; and $R^3$ and $R^4$ together form a group of the formula:

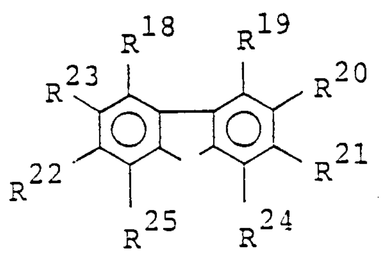

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group; and

(ii) in the case of n being an integer of 1 or 2, $R^1$ is a group of the formula:

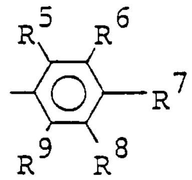

or

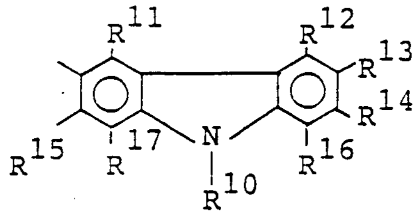

in which $R^5$ through $R^9$ are as defined above; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocylclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, an alkoxy group having 1 to 4 carbon atoms, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups,; $R^{16}$ and $R^{17}$ are independently hydrogen, a halogen atom, a straight-or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and

aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, and further $R^2$ may be a hydrogen atom, and $R^3$ and $R^4$ may together form a group of the formula:

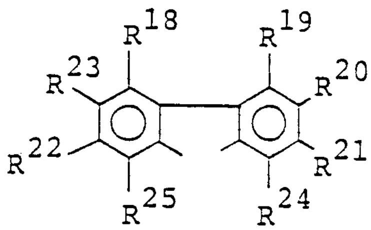

(II)

in which $R^{18}$ through $R^{25}$ are as defined above.

In the formulae (I) and (II), the straight- or branched-chain alkyl group having 1 to 9 carbon atoms includes a methyl group, an ethyl group, an n-propyl group, an isopropyl group, an n-butyl group, an isobutyl group, a sec-butyl group, a tert-butyl group, a pentyl group, a hexyl group, an octyl group, and a nonyl group. These alkyl groups may be substituted with one or more halogen atoms, hydroxyl groups, cyano groups, nitro groups, alkylamino groups, arylamino groups, aryl groups, aryloxy groups. Examples of such substituted alkyl groups include a monochloromethyl group, a 1-hydroxyethyl group, an N,N-diphenylaminomethyl group, a 3-N,N-diethylaminobutyl group, a 2-cyanopentyl group, a 2-p-ethylphenoxyethyl group,

The term "aryl group" includes a phenyl group, a biphenyl group, a xylyl group, a hydroxyphenyl group, a naphthyl group, an anthryl group, a phenanthryl group, and a pyrenyl group.

These aryl groups may be substituted with one or more halogen atoms, hydroxyl groups, cyano groups, nitro groups, alkylamino groups, amino groups, alkyl groups, alkoxy group, acetyl groups, acetoxy groups, arylamino and aralkylamino groups such as diphenylamino, ditolylamino, naphthylamino, di(m-methylphenyl)amino, di(n-ethylphenyl)amino, di(p-methylphenyl)amino, di(p-ethylphenyl)amino, dibenzylamino, diphenethylamino, di(p-methoxy)benzylamino, di(p-ethoxy)benzylamino, di(m-methoxy)benzylamino, and di-(m-ethoxy)benzylamino; aryl groups such as phenyl, naphthyl, p-methoxyphenyl, p-ethoxyphenyl, p-dimethylaminophenyl, p-diethylaminophenyl, and p-di-tert-butylaminophenyl; aryloxy groups such as phenoxy, naphthoxy, anthryl, anthryloxy, p-methoxyphenyloxy, p-ethoxyphenyloxy, p-dimethylaminophenyloxy, p-diethylaminophenyloxy, and p-dimethylaminophenyloxy, p-diethylaminophenyloxy, and p-di-tert-butylaminophenyloxy groups.

Examples of substituted aryl groups are a hydroxyphenyl groups, a tolyl group, a xylyl group, a chlorophenyl group, a dimethylaminophenyl group, a diphenylaminophenyl group, a dibenzylaminophenyl group, a dianthrylaminophenyl group.

The term "aryloxy group" includes a phenoxy group, a naphthoxy group, a tolyloxy group, a biphenyloxy group, a xylyloxy group, a hydroxyphenyloxy group, a naphthyloxy group, an anthryloxy group, a phenanthryloxy group, and a pyrenyloxy group.

These aryloxy groups may be substituted with one or more halogen atoms, hydroxyl groups, cyano groups, nitro groups, alkylamino groups, amino groups, alkyl groups, alkoxy groups, acetyl groups, acetoxy groups, the same arylamino groups, aralkylamino groups, aryl groups, and aryloxy groups, as mentioned above.

Examples of substituted aryloxy groups are a chlorophenyloxy group, a hydroxyphenyloxy group, a tolyloxy group, a xylyloxy group, a dimethylaminophenyloxy group, a diphenylaminophenyloxy group, a dibenzylaminophenyloxy group, a dianthrylaminophenyloxy group, a 3-methylnaphthyloxy group.

The term "aralkyl group" includes a benzyl group, a phenylethyl group, a naphthylethyl group, and a naphthylmethyl group.

These aralkyl groups may be substituted with one or more halogen atoms, hydroxyl groups, cyano groups, nitro groups, alkylamino groups, amino groups, alkyl groups, alkoxy groups, acetyl groups, acetoxy

groups, the same aryl groups, aryloxy groups, arylamino groups, and aralkylamino groups as mentioned above.

Examples of substituted aralkyl groups are a diethylaminobenzyl group, a 3-hydroxyphenyl group, and a p-ethoxy-o-chlorobenzyl group.

The term "heterocyclic ring group" includes a furyl group, a thiofuryl group, a pyrrolyl group, a pyranyl group, a pyridyl group, an oxazolyl group, a thiazolyl group, a pyrimidinyl group, a triazolyl group, an acridinyl group, a carbazolyl group, a carbolinyl group, a quinolyl group, a phenothiazinyl group, a quinoxalinyl group, a pyrazolinyl group, an imidazolyl group, an oxadiazolyl group, and an indolyl group.

These heterocyclic ring groups may be substituted with one or more halogen atoms, hydroxyl groups, cyano groups, nitro groups, alkylamino groups, amino groups, alkyl groups, alkoxy groups, acetyl groups, acetoxy groups, the same aryl groups, aryloxy groups, arylamino groups, and aralkylamino groups as mentioned above.

Examples of substituted heterocyclic ring groups are a 2-methylpyrrolyl group, a 3-p-dimethylphenyl-1,3-oxazolyl group, and a 4-ethylcarbazolyl group.

The straight- or branched-chain alkoxy group having 1 to 4 carbon atoms includes a methoxy group, an ethoxy group, a propoxy group, an isopropoxy group, a butoxy group, a sec-butoxy group, and a tert-butoxy group.

The amino group substituted with one or two straight- or branched-chain, non-substituted or substituted alkyl groups having 1 to 9 carbon atoms includes a monomethylamino group, a dimethylamino group, a diethylamino group, and a monoisopropylamino group.

The amino group substituted with one or two non-substituted or substituted aralkyl groups includes a monobenzylamino group, a dibenzylamino group, a monophenylethylamino group, a di(phenylethyl)amino group, a monomethylbenzylamino group, and a di (methylbenzyl) amino group.

The amino group substituted with one or two non-substituted or substituted aryl groups includes a monophenylamino group, a diphenylamino group, a monotolylamino group, a ditolylamino group, a mononaphthylamino group, and a dinaphthylamino group.

The amino group substituted with one or two non-substituted or substituted heterocyclic ring groups includes a monopyridylamino group, a dipyridylamino group, a monoquinolylamino group, a diquinolylamino group, a monocarbazolylamino group, a dicarbazolylamino group, a monoacridinylamino group, a diacridinylamino group, a monooxazolylamino group, and a dioxazolylamino group.

The enamine derivatives of the formula (I) include those of the formula:

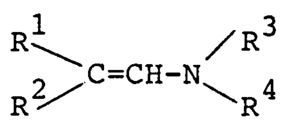

(I-1)

wherein $R^1$ is a group of the formula:

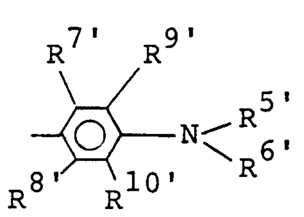

or

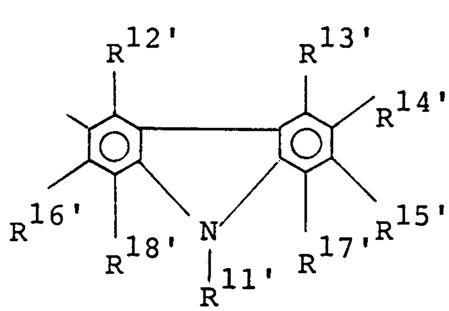

in which $R^{5'}$ and $R^{6'}$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{7'}$

and $R^{8'}$ are independently hydrogen, an alkyl group having 1 to 9 carbon atoms, or an alkoxy group having 1 to 4 carbon atoms; $R^{9'}$ and $R^{10'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group, or a cyano group; $R^{11'}$ is an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{12'}$, $R^{13'}$, $R^{14'}$, $R^{15'}$ and $R^{16'}$ are independently hydrogen, an alkyl group having 1 to 9 carbon atoms, or an alkoxy group having 1 to 4 carbon atoms; $R^{17'}$ and $R^{18'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group; $R^2$, $R^3$ and $R^4$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group, and further $R^2$ may be hydrogen, and $R^3$ and $R^4$ may together form a group of the formula:

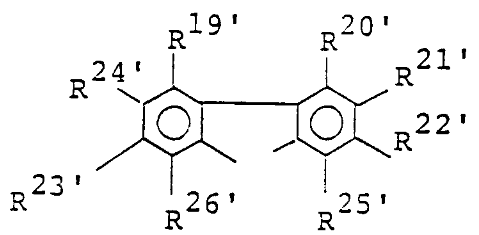

in which $R^{19'}$, $R^{20'}$, $R^{21'}$, $R^{22'}$, $R^{23'}$ and $R^{24'}$ are independently hydrogen, an alkyl group having 1 to 9 carbon atoms or an alkoxy group having 1 to 4 carbon atoms; $R^{25'}$ and $R^{26'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group.

The enamine derivatives of the formula (I) include those of the formula:

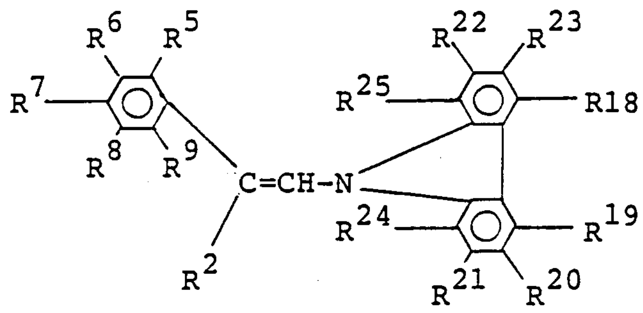

wherein $R^5$ through $R^9$ and $R^{18}$ through $R^{25}$ are as defined above; and $R^2$ is hydrogen, an alkyl group having 1 to 9 carbon atoms, an aryl group which may have one or more substituents, an aralkyl group which may have one or more substituents, or a heterocyclic ring group.

The enamine derivatives of the formula (I) include those of the formula:

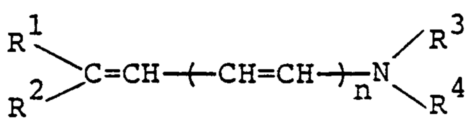

(I)

wherein $R^1$ is a group of the formula:

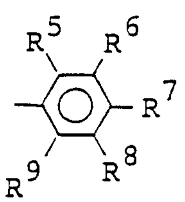

or

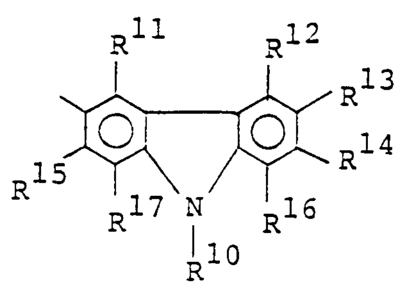

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain alkyl groups having 1 to 9 carbon atoms, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a

hydroxyl group, a nitro group or a cyano group; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{16}$ and $R^{17}$ are independently hydrogen, a straight-or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, and further $R^2$ may be a hydrogen atom, and $R^3$ and $R^4$ may together form a group of the formula:

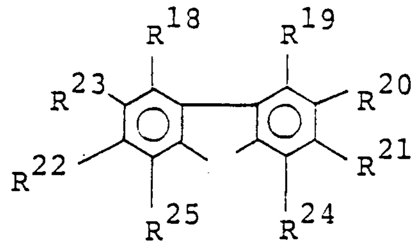 (II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group; and n is an integer of 1 or 2.

The enamine derivative of the formula (I) functions as a charge transport material. By using the enamine derivative of the formula (I) as a charge transport material, there can be obtained an electrophotographic plate excellent in photoresponse and durability in repeated use.

Among the enamine derivatives of the formula (I), preferable ones are those having as $R^1$ a group of the formula:

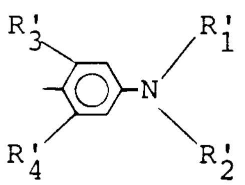 , 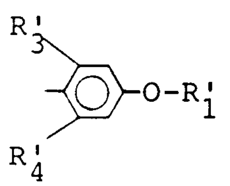 or

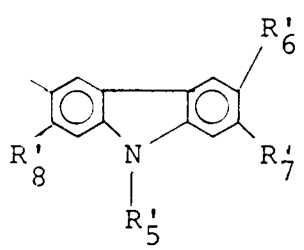

wherein $R_1'$ and $R_2'$ are independently a straight- or branched-chain alkyl group having 1 to 4 carbon atoms, a group of the formula:

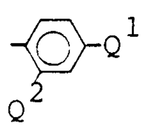 or 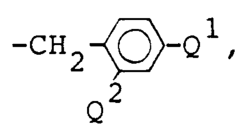 ,

in which $Q^1$ and $Q^2$ are independently hydrogen, a straight-or branched-chain alkyl group having 1 to 4 carbon atoms, or a straight- or branched-chain alkoxy group having 1 to 4 carbon atoms; $R_5'$ is a straight- or branched-chain alkyl group having 1 to 4 carbon atoms or a phenyl group; $R_3'$ , $R_4'$ , $R_6'$ , $R_7'$ and $R_8'$ are independently hydrogen, a straight- or branched-chain alkyl group having 1 to 4 carbon atoms, an alkoxy group having 1 to 4 carbon atoms, or a halogen atom; as $R^2$ hydrogen, a straight- or branched-chain alkyl group having 1 to 4 carbon atoms or a group of the formula:

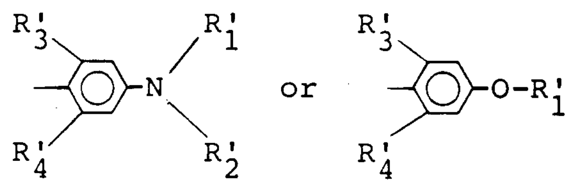

in which $R_1'$ , $R_2'$ , $R_3'$ and $R_4'$ are as defined above; as $R^3$ and $R^4$ independently a group of the formula:

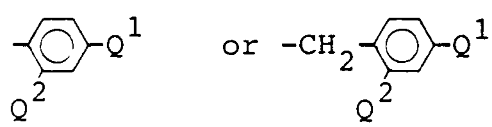

in which $Q^1$ and $Q^2$ are as defined above, or $R^4$ is an alkyl group having 1 to 4 carbon atoms, or $R^3$ and $R^4$ together form a group of the formula:

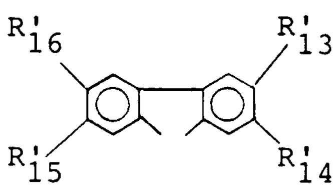

in which $R_{13}'$ , $R_{14}'$ , $R_{15}'$ and $R_{16}'$ are independently hydrogen, a straight- or branched-chain alkyl group having 1 to 4 carbon atoms, an alkoxy group having 1 to 4 carbon atoms, or a halogen atom; and n being zero or an integer of 1 or 2.

Particularly preferable enamine derivatives of the formula (I) are those having as $R^1$ the groups mentioned above and as $R^3$ the groups mentioned above.

More concretely, preferable substituents as $R^1$, $R^2$, $R^3$ and $R^4$ in the formula (I) are as follows:

$R^1$:

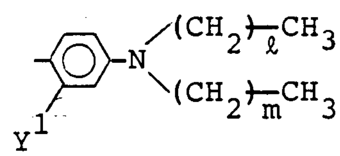

$\ell$ = 0, 1, 2, or 3
m = 0, 1, 2, or 3
$Y^1$ = hydrogen, methoxy or ethoxy

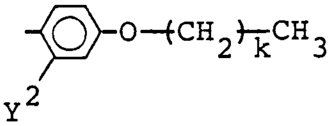

k = o, 1, 2, or 3
$Y^2$ = hydrogen, methoxy, or ethoxy

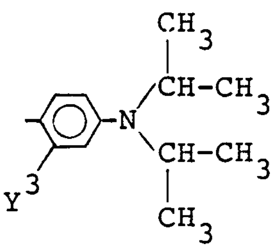

$Y^3$ = hydrogen, methoxy or ethoxy

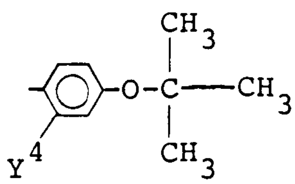

$Y^4$ = hydrogen, methoxy, or ethoxy

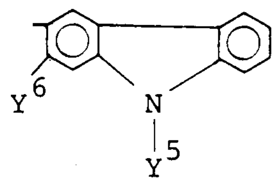

$Y^5$ = methyl or ethyl
$Y^6$ = hydrogen, or $C_{1-4}$ alkoxy

$R^2$: hydrogen, methyl, ethyl or

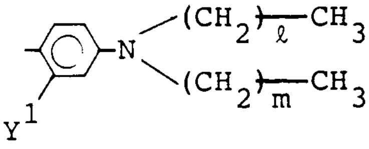

ℓ, m, $Y^1$ = as defined above

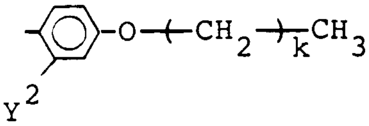

K, $Y^2$ = as defined above

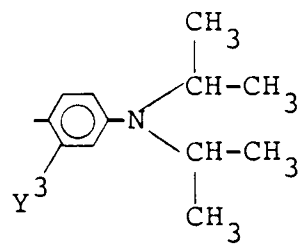

$Y^3$ = as defined above

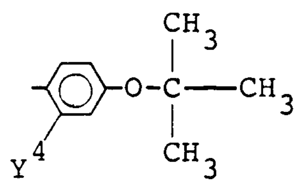

$Y^4$ = as defined above

$R^3$ and $R^4$: independently

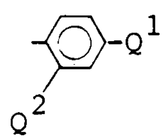

or

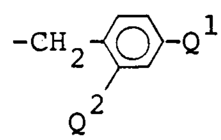

$Q^1$, $Q^2$ = as defined above

In combination to form

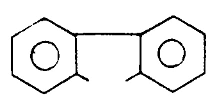

n: zero or an integer of 1 or 2

Examples of the enamine derivatives of the formula (I) are as follows:

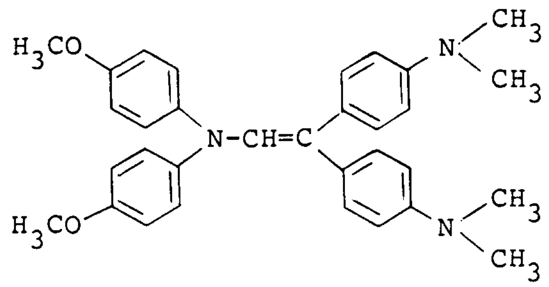

Compound 1

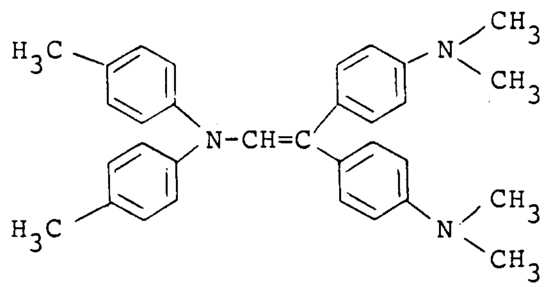

Compound 2

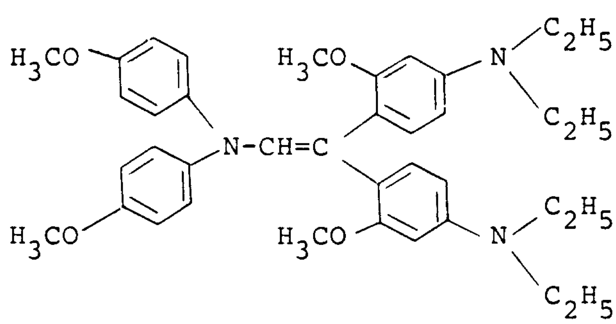

Compound 3

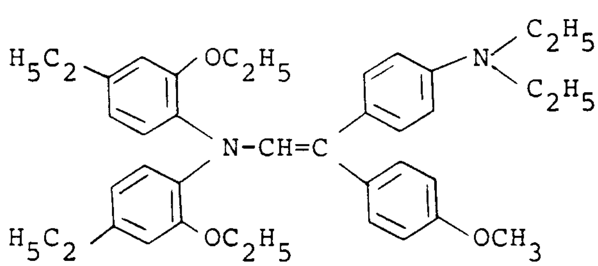

Compound 4

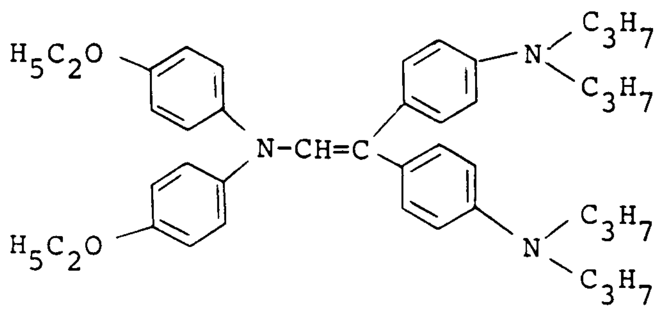

Compound 5

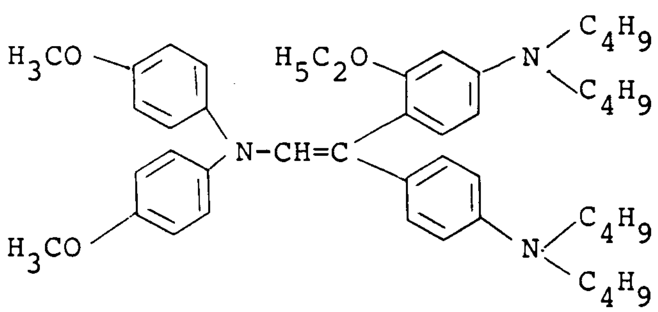

Compound 6

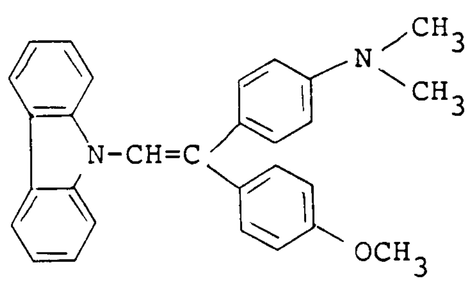

Compound 7

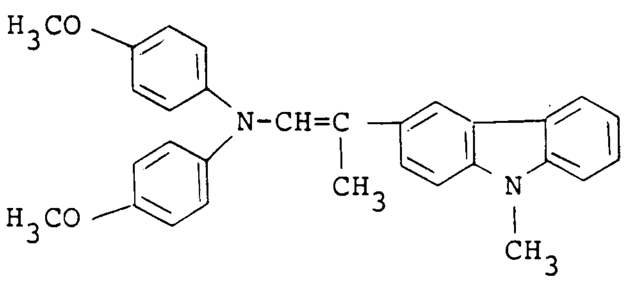

Compound 8

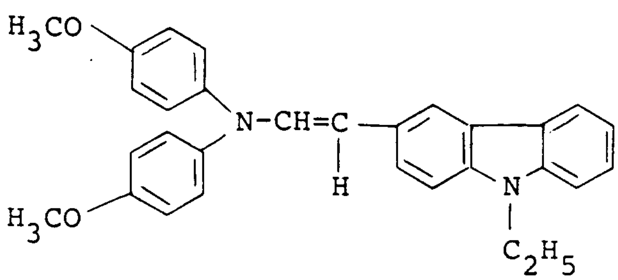

Compound 9

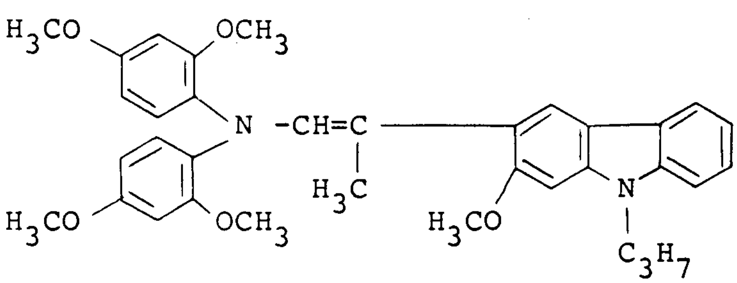

Compound 10

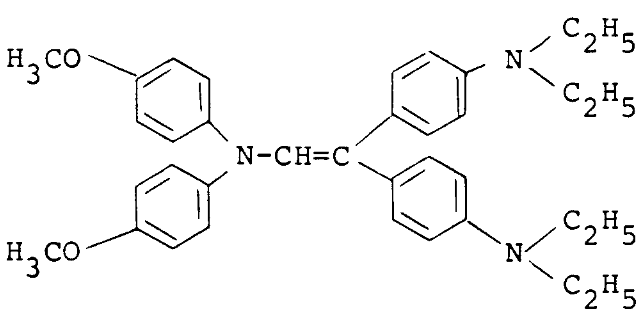

Compound 11

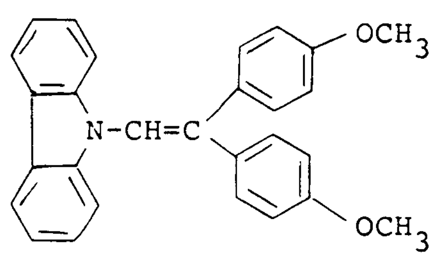

Compound 12

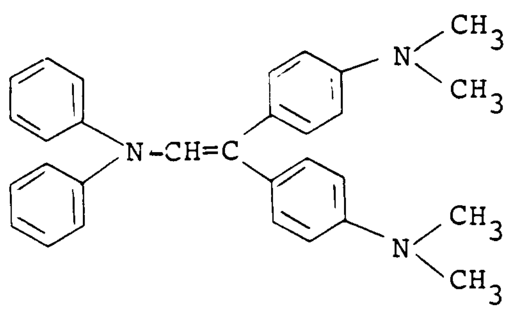

Compound 13

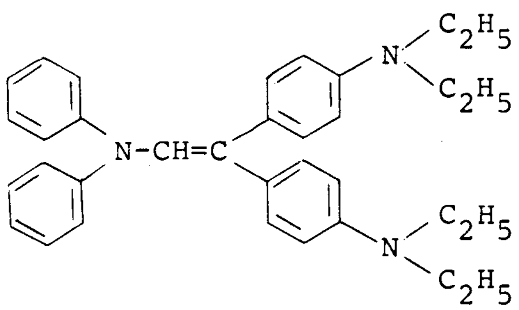

Compound 14

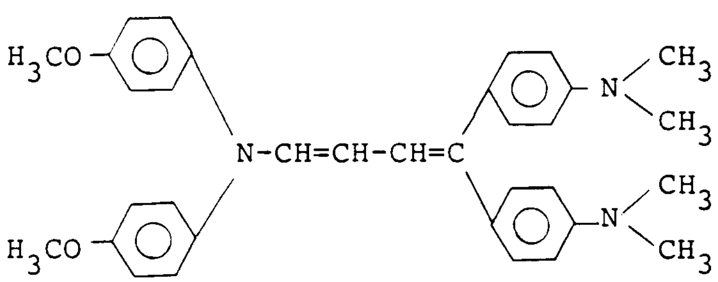

Compound 15

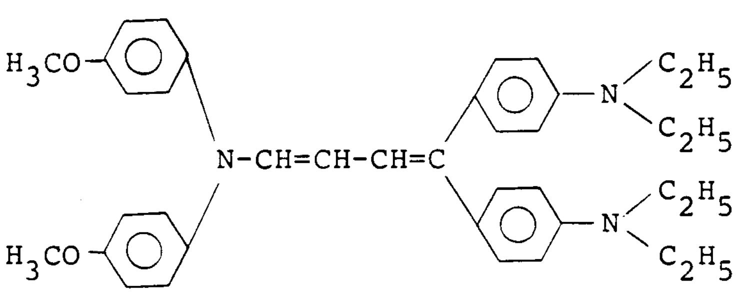

Compound 16

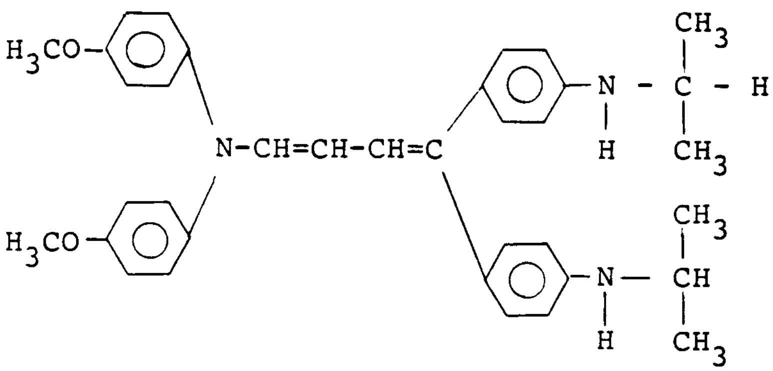

Compound 17

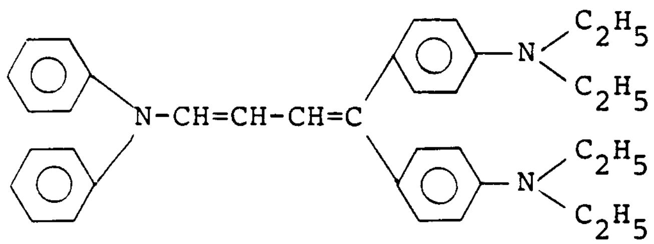

Compound 18

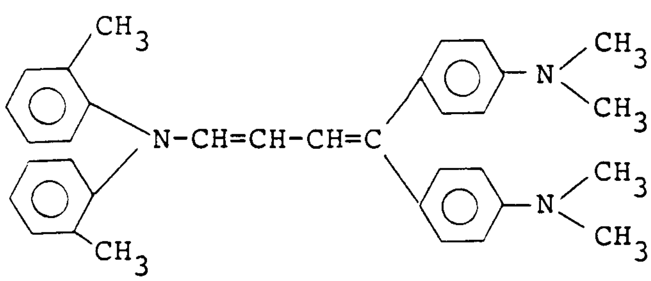

Compound 19

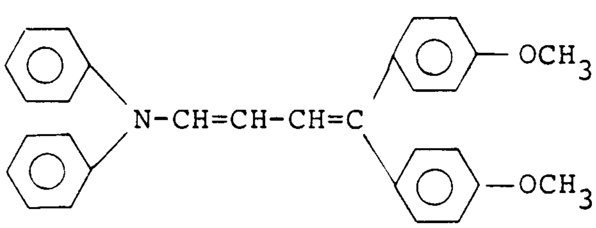

Compound 20

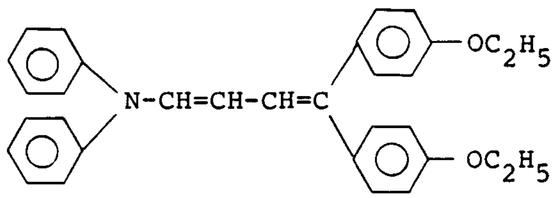

Compound 21

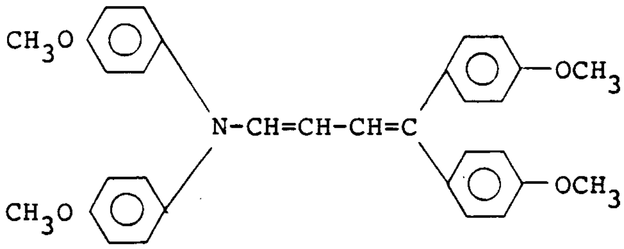

Compound 22

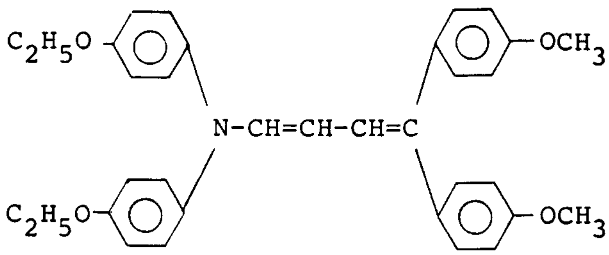

Compound 23

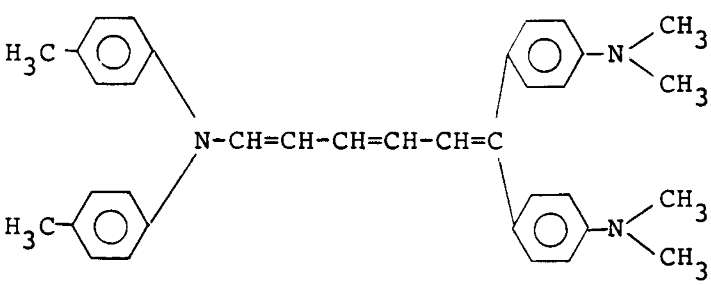

Compound 24

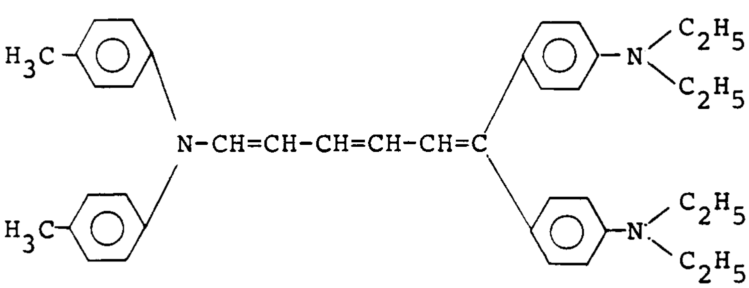

Compound 25

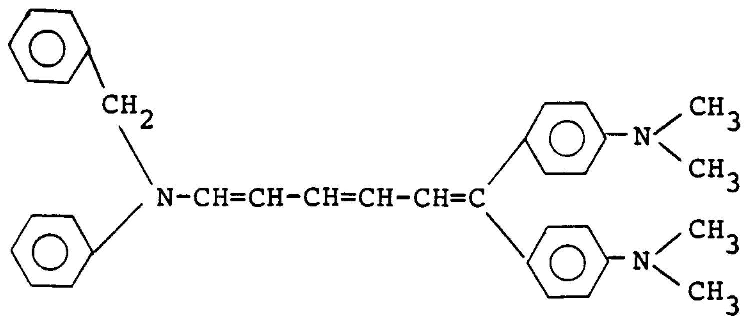

Compound 26

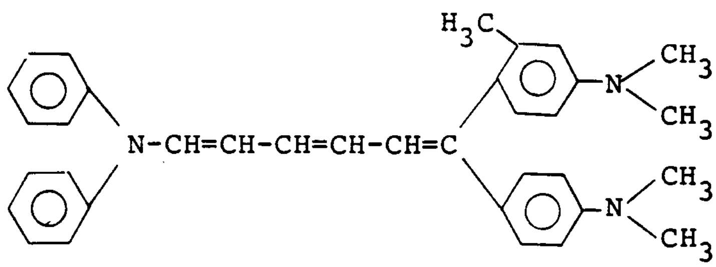

Compound 27

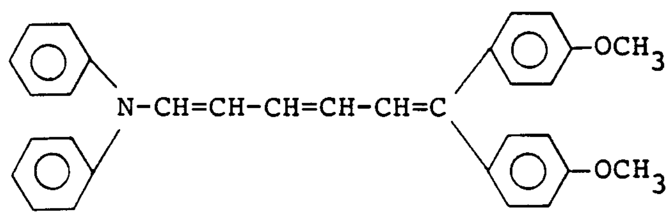

Compound 28

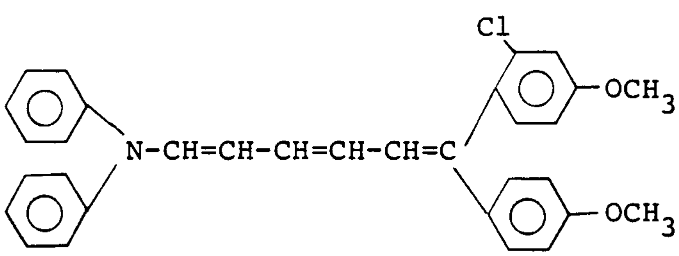

Compound 29

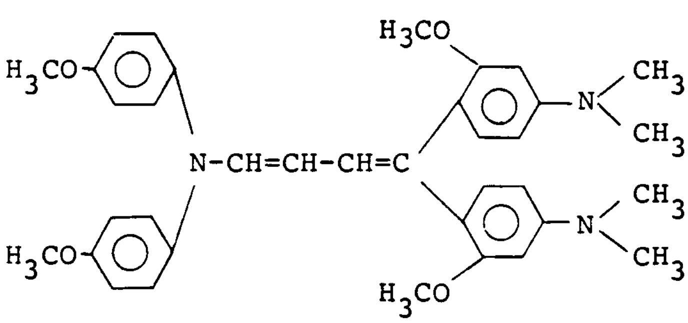

Compound 30

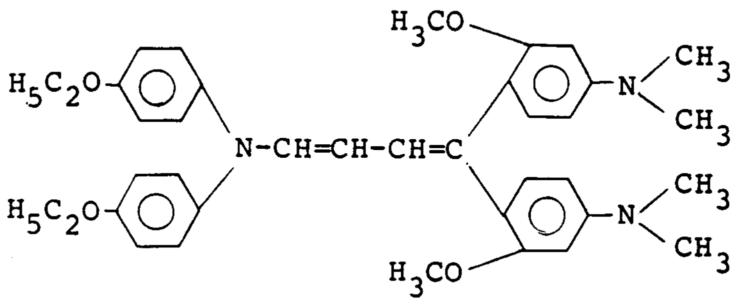

Compound 31

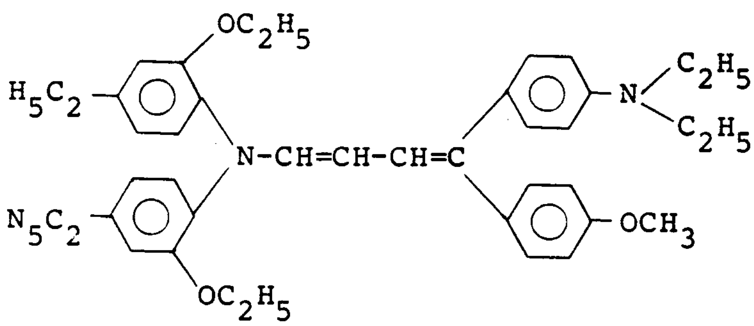

Compound 32

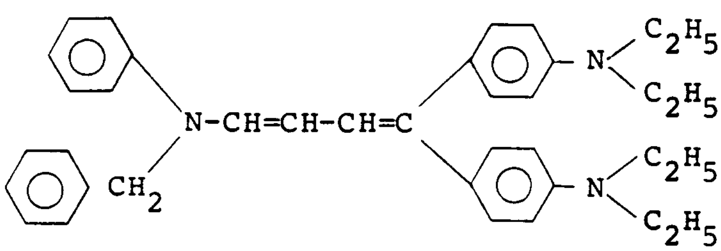

Compound 33

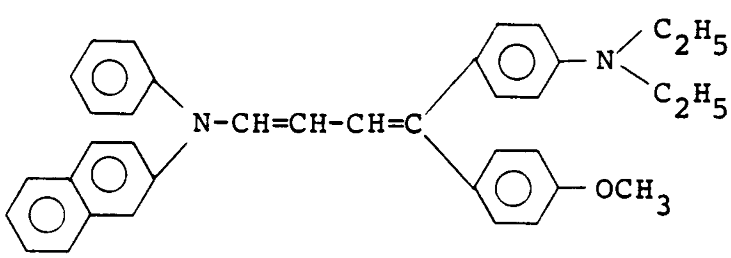

Compound 34

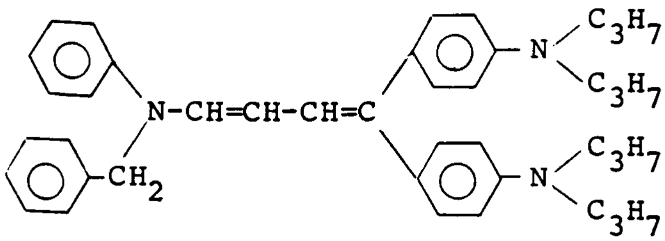

Compound 35

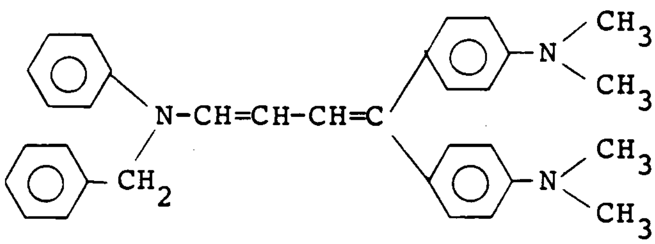

Compound 36

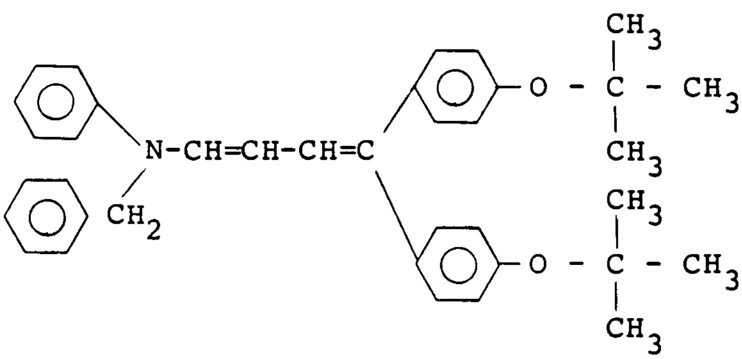

Compound 37

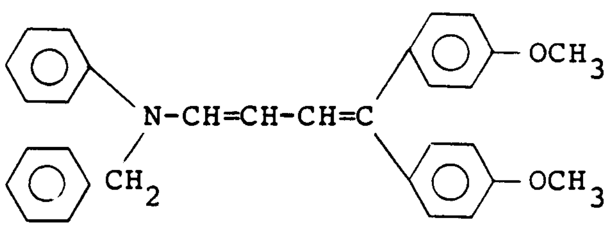

Compound 38

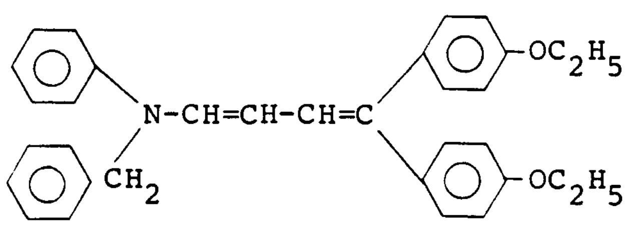

Compound 39

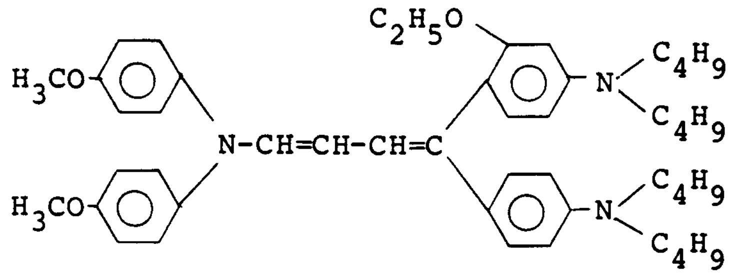

Compound 40

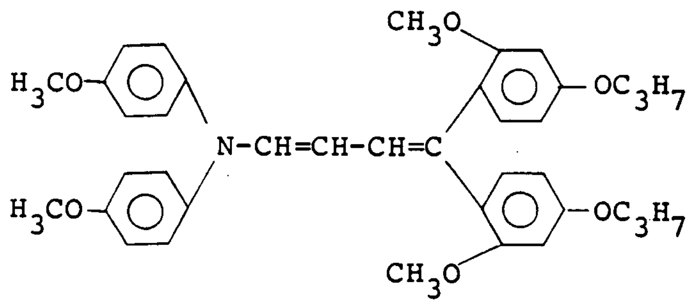

Compound 41

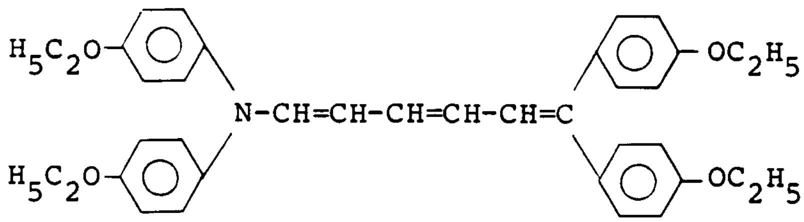

Compound 42

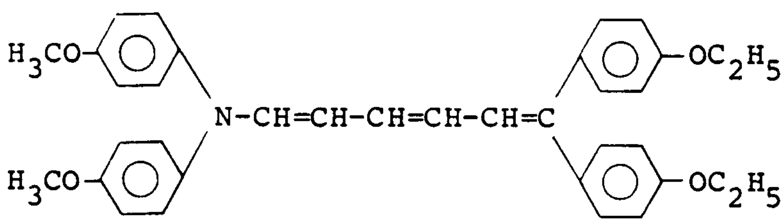

Compound 43

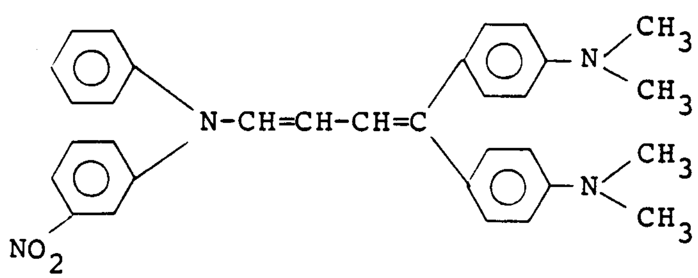

Compound 44

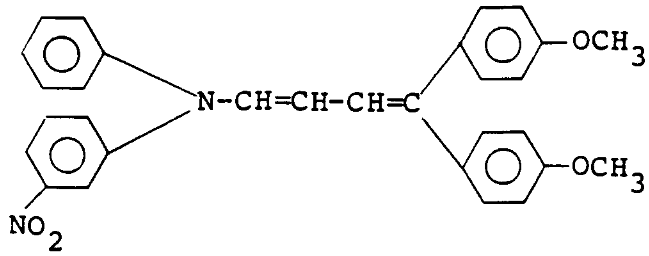

Compound 45

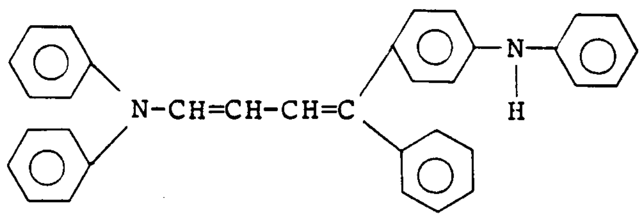

Compound 46

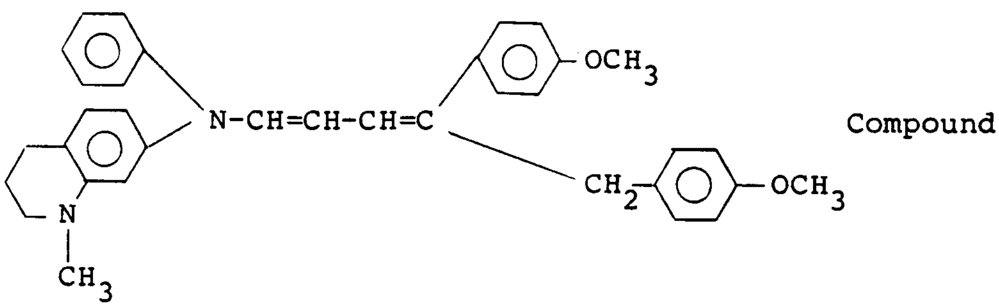

Compound 47

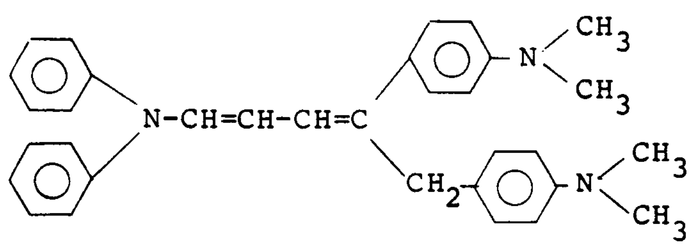

Compound 48

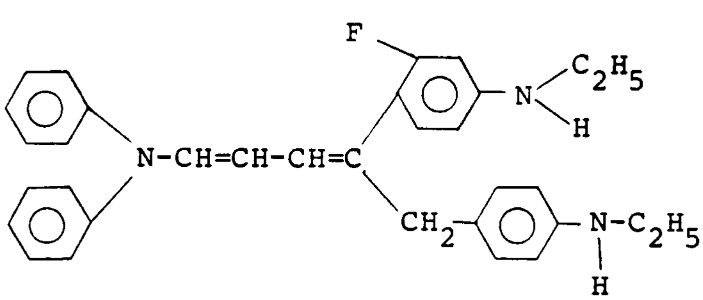

Compound 49

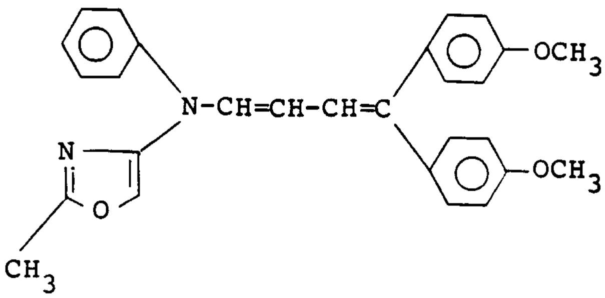

Compound 50

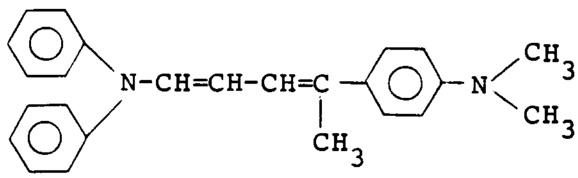

Compound 51

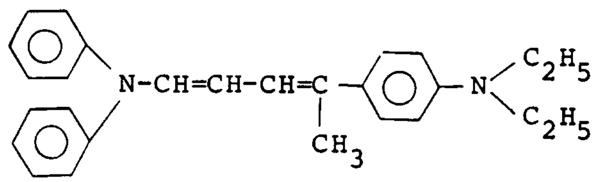

Compound 52

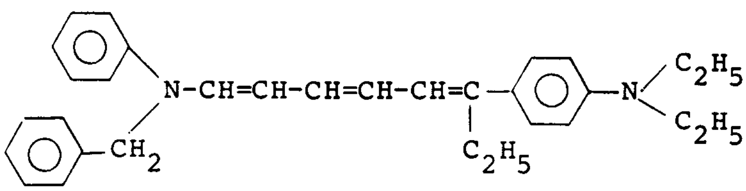

Compound 53

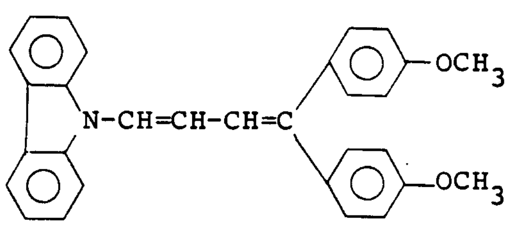

Compound 54

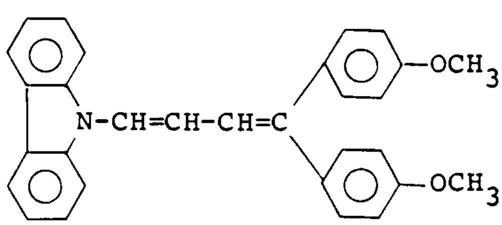

Compound 55

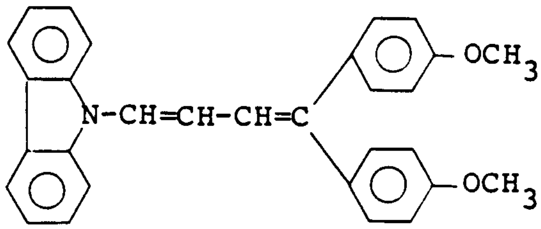

Compound 56

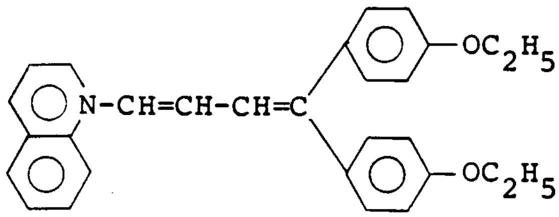

Compound 57

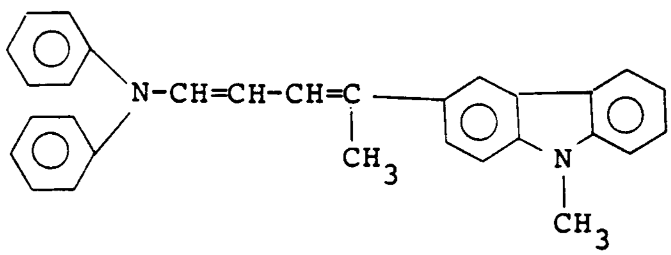

Compound 58

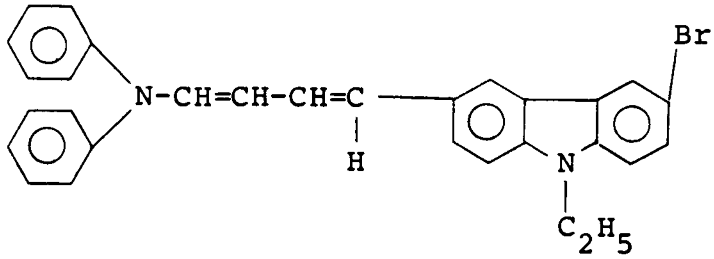

Compound 59

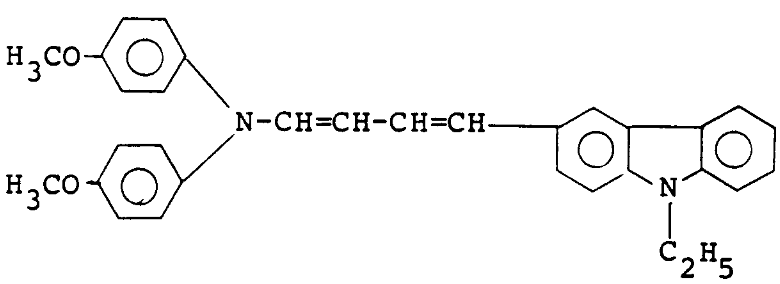

Compound 60

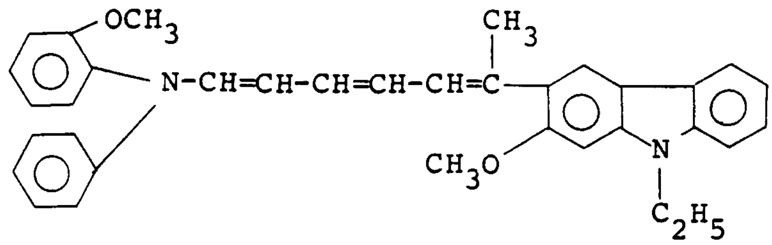

Compound 61

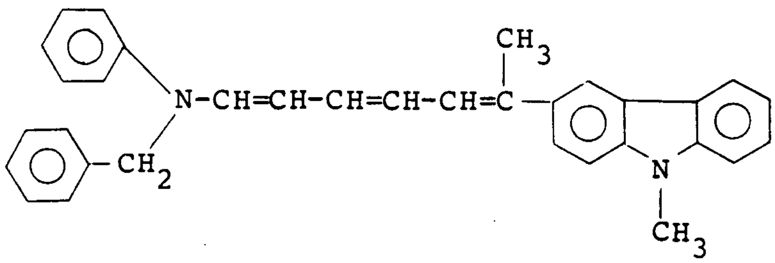

Compound 62

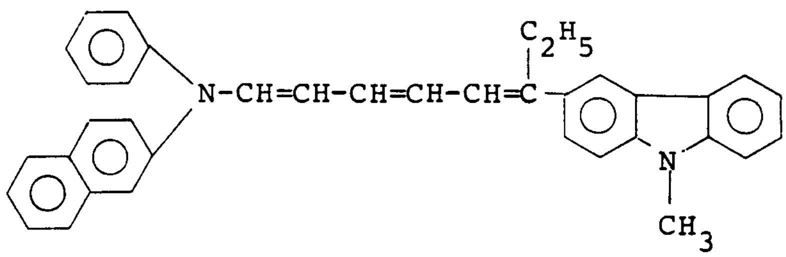

Compound 63

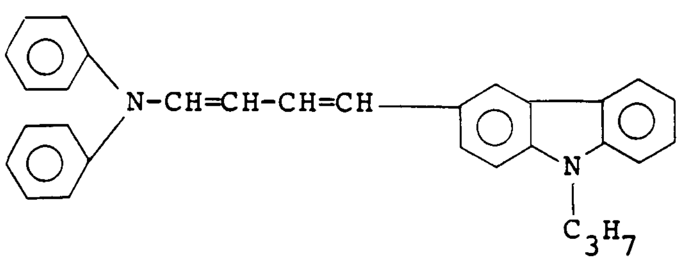

Compound 64

The compounds of the formula (I) can be produced by the processes A to D.

PROCESS A

Particularly, the compound of the formula:

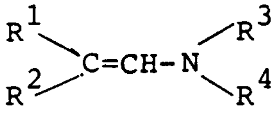

(I-1)

wherein $R^1$, $R^2$, $R^3$ and $R^4$ are as defined above, can be produced by reacting a compound of the formula:

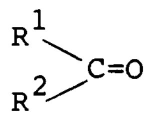

(III)

wherein $R^1$ and $R^2$ are as defined above, with a compound of the formula:

$$Ph_3\overset{+}{P}\overset{-}{Cl}CH_2OCH_3 \quad (IV-1)$$

wherein Ph is a phenyl group, in a solvent in the presence of an alkoxide or an organometallic compound to yield a compound of the formula:

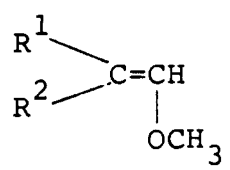

(V)

wherein $R^1$ and $R^2$ are as defined above, hydrolyzing the compound of the formula (V) with an acid, followed by alkali treatment to yield a compound of the formula:

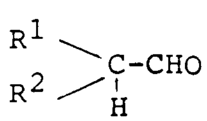

(VI)

wherein $R^1$ and $R^2$ are as defined above, and reacting the compound of the formula (VI) with a compound of the formula:

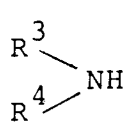

(VII)

wherein $R^3$ and $R^4$ are as defined above, in a solvent in the presence of a sulfonic acid.

The reaction of the compound of the formula (III) with the compound of the formula (IV-1) is known as the Wittig reaction and carried out in a solvent in the presence of an alkoxide or an organometallic compound. As the solvent, there can be used tetrahydrofuran, dimethylformamide, etc. As the alkoxide, there can be used sodium methoxide, sodium ethoxide, potassium methoxide, potassium ethoxide, etc. As the organometallic compound, there can be used n-butyllithium, phenyllithium, etc. The reaction is preferably carried out at -20 ° C to 50 ° C for 30 minutes to 10 hours. The compound of the formula (V) is obtained by pouring the reaction mixture into a large amount of water, extracting with benzene or the like, removing the benzene or the like by distillation after dried, and purifying the residue by a silica gel column or the like.

The compound of the formula (V) is then hydrolyzed with an acid, followed by an alkali treatment to yield the compound of the formula (VI). As the acid, there can be used perchloric acid, hydrochloric acid, sulfuric acid, nitric acid, hydrobromic acid, hydroiodic acid, etc. The hydrolysis reaction can be carried out

by dissolving the compound of the formula (V) in an acid with stirring preferably at 0° to 50°C for 5 minutes to 2 hours. As the alkali used for the alkali treatment, there can be used sodium hydroxide, potassium hydroxide, sodium carbonate, potassium carbonate, etc. The alkali treatment is carried out at -20°C to 30°C. The compound of the formula (VI) is obtained by extracting the reaction mixture with benzene or the like, removing the benzene or the like by distillation after dried to yield a crude product, which is recrystallized from methanol or the like.

The compound of the formula (VI) is then reacted with the compound of the formula (VII) in a solvent in the presence of a sulfonic acid. As the solvent, there can be used benzene, toluene, xylene, chlorobenzene, dichlorobenzene, etc. As the sulfonic acid, there can be used p-toluenesulfonic acid, benzenesulfonic acid, sulfuric acid, etc. Since the reaction is a dehydration reaction, the reaction can be carried out by removing the water from the reaction system placing molecular sieves 4A in the reaction system. The compound of the formula (VI) and the compound of the formula (VII) are used in almost equimolar amounts. The reaction can be carried out preferably at 50 to 130°C for 30 minutes to 5 hours. The desired compound of the formula (I-1) can be obtained by removing the solvent from the reaction mixture by distillation to give a crude product, which is recrystallized from a suitable solvent such as isopropyl ether, or the like.

PROCESS B

The compound of the formula (I-1) can also be produced by reducing a compound of the formula:

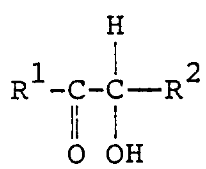

(VIII)

wherein $R^1$ and $R^2$ are as defined above, with a metal hydride, followed by an acid treatment and an alkali treatment to yield a compound of the formula:

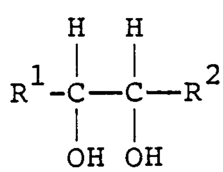

(IX)

wherein $R^1$ and $R^2$ are as defined above, reacting the compound of the formula (IX) with an acid to yield a compound of the formula:

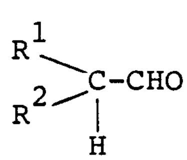

(VI)

wherein $R^1$ and $R^2$ are as defined above, and reacting the compound of the formula (VI) with a compound of the formula:

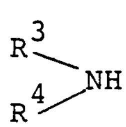

(VII)

wherein $R^3$ and $R^4$ are as defined above, in a solvent in the presence of a sulfonic acid.

The reaction of the compound of the formula (VI) with the compound of the formula (VII) is explained above

PROCESS A.

The compound of the formula (VI) can be obtained by reacting the compound of the formula (IX) with an acid. This reaction is known as the pinacol-pinacoline rearrangement reaction. As the acid, there can be used acetic acid, propionic acid, phosphoric acid, sulfuric acid, etc. The reaction is carried out by charging the compound of the formula (IX) in an acid with stirring. The reaction temperature is preferably 60° to 130°C. The reaction time is preferably 30 minutes to 5 hours. The compound of the formula (VI) is obtained by pouring the reaction mixture into a large amount of ice water, filtering deposited crystals, drying and recrystallizing from isopropyl ether or the like.

The compound of the formula (IX) can be obtained by reducing the compound of the formula (VIII) with a metal hydride, followed by the acid treatment and the alkali treatment. As the metal hydride, there can be used $LiAlH_4$, $NaBH_4$, etc. As the acid, there can be used hydrochloric acid, hydrobromic acid, hydroiodic acid, etc. As the alkali, there can be used sodium hydroxide, potassium hydroxide, sodium carbonate, potassium carbonate, etc. The reduction reaction is preferably carried out in a solvent such as anhydrous ethanol, or the like at 30° to 100°C for 30 minutes to 5 hours. The compound of the formula (IX) can be obtained by pouring the reaction mixture into a large amount of ice water, filtering deposited crystals and recrystallizing from ethanol, or the like.

PROCESS C

The compound of the formula:

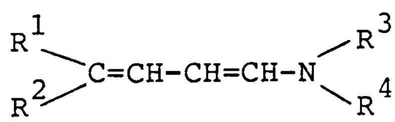

(I-2)

wherein $R^1$, $R^2$, $R^3$ and $R^4$ are as defined above, can be produced by reacting a compound of the formula:

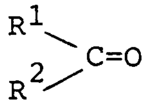

(III)

wherein $R^1$ and $R^2$ are as defined above, with a compound of the formula:

$$Ph_3\overset{+}{P}\overset{-}{I}CH_3 \qquad (IV-2)$$

wherein Ph is a phenyl group, in a solvent in the presence of an alkoxide or an organometallic compound to yield a compound of the formula:

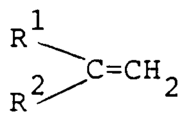

(X)

wherein $R^1$ and $R^2$ are as defined above, formylating the compound of the formula (X) in dimethylformamide in the presence of $POCl_3$ to yield a compound of the formula:

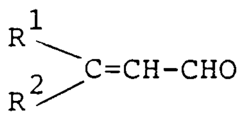

(XI)

wherein $R^1$ and $R^2$ are as defined above, reacting the compound of the formula (XI) with a compound of the formula:

$$Ph_3\overset{+}{P}\overset{-}{Cl}CH_2OCH_3 \qquad (IV-1)$$

wherein Ph is a phenyl group, in a solvent in the presence of an alkoxide or an organometallic compound to yield a compound of the formula:

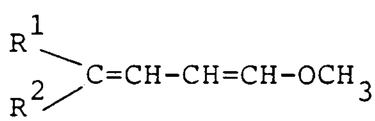

(XII)

wherein $R^1$ and $R^2$ are as defined above, hydrolyzing the compound of the formula (XII) with an acid, followed by an alkali treatment to yield a compound of the formula:

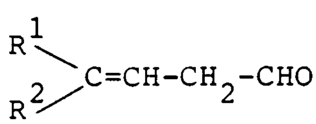

(XIII)

wherein $R^1$ and $R^2$ are as defined above, and reacting the compound of the formula (XIII) with a compound of the formula:

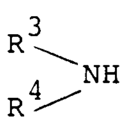

(VII)

wherein $R^3$ and $R^4$ are as defined above, in a solvent in the presence of a sulfonic acid.

The reaction of the compound of the formula (XIII) with the compound of the formula (VII) in a solvent in the presence of a sulfonic acid to yield the desired enamine derivative of the formula (I-2) can be carried out in the same manner as mentioned in the reaction of the compound of the formula (VI) with the compound of the formula (VII) in PROCESS A.

The compound of the formula (XIII) can be obtained by hydrolyzing the compound of the formula (XII) with an acid, and if required, followed by an alkali treatment, in the same manner as described in the hydrolysis of the compound of the formula (V), and if required, followed by the alkali treatment to yield the compound of the formula (VI) in PROCESS A.

The compound of the formula (XII) can be obtained by reacting the compound of the formula (XI) with the compound of the formula (IV-1) in a solvent in the presence of an alkoxide or an organometallic compound, in the same manner as described in the reaction of the compound of the formula (III) with the compound of the formula (IV-1) in a solvent in the presence of an alkoxide or an organometallic compound, that is, the Wittig reaction, in PROCESS A.

The compound of the formula (XI) can be obtained by formylating the compound of the formula (X) in dimethylformamide in the presence of $POCl_3$. This reaction is generally known as the Vilsmeier reaction. The reaction temperature is preferably 0° to 80°C. The reaction time is preferably 30 minutes to 5 hours.

The compound of the formula (X) can be obtained by reacting the compound of the formula (III) with the compound of the formula (IV-2) in a solvent in the presence of an alkoxide, or an organometallic compound in the same manner as described in the reaction of the compound of the formula (III) with the compound of the formula (IV) in a solvent in the presence of an alkoxide or an organometallic compound, that is, the Wittig reaction, in PROCESS A.

PROCESS D

The enamine derivative of the formula:

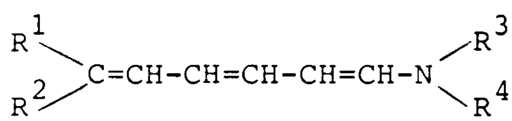

(I-3)

wherein $R^1$, $R^2$, $R^3$ and $R^4$ are as defined above, can be produced by reacting a compound of the formula:

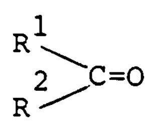

(III)

wherein $R^1$ and $R^2$ are as defined above, with a compound of the formula:

$$Ph_3\overset{+}{P}\overset{-}{I}CH_3 \qquad (IV\text{-}2)$$

wherein Ph is a phenyl group, in a solvent in the presence of an alkoxide or an organometallic compound to yield a compound of the formula:

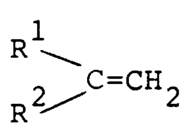

(X)

wherein $R^1$ and $R^2$ are as defined above, formylating the compound of the formula (X) in dimethylformamide in the presence of $POCl_3$ to yield a compound of the formula:

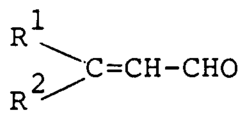

(XI)

wherein $R^1$ and $R^2$ are as defined above, reacting the compound of the formula (XI) with the compound of the formula (IV-2) in a solvent in the presence of an alkoxide or an organometallic compound to yield a compound of the formula:

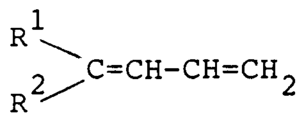

(XIV)

wherein $R^1$ and $R^2$ are as defined above, formylating the compound of the formula (XIV) in dimethylformamide in the presence of $POCl_3$ to yield a compound of the formula:

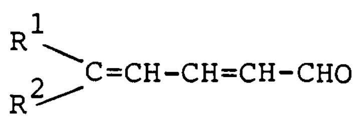

(XV)

wherein $R^1$ and $R^2$ are as defined above, reacting the compound of the formula (XV) with a compound of the formula:

$$Ph_3\overset{+}{P}\overset{-}{Cl}CH_2OCH_3 \qquad (IV\text{-}1)$$

wherein Ph is a phenyl group, in a solvent in the presence of an alkoxide or an organometallic compound to yield a compound of the formula:

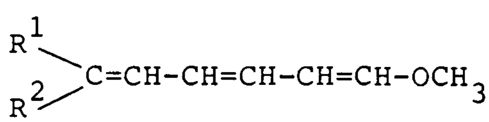

(XVI)

wherein $R^1$ and $R^2$ are as defined above, hydrolyzing the compound of the formula (XVI) with an acid, followed by an alkali treatment to yield a compound of the formula:

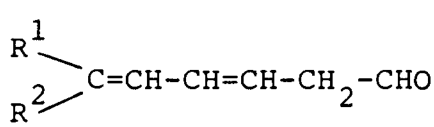

(XVII)

wherein $R^1$ and $R^2$ are as defined above, and reacting the compound of the formula (XVII) with a compound of the formula:

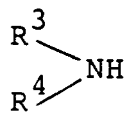

(VII)

wherein $R^3$ and $R^4$ are as defined above, in a solvent in the presence of a sulfonic acid.

The enamine derivative of the formula (I-3) can be produced by reacting the compound of the formula (XVII) with the compound of the formula (VII) in a solvent in the presence of a sulfonic acid in the same manner as described in the reaction of the compound of the formula (VI) with the compound of the formula (VII) in PROCESS A.

The compound of the formula (XVII) can be produced by hydrolyzing the compound of the formula (XVI) with an acid, and if required, followed by an alkali treatment in the same manner as described in the hydrolysis of the compound of the formula (V) with an acid, and if required, followed by an alkali treatment to yield the compound of the formula (VI) in PROCESS A.

The compound of the formula (XVI) can be obtained by reacting the compound of the formula (XV) with the compound of the formula (IV-1) in a solvent in the presence of an alkoxide or an organometallic compound in the same manner as described in the reaction of the compound of the formula (III) with the compound of the formula (IV) in a solvent in the presence of an alkoxide or an organometallic compound, that is, the Wittig reaction, in PROCESS A.

The compound of the formula (XV) can be obtained by formylating the compound of the formula (XIV) in dimethylformamide in the presence of $POCl_3$. This reaction is known as the Vilsmeier reaction. The reaction is preferably carried out at 0° to 80°C for 30 minutes to 5 hours.

The compound of the formula (XIV) can be obtained by reacting the compound of the formula (XI) with the compound of the formula (IV-2) in a solvent in the presence of an alkoxide or an organometallic compound in the same manner as described in the reaction of the compound of the formula (III) with the compound of the formula (IV) in a solvent in the presence of an alkoxide or an organometallic compound, that is, the Wittig reaction in PROCESS A.

The compound of the formula (XI) can be obtained by formylating the compound of the formula (X) in dimethylformamide in the presence of $POCl_3$. This reaction is known as the Vilsmeier reaction. The reaction is preferably carried out at 0° to 80°C for 30 minutes to 5 hours.

The compound of the formula (X) can be obtained by reacting the compound of the formula (III) with the compound of the formula (IV-2) in a solvent in the presence of an alkoxide or an organometallic compound in the same manner as described in the reaction of the compound of the formula (III) with the compound of the formula (IV) in a solvent in the presence of an alkoxide or an organometallic compound, that is, the Wittig reaction, in PROCESS A.

The enamine derivatives of the formula (I) are effective as a charge transport material in an electrophotographic plate.

The electrophotographic plate can be obtained by composing a photosensitive layer on an electroconductive substrate.

As the electroconductive substrate, there can be used paper or a plastic film subjected to electroconductive treatment, a plastic film laminated with a foil of metal such as aluminum, aluminum or the like electroconductive metal plate or drum, etc.

The photosensitive layer can be divided into two groups, one of which is a two layers type photoconductive layer comprising a charge generating layer and a charge transport layer wherein functions for generating charge and transporting charge are separated, and the other of which is one layer type photoconductive layer.

The two layers type photoconductive layer is explained below.

The charge generating layer includes an organic pigment which generates charge. As the organic pigment, there can be used azoxybenzenes, disazos, trisazos, benzimidazoles, polycyclic quinolines, indigoids, quinacridones, phthalocyanines, perylenes, methines, etc. These pigments are disclosed, for example, in U.S. Patent Nos. 3,898,084; 3,887,366; and 4,028,102; British Patent Nos. 1,370,197; 1,337,222; 1,337,224 and 1,402,967; Canadian Patent No. 1,007,095; and German Offenlegungsschrift 2,260,540. Particularly, τ-, τ'-, η- and η'-form metal-free phthalocyanines disclosed in U.S. Patent No. 4,619,879 and European Patent Publication No. 92255 have high sensitivity even to a long wavelength and are effective as an electrophotographic plate for printer mounting a diode laser. It is also possible to use other organic pigments generating charge carriers by exposure to light.

The charge generating layer includes a binder and/or additives such as a plasticizer, a fluidity-imparting agent, and, if necessary, a pin hole controlling agent, etc.

As the binder, there can be used silicone resins, polyamide resins, polyurethane resins, polymethyl methacrylate resins, polyacrylamide resins, etc. Further, it is also possible to use heat and/or photo curable resins. Any resins which can form a coating having electrical insulating properties by a conventional process can be used. The binder is used in the charge generating layer in an amount of 300 parts by weight or less per 100 parts by weight of the organic pigment. When the amount is more than 300 parts by weight, there is a tendency to lower electrophotographic porperties.

As the plasticizer, there can be used halogenated paraffins, dimethylnaphthalene, dibutyl phthalate, etc.

As the fluidity imparting agent, there can be used Modaflow (mfd. by Monsanto Chemical Co.), etc.

As the pin hole controlling agent, there can be used benzoin, dimethyl phthalate, etc.

These additives can be used in amounts of 5% by weight or less, respectively, based on the weight of the organic pigment.

The charge transport layer includes a charge transport material. As the charge transport material, there can be used the enamine derivative of the formula (I). It is possible to co-use other charge transport materials.

Examples of the other charge transport materials are 3-phenylcarbazole, 2-phenylindole, oxadiazole, oxatriazole, 1-phenyl-3-(4-diethylaminostyryl)-5-(4-diethylaminophenyl)pyrazoline, 2-phenyl-4-(4-diethylaminophenyl)-5-phenyloxazole, 2-(p-dimethylaminophenyl)-4-(p-dimethylamino)phenyl-5-(o-chlorophenyl)-1,3-oxazole, triphenylamine, imidazole, 2,7-dinitro-9-fluorenone, 2,4,7-trinitro-9-flurenone, 4H-indeno(1,2,6)thiophen-4-one, 1-bromopyrene, 2-phenylpyrene, poly-N-vinylcarbazole, polyvinylpyrene, polyvinylbenzothiophene, polyvinyl anthrathene, polyvinyl pyrazoline, etc. and derivatives thereof.

It is preferable to use the enamine derivative of the formula (I) in an amount of 10 to 100% by weight based on the total weight of the charge transport materials. When the amount of the enamine derivative of the formula (I) is too small, the photoresponse and durability are easily lowered. It is particularly preferable to use the enamine derivative of the formula (I) in an amount of 40 to 100% by weight based on the total weight of the charge transport materials.

The charge transport layer can also include a binder, additives such as a plastizer, a fluidity imprating agent, a pin hole controlling agent like the charge generating layer. The binder is preferably used in an amount of 400 parts by weight or less per 100 parts by weight of the charge transport materials so as not to lower electrophotographic properties. When only low-molecular weight charge transport materials such as the enamine derivative of the formula (I) are used, it is preferable to use the binder in an amount of 50% by weight or more considering film properties. The additives can preferably be used in an amount of 5% by weight or less based on the weight of the charge transport materials.

The two layers type photoconductive layer can be formed by laminating a charge generating layer and a charge transport layer in this order, or by laminating a charge transport layer and a charge generating layer in this order. It is also possible to take a sandwich structure wherein two charge generating layers sandwich a charge transport layer.

The thickness of the charge generating layer is preferably 0.001 to 10 µm, more preferably 0.2 to 5 µm. The thickness of the charge transport layer is preferably 5 to 50 µm, more preferably 8 to 20 µm. When the thickness of the charge generating layer is less than 0.001 µm, there is a tendency to lower the sensitivity, whereas when the thickness is more than 10 µm, there is a tendency to increase the residual potential. Further, when the thickness of the charge transport layer is less than 5 µm, there is a tendency to deteriorate the charging characteristics, whereas when the thickness is more than 50 µm, there is a tendency to lower the sensitivity.

The charge generating layer can be formed by a conventional vacuum deposition method in the case of using only the organic pigment mentioned above. It is also possible to form the charge generating layer by uniformly dissolving or dispersing an organic pigment, a binder and if necessary one or more additives in a solvent such as acetone, methyl ethyl ketone, tetrahydrofuran, toluene, xylene, methylene chloride, trichloroethane, or the like, coating the resulting solution or dispersion, and drying it.

Next, one layer type photoconductive layer is explained below.

The one layer type photoconductive layer includes a charge generating material and a charge transport material. As the charge generating material, there can be used the same organic pigments as used for the charge generating layer. As the charge transport material, there can be used the enamine derivative of the formula (I) alone or as a mixture with other charge transport material mentioned above. The amount of the enamine derivative of the formula (I) is 10 to 100% by weight based on the total weight of the charge transport materials.

The one layer type photoconductive layer may further contain a binder and one or more additives such as a plasticizer, a fluidity imparting agent, a pin hole controlling agent, etc., as in the case of the two layers type photoconductive layer. Among them, the role of the binder is important. The amount of the binder is preferably 80 to 450 parts by weight, more preferably 100 to 300 parts by weight, per 100 parts by weight of the charge transport material. If the amount of the binder is too small, the charging characteristics are deteriorated, whereas if the amount is too much, there is a tendency to lower the sensitivity. In this case, the charge generating material is used in an amount of preferably 0.1 to 20% by weight, more preferably 0.5 to 5% by weight based on the total weight of the charge transport material and the binder. If the amount of the charge generating material is too small, there is a tendency to lower the sensitivity, whereas if the amount is too much, there is a tendency to deteriorate the charging characteristics. The amounts of the additives such as the plasticizer, fluidity imparting agent, and pin hole controlling agent are 5% by weight or less in the one layer type photoconductive layer, respectively depending on purposes.

The thickness of the one layer type photoconductive layer is preferably 5 to 50 µm, more preferably 8 to 20 µm. If the thickness is less than 5 µm, there is a tendency to deteriorate the charging characteristics, whereas if the thickness is more than 50 µm, there is a tendency to lower the sensitivity.

The one layer type photoconductive layer can be formed by uniformly dissolving or dispersing a charge generating material, a charge transport material, a binder and other additives if necessary in a solvent in the same manner as described in the formation of the charge generating layer, coating it and drying it.

In this invention, it is also possible to employ as the photosensitive layer a multi layer type photoconductive layer wehrein the same charge transport layer as used in the two layers type photoconductive layer is formed immediately on or under or both sides of the one layer type photoconductive layer.

The electrophotographic plate of this invention may further contain a thin adhesive layer or a barrier layer just on the electroconductive substrate and between the electroconductive substrate and the photosensitive layer. Further, a surface protective layer may be formed on the surface of electrophotographic plate.

This invention is illustrated by way of the following Examples, wherein all percents are by weight unless otherwise specified. Further, the following materials were used therein.

(1) Organic Pigment Generating Charge

Phthalocyanine: α-form metal-free phthalocyanine ($H_2Pc$). (mfd. by BASF AG)

(2) Charge Transport Materials (Comparison)

Pyrazoline derivative: 1-phenyl-3-(p-diethylaminostyryl)-5-(p-diethylaminophenyl)pyrazoline (PYZ)

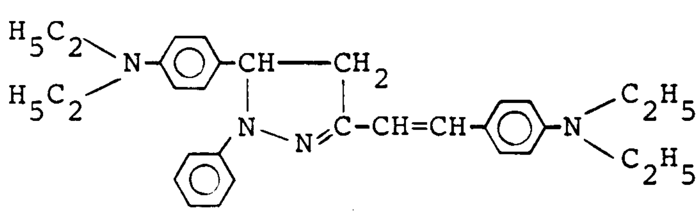

Oxazole derivative: 2-(p-dimethylamino)phenyl-4-(p-dimethylamino)phenyl-5-o-chlorophenyl-1,3-oxazole (OXZ)

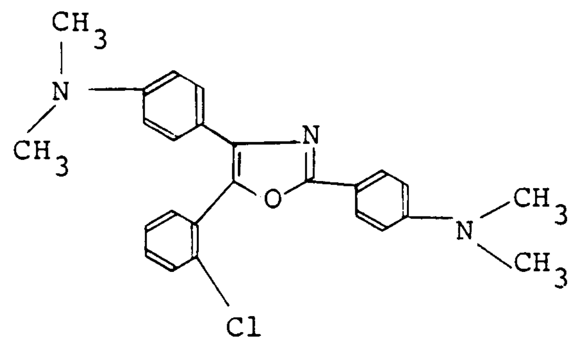

Hydrazone derivative: p-dimethylamino-(oethoxy)benzaldehydediphenylhydrazone (HYZ)

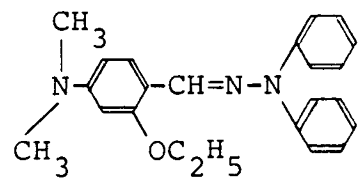

Enamine derivative: tetramethoxyphenylenamine (ENM)

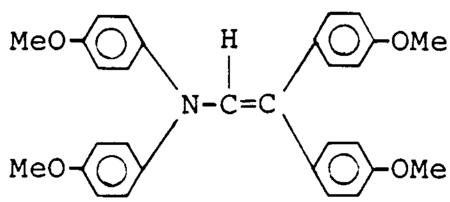

(3) Binder

Silicone varnish: KR-255 (solids content 50%) [mfd. by Sin-etsu Chemical Industry Co., Ltd.]
Polycarbonate resin: Iupilon S-2000 (solids content 50%) [mfd. by Mitsubishi Gas Chemical Co., Ltd.]

Examples 1 to 24, Comparative Examples 1 to 4

(a) In a ball mill (a pot having a diameter of about 10 cm, mfd. by Nippon Kagaku Kogyo Co., Ltd.), 2.5 g of $H_2Pc$, 5.0 g of silicone varnish and 92.5 g of methyl ethyl ketone were placed and kneaded for 8 hours. The resulting pigment dispersion was coated on an aluminum plate (an electroconductive layer) by using an applicator. After drying at 90 ° C for 15 minutes, a charge generating layer having a thickness of 1 μm was formed.

(b) A uniform coating composition for a charge transport layer comprising 10 g of a charge transport material as listed in Table 1, 10 g of Iupilon S-2000 as a binder, and 40 g of methylene chloride and 40 g of 1,1,2-trichloroethane as a solvent was prepared and immediately coated on the charge generating layer mentioned above so as to make the film thickness 15 μm after dried. After drying at 120 ° C for 2 hours, a charge transport layer is formed to give an electrophotographic plate.

Electrophotographic plates obtained in Examples 1 to 24 and Comparative Examples 1 to 4 were subjected to measuring of electrophotographic properties, print resolution, photoresponse and durability as follows.

(i) Electrophotographic properties

Using an electrophotographic plate cut to a size of 60 mm long and 70 mm wide, electrophotographic porperties were measured by using an electrostatic recording paper analyzer (SP-428 mfd. by Kawaguchi Electric Works Co., Ltd.).

Initial potential $V_o$ in Table 1 means a charging potential obtained by fixing a sample on a revolving disc of SP-428, revolving at a rate of 1000 r.p.m. and subjecting to a corona discharge at about 5 KV for 10 seconds.

Dark decay $V_K$ means a potential decay after allowed to stand in the dark for 30 seconds after the corona discharge ($V_K = V_{30} / V_o \times 100$, $V_{30}$ means the potential after 30 seconds).

Half decay exposure $E_{50}$ means a light amount necessary for making the potential a half after exposing to a white light of 10 lux after measuring the dark decay.

Residual potential $V_R$ means a potential after exposure to the white light for 60 seconds.

(ii) Print resolution

After the measurement of electrophotographic properties, the surface of electrophotographic plate was charged with corona discharge so as to make the surface potential -600 V to -700 V. Then, using as an original image an Electrophotographic Society Chart No. 1-T, exposure to a light image was conducted at 100 lux•s, followed by development with positively charged toner. The resulting toner image was transferred to a white sheet of paper, followed by fixation to give a test image. The print resolution was evaluated by the distinguishable number of fine lines per 1 mm. In each test, the same toner, and the same transfer and fixation methods were employed.

In Table 1, the electrophotographic properties and print resolution immediately after the production of electrophotographic plates (initial values) were measured and listed. Further, after repeating 10000 times of corona charging (surface potential -1000 V ±100 V) and removal of charging (exposure to light of a wavelength of 500 nm : a light amount 50 $mJ/m^2$) on each sample of electrophotographic plate (60 mm x 70 mm), the electrophotographic properties and print resolution were measured and listed in Table 1.

(iii) Photoresponse

The electrophotographic plates obtained in Examples 1 to 24 and Comparative Examples 1 to 4 were subjected to photoresponse test using a light decay measuring apparatus (Cincia 30, mfd. by Midoriya Denki K.K.). The surface potential of photosensitive layer was previously adjusted to -700 V by corona charging.

In the case of the samples of Examples 1 to 14 and Comparative Examples 1 to 4, the following method was employed.

A sample was then subjected to exposure to a light having a wavelength of 660 nm obtained by filtering a light from a halogen lamp with a diffraction grating for 50 msec, and then decay of surface potential of the photosensitive layer with the lapse of time was measured and plotted in graphs. The total exposure light energy was 20 $mJ/m^2$.

The results are shown in Figs. 1 to 3. In Fig. 1, the curves 1 to 5 correspond to Examples 1 to 5. In Fig. 2, the curves 6 to 10 correspond to Examples 6 to 10. In Figs. 1 and 2, the curves 15 to 18 correspond to Comparative Examples 1 to 4. In Fig. 3, the curves 11 to 14 correspond to Examples 11 to 14.

In the case of the samples of Examples 15 to 24 and Comparative Examples 1 to 4, the following method was employed.

A sample was then subjected to exposure to a light having a wavelength of 600 nm obtained from light emission diode (LED) for 1 msec, and then a time (t 1/2) necessary for decaying the surface potential of the photosensitive layer to a half (-350 V) of the initial value was measured. The total exposure light energy was 50 $mJ/m^2$.

The results are shown in Table 2.

Table 1

| Example No. | Charge transport material | | Electrophotographic properties and print resolution (initial values) | | | | |
|---|---|---|---|---|---|---|---|
| | Kind of compound | Compound No. | $V_O$ (-V) | $V_K$ (%) | $E_{50}$ (lux·s) | $V_R$ (-V) | Print resolution (no./mm) |
| Example 1 | Enamine derivative | 1 | 1050 | 75 | 1.3 | 0 | 12.5 |
| " 2 | " | 2 | 1040 | 75 | 1.5 | 0 | 12.5 |
| " 3 | " | 3 | 1000 | 72 | 1.2 | 0 | 12.5 |
| " 4 | " | 4 | 1020 | 74 | 1.2 | 0 | 12.5 |
| " 5 | " | 5 | 1080 | 76 | 1.5 | 0 | 12.5 |
| " 6 | " | 6 | 1000 | 77 | 1.6 | 0 | 12.5 |
| " 7 | " | 7 | 1000 | 77 | 1.4 | 0 | 12.5 |
| " 8 | " | 8 | 1150 | 78 | 1.7 | 0 | 12.5 |
| " 9 | " | 9 | 1120 | 77 | 1.9 | 0 | 12.5 |
| " 10 | " | 10 | 1100 | 80 | 1.7 | 0 | 12.5 |
| " 11 | " | 11 | 950 | 70 | 0.8 | 0 | 12.5 |
| " 12 | " | 12 | 1020 | 75 | 1.5 | 0 | 12.5 |
| " 13 | " | 13 | 1040 | 75 | 1.4 | 0 | 12.5 |
| " 14 | " | 14 | 1010 | 76 | 1.2 | 0 | 12.5 |
| " 15 | " | 15 | 1030 | 75 | 1.2 | 0 | 12.5 |
| " 16 | " | 20 | 1070 | 80 | 1.6 | 0 | 12.5 |

- Cont'd -

Table 1 (Cont'd)

| Electrophotographic properties and print resolution | | | | |
|---|---|---|---|---|
| $V_O$ (-V) | $V_K$ (%) | $E_{50}$ (lux·s) | $V_R$ (-V) | Print resolution (no./mm) |
| 1060 | 75 | 1.3 | 0 | 12.5 |
| 1050 | 75 | 1.4 | 0 | 12.5 |
| 1040 | 73 | 1.2 | 0 | 10.0 |
| 1000 | 74 | 1.2 | 0 | 10.0 |
| 1080 | 76 | 1.5 | 0 | 10.0 |
| 1110 | 77 | 1.5 | 0 | 10.0 |
| 1120 | 78 | 1.4 | 0 | 12.5 |
| 1150 | 78 | 1.7 | 0 | 12.5 |
| 1120 | 78 | 1.8 | 0 | 12.5 |
| 1220 | 81 | 1.7 | 0 | 12.5 |
| 980 | 70 | 0.8 | 0 | 10.0 |
| 1030 | 76 | 1.5 | 0 | 12.5 |
| 1070 | 76 | 1.4 | 0 | 12.5 |
| 1010 | 76 | 1.2 | 0 | 12.5 |
| 1040 | 75 | 1.4 | 0 | 12.5 |
| 1050 | 76 | 1.5 | 0 | 12.5 |

- Cont'd -

Table 1 (Cont'd)

| | | | | | | | |
|---|---|---|---|---|---|---|---|
| Example 17 | Enamine derivative | 24 | 1010 | 72 | 1.1 | 0 | 12.5 |
| " 18 | " | 28 | 1000 | 76 | 1.5 | 0 | 12.5 |
| " 19 | " | 30 | 1080 | 73 | 1.3 | 0 | 12.5 |
| " 20 | " | 35 | 1020 | 78 | 1.2 | 0 | 12.5 |
| " 21 | " | 42 | 1140 | 72 | 1.4 | 0 | 12.5 |
| " 22 | " | 51 | 1060 | 77 | 1.4 | 0 | 12.5 |
| " 23 | " | 58 | 1090 | 73 | 1.3 | 0 | 12.5 |
| " 24 | " | 61 | 1050 | 74 | 1.6 | 0 | 12.5 |
| Comparative Example 1 | Pyrazoline derivative | PYZ | 880 | 60 | 1.8 | 0 | 12.5 |
| " 2 | Oxazole derivative | OXZ | 900 | 62 | 2.0 | 0 | 12.5 |
| " 3 | Hydrazone derivative | HYZ | 1100 | 75 | 2.4 | 0 | 12.5 |
| " 4 | Enamine derivative | ENM | 1040 | 74 | 2.2 | 0 | 12.5 |

- Cont'd -

Table 1 (Cont'd)

| | | | | |
|---|---|---|---|---|
| 1090 | 71 | 1.2 | 0 | 10.0 |
| 1090 | 79 | 1.3 | 0 | 12.5 |
| 1180 | 77 | 1.2 | 0 | 10.0 |
| 1150 | 72 | 1.5 | 0 | 10.0 |
| 1120 | 74 | 1.5 | 0 | 12.5 |
| 1090 | 74 | 1.6 | 0 | 12.5 |
| 1140 | 76 | 1.8 | 0 | 10.0 |
| 1080 | 78 | 1.8 | 0 | 10.0 |
| 720 | 52 | 1.6 | 0 | 1.6 |
| 860 | 58 | 1.9 | 0 | 3.2 |
| 1150 | 76 | 2.6 | 50 | 8.0 |
| 1100 | 75 | 2.3 | 20 | 10.0 |

Table 2

| Example No. | Charge transport material | | Photo-response t 1/2(msec) |
|---|---|---|---|
| | Kind of compound | Compound No. | |
| Example 15 | Enamine derivative | 15 | 7 |
| " 16 | " | 20 | 18 |
| " 17 | " | 24 | 9 |
| " 18 | " | 28 | 25 |
| " 19 | " | 30 | 21 |
| " 20 | " | 35 | 13 |
| " 21 | " | 42 | 19 |
| " 22 | " | 51 | 18 |
| " 23 | " | 58 | 10 |
| " 24 | " | 61 | 20 |
| Comparative Example 1 | Pyrazoline derivative | PYZ | 42 |
| " 2 | Oxazole derivative | OXZ | 55 |
| " 3 | Hydrazone derivative | HYZ | 93 |
| " 4 | Enamine derivative | ENM | 68 |

As is clear from the results shown in Table 1, the electrophotographic plates obtained in Examples 1 to 24 have $V_o$ of 1000 V or more in the absolute values and thus are excellent in charging characteristics, have $E_{50}$ of less than 2.0 lux•s and are excellent in the sensitivity and small in the dark decay. Further, even after repeating the corona charging and removal of charging 10,000 times, the electrophotographic properties are not lowered and the print resolution was 10.0 to 12.5 number/mm, which values are almost the same as those of initial values: this means that durability is also excellent.

In contrast, the electrophotographic plates obtained in Comparative Examples 1 and 2 are low in $V_o$ and $V_K$, deteriorated in charging properties, and large in the dark decay. Further, after repeating 10000 times of corona charging and removal of charging, not only these properties are lowered but also the print resolution is remarkably lowered to deteriorate the durability. The electrophotographic plates obtained in Comparative Examples 3 and 4 are inferior in the sensitivity, and the residual potential is increased after repeating 10000 times of the corona charging and removal of charging. In addition, the electrophotographic plate obtained in Comparative Example 3 is considerably lowered in the resolving power and inferior in the durability.

Further, as is clear from the results shown in Figs. 1 to 3 and Table 2, the electrophotographic plates obtained in Examples 1 to 24 are remarkably superior to those obtained in Comparative Examples 2 to 4 in the photoresponse. The electrophotographic plate obtained in Comparative Example 1 shows almost the same photoresponse as those obtained in Examples 1 to 24, but is inferior in the charging characteristics, large in the dark decay and inferior in the durability.

As mentioned above, the electrophotographic plate obtained in Examples 1 to 24 are excellent in the sensitivity, photoresponse and durability.

The enamine derivatives (Compound Nos. 11 to 14) used in Examples 11 to 14 were synthesized as follows.

Synthesis Example 1

[Synthesis of 1,1-bis(p-diethylaminophenyl)-methoxyethylene (Compound (a))]

To 105 ml of dry tetrahydrofuran (THF) containing 15 g of methoxymethyltriphenylphosphonium chloride, 27 ml of n-butyllithium (n-hexane solution 1.6 mole/l) was added dropwise gradually in a nitrogen stream at 5°C with stirring. After dissolving the starting material, the stirring was continued at that temperature for 2.5 hours. After adding 140 ml of THF containing 14 g of 4′-bisdiethylaminobenzophenone dropwise, the reaction was carried out at room temperature for 3 hours. Then, the reaction mixture was poured into 500 ml of water, followed by extraction with benzene. After dried, the benzene was removed by distillation. The residue was purified with a silica gel column to yield 8.1 g of compound (a) in 53% yield.

NMR ($CDCl_3$): δ = 1.1 (t 12H $-CH_2-CH_3$)
3.3 (q 8H $-CH_2-CH_3$)
3.6 (s 3H $C=CH-OCH_3$)
6.1 (s 1H $C=CH-OCH_3$)
6.4-7.2 (m 8H arom.)

Melting point (oily matter):

NMR spectrum of Compound (a) is shown in Fig. 4.

Synthesis Example 2

[Synthesis of 1,1-bis(p-diethylaminophenyl)acetaldehyde (Compound (b))]

Compound (a) in an amount of 7.5 g was dissolved in 60 ml of 60% perchloric acid aqueous solution at 20°C or lower and stirred for 20 minutes. Then the solution was made alkaline with a sodium hydroxide aqueous solution under cooling. The deposited sirupy material was extracted with benzene, followed by drying. After removal of the benzene by distillation, the residue was recrystallized from methanol to give 4.8 g of compound (b) in yield of 67%.

NMR ($CDCl_3$): δ = 1.1 (t 12H $-CH_2-CH_3$)
3.3 (q 8H $-CH_2-CH_3$)
4.6 (d 1H $=CH-CHO$)
9.7 (d 1H $=CH-CHO$)
6.5-7.0 (m 8H arom.)

IR (KBr): 1724 $cm^{-1}$ (-CHO)

Melting point: 74.5-76.5°C

NMR spectrum of Compound (b) is shown in Fig. 5, and IR spectrum of compound (b) is shown in Fig. 6.

Synthesis Example 3

[Synthesis of 1,1-bis(p-dimethylaminophenyl)-methoxyethylene (Compound (c))]

Compound (c) was synthesized from 4,4′-bis(p-dimethylamino)benzophenone in the same manner as described in Synthesis Example 1 in 66% yield.

NMR ($CDCl_3$): δ = 2.9 (s 12H $-N-CH_3$)
3.6 (s 3H $C=CH-O-CH_3$)
6.2 (s 1H $C=CH-O-CH_3$)
6.5-7.3 (m 8H arom.)

Melting point: 89.5-91°C

NMR spectrum of Compound (c) is shown in Fig. 7.

Synthesis Example 4

[Synthesis of 1,1-bis(p-dimethylaminophenyl)acetaldehyde (Compound (d))]

Compound (d) was synthesized from Compound (c) in the same manner as described in Synthesis Example 2 in 62% yield.

NMR ($CDCl_3$): δ = 2.9 (s 12H -N-$CH_3$)
4.7 (d 1H = $\underline{CH}$-CHO)
9.8 (d 1H = CH-$\underline{CHO}$)
6.6-7.6 (m 8H arom.)
IR (KBr): 1722 $cm^{-1}$ (-CHO)
Melting point: 72-75 °C

NMR spectrum of Compound (d) is shown in Fig. 8, and IR spectrum of Compound (d) is shown in Fig. 9.

Synthesis Example 5

[Synthesis of 1,1-bis(p-methoxyphenyl)acetaldehyde (Compound (e))]

In 3 liters of dry ethanol, 75 g of 4,4′-bis-methoxybenzoin and 21 g of sodium borohydride were refluxed for 2 hours. After adding 250 ml of concentrated hydrochloric acid to the reaction mixture, the resulting mixture was poured into 1300 ml of ice water, followed by filtration of deposited crystals. After recrystallized from 95% ethanol, there was obtained 57 g of 1,2-di(p-methoxyphenyl)ethane-1,2-diol, which was then added to 570 ml of 80% acetic acid and stirred at 90 °C for 50 minutes. Then, the reaction mixture was poured into 4 liters of ice water and deposited crystals were filtered. After drying, the crystals were recrystallized from isopropyl ether to give 40 g of Compound (e) in 69% yield.

NMR ($CDCl_3$): δ = 3.8 (s 6H -$OCH_3$)
4.7 (d 1H = $\underline{CH}$-CHO)
9.8 (d 1H = CH-$\underline{CHO}$)
6.7-7.2 (m 8H arom.)
IR (KBr): 1722 $cm^{-1}$ (-CHO)
Melting point: 101-103 °C

NMR spectrum of Compound (e) is shown in Fig. 10, and IR spectrum of Compound (e) is shown in Fig. 11.

Synthesis Example 6

[Synthesis of 1,1-bis(p-diethylaminophenyl)-2-[N,N-bis(p-methoxyphenyl)amino]ethylene: Compound 11]

Compound (b) in an amount of 19.6 g and 16.1 g of bis(p-anisyl)amine were dissolved in 350 ml of dry toluene. Then, 30 g of molecular sieves 4A and 0.2 g of p-toluenesulfonic acid were added thereto, followed by reflux for 2.5 hours. After cooling to room temperature, the reaction mixture was filtered. After removing the solvent by distillation, the resulting crystals were recrystallized from isopropyl ether (IPE) to give Compound 11 having a melting point of 120-122 °C in an amount of 15.8 g in 46% yield.

Maximum absorption wavelength: 308 nm ($\varepsilon_{max}$ $3.50 \times 10^4$) (methanol)

Elementary analysis:

| | C (%) | H (%) | N (%) |
|---|---|---|---|
| Calcd. | 78.18 | 6.77 | 8.55 |
| Found | 78.85 | 6.82 | 8.50 |

Synthesis Example 7

[Synthesis of 1,1-bis(p-methoxyphenyl)-2-(9-carbazolyl)aminoethylene: Compound 12]

A crude product of Compound 12 was synthesized from Compound (e) and carbazole in the same manner as described in Synthesis Example 6. After purifying with a silica gel column, recrystallization was conducted from IPE to give Compound 12 having a melting point of 201.5-202.5°C in 52% yield.

Maximum absorption wavelength: 252 nm ($\varepsilon_{max}$ 4.50x$10^4$) (methanol)

Elementary analysis:

| | C (%) | H (%) | N (%) |
|---|---|---|---|
| Calcd. | 82.94 | 5.72 | 3.45 |
| Found | 82.96 | 5.76 | 3.41 |

Synthesis Example 8

[Synthesis of 1,1-bis(p-dimethylaminophenyl)-2-(N,N-diphenylamino)ethylene: Compound 13]

A crude product of compound 13 was synthesized from Compound (d) and diphenylamine in the same manner as described in Synthesis Example 6. After purifying with a silica gel column, recrystallization was conducted from IPE to give Compound 13 having a melting point of 141.5-143°C in 54% yield.

Maximum absorption wavelength: 301 nm ($\varepsilon_{max}$ 3.51x$10^4$) (methanol)

Elementary analysis:

| | C (%) | H (%) | N (%) |
|---|---|---|---|
| Calcd. | 83.10 | 7.21 | 9.69 |
| Found | 83.29 | 7.26 | 9.70 |

Synthesis Example 9

[Synthesis of 1,1-bis(p-diethylaminophenyl)-2-(N,N-diphenylamino)ethylene: Compound 14]

A crude product of Compound 14 was synthesized from Compound (b) and diphenylamine in the same manner as described in Synthesis Example 6. After purifying with a silica gel column, recrystallization was conducted from IPE to give Compound 14 having a melting point of 123-124°C in 39% yield.

Maximum absorption wavelength: 307 nm ($\varepsilon_{max}$ 3.94x$10^4$) (methanol)

Elementary analysis:

| | C (%) | H (%) | N (%) |
|---|---|---|---|
| Calcd. | 83.39 | 8.03 | 8.58 |
| Found | 83.49 | 8.10 | 8.51 |

Compounds 11 to 14 obtained in Synthesis Examples 6 to 9 have the following belonging in NMR Spectra:

| Belonging | Compound 11 | Compound 12 | Compound 13 | Compound 14 |
|---|---|---|---|---|
| $-OCH_3$ | | 3.6 s 3H | | |
| $-OCH_3$ | | 3.8 s 3H | | |
| $-OCH_3$ | 3.7 s 6H | | | |
| $-NCH_3$ | | | 2.8 s 6H | |
| $-NCH_3$ | | | 2.9 s 6H | |
| $-NCH_2CH_3$ | 1.05 t 6H | | | 1.05 t 6H |
| $-NCH_2CH_3$ | 1.10 t 6H | | | 1.15 t 6H |
| $-NCH_2CH_3$ | 3.05-3.50 | | | 3.2 q 4H |
| $-NCH_2CH_3$ | m 8H | | | 3.3 q 4H |
| arom. H | | 6.3-8.1 | 6.2-7.3 | 6.2-7.3 |
| | | m 17H | m 19H | m 19H |
| | Containing =CH- | Containing =CH- | Containing =CH- | Containing =CH- |

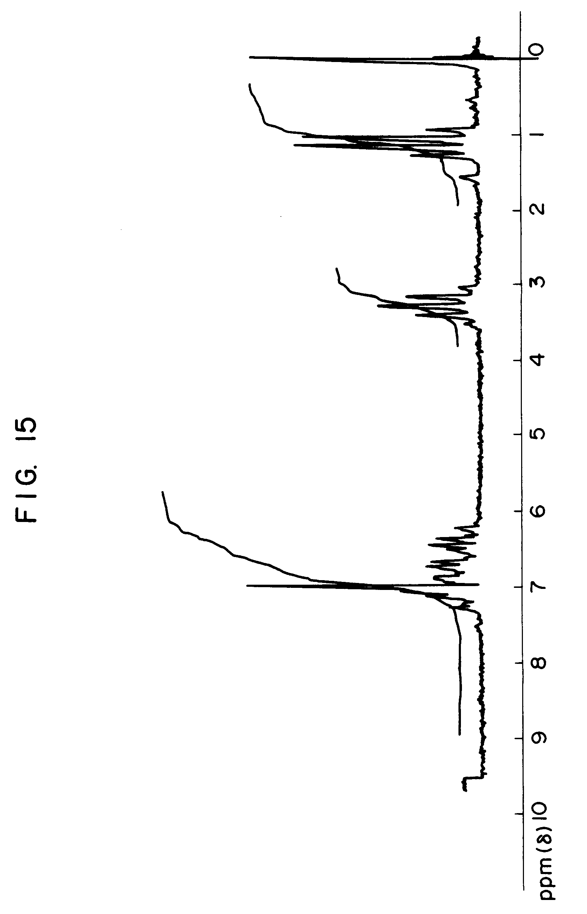

NMR spectra of Compounds 11 to 14 are shown in Figs. 12 to 15, respectively.

As mentioned above, electrophotographic plates obtained by using the enamine derivatives of the formula (I) are excellent in the sensitivity, photoresponse and durability.

**Claims**

**1.** An enamine derivative of the formula:

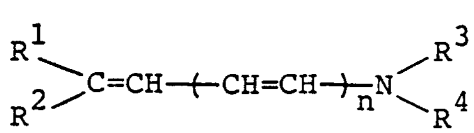 (I)

wherein

(i) in the case of n being zero,

(a) $R^1$ is a group of the formula:

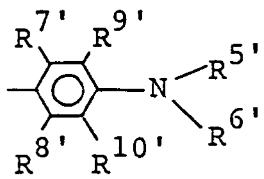

or

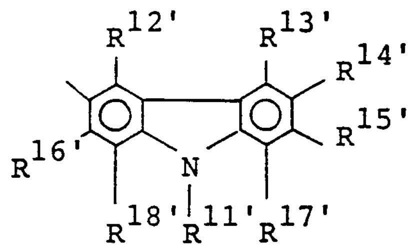

in which $R^{5'}$ and $R^{6'}$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{7'}$ and $R^{8'}$ are independently hydrogen, an alkyl group having 1 to 9 carbon atoms, or an alkoxy group having 1 to 4 carbon atoms; $R^{9'}$ and $R^{10'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group, or a cyano group; $R^{11'}$ is an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{12'}$, $R^{13'}$, $R^{14'}$, $R^{15'}$ and $R^{16'}$ are independently hydrogen, an alkyl group having 1 to 9 carbon atoms, or an alkoxy group having 1 to 4 carbon atoms; $R^{17'}$ and $R^{18'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group; $R^2$, $R^3$ and $R^4$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group, and further $R^2$ may be hydrogen, and $R^3$ and $R^4$ may together form a group of the formula:

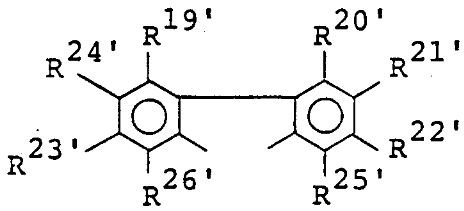

in which $R^{19'}$, $R^{20'}$, $R^{21'}$, $R^{22'}$, $R^{23'}$ and $R^{24'}$ are independently hydrogen, an alkyl group having 1 to 9 carbon atoms or an alkoxy group having 1 to 4 carbon atoms; $R^{25'}$ and $R^{26'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group, or

(b) $R^1$ is a group of the formula:

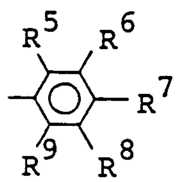

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain non-substituted or substituted alkyl groups having 1 to 9 carbon atoms, said substituted alkyl groups including alkyl groups substituted with one or more halogen atoms, hydroxyl groups, cyano groups, nitro groups, alkylamino groups, arylamino groups, aryl groups, and aryloxy groups, an amino group substituted with one or two non-substituted or substituted aralkyl groups, said substituted aralkyl groups including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two non-substituted or substituted heterocyclic ring groups, said substituted heterocyclic ring groups including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and

aralkylamino groups, a hydroxyl group, a nitro group or a cyano group; $R^2$ is hydrogen, an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; and $R^3$ and $R^4$ together form a group of the formula:

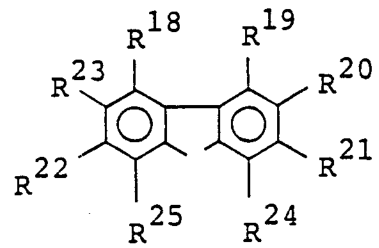

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group; and

(ii) in the case of n being an integer of 1 or 2, $R^1$ is a group of the formula:

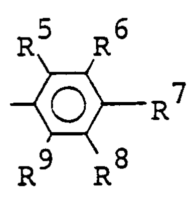

or

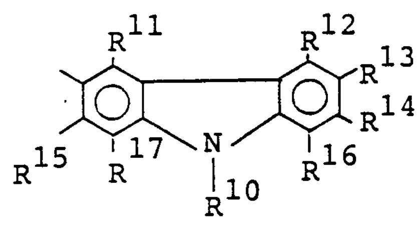

in which $R^5$ through $R^9$ are as defined above; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl

groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocylclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, an alkoxy group having 1 to 4 carbon atoms, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups,; $R^{16}$ and $R^{17}$ are independently hydrogen, a halogen atom, a straight-or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, and further $R^2$ may be a hydrogen atom, and $R^3$ and $R^4$ may together form a group of the formula:

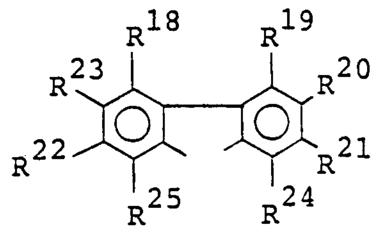

(II)

in which $R^{18}$ through $R^{25}$ are as defined above.

2. An enamine derivative according to claim 1, which is represented by the formula:

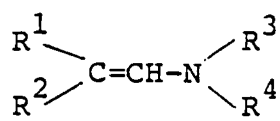

(I-1)

wherein $R^1$ is a group of the formula:

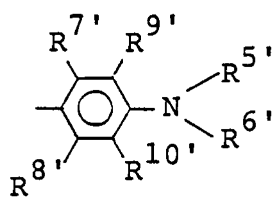

or

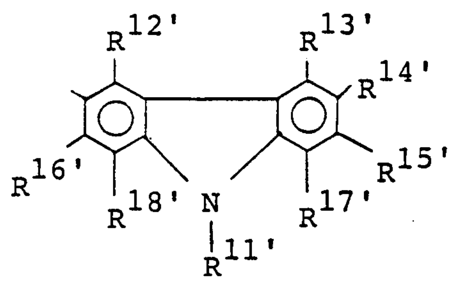

in which $R^{5'}$ and $R^{6'}$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{7'}$ and $R^{8'}$ are independently hydrogen, an alkyl group having 1 to 9 carbon atoms, or an alkoxy group having 1 to 4 carbon atoms; $R^{9'}$ and $R^{10'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group, or a cyano group; $R^{11'}$ is an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{12'}$, $R^{13'}$, $R^{14'}$, $R^{15'}$ and $R^{16'}$ are independently hydrogen, an alkyl group having 1 to 9 carbon atoms, or an alkoxy group having 1 to 4 carbon atoms; $R^{17'}$ and $R^{18'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group

or a cyano group; $R^2$, $R^3$ and $R^4$ are independently an alkyl group having 1 to 9 carbon atoms, an aryl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group, and further $R^2$ may be hydrogen, and $R^3$ and $R^4$ may together form a group of the formula:

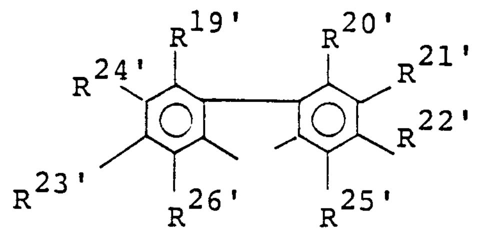

in which $R^{19'}$, $R^{20'}$, $R^{21'}$, $R^{22'}$, $R^{23'}$ and $R^{24'}$ are independently hydrogen, an alkyl group having 1 to 9 carbon atoms or an alkoxy group having 1 to 4 carbon atoms; $R^{25'}$ and $R^{26'}$ are independently hydrogen, a halogen atom, an alkyl group having 1 to 9 carbon atoms, an alkoxy group having 1 to 4 carbon atoms, an aralkyl group which may have one or more substituents selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group.

**3.** An enamine derivative according to claim 1, which is represented by the formula:

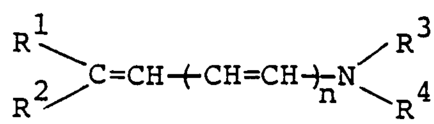

(I)

wherein $R^1$ is a group of the formula:

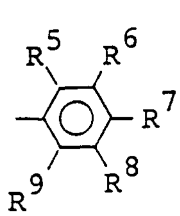

or

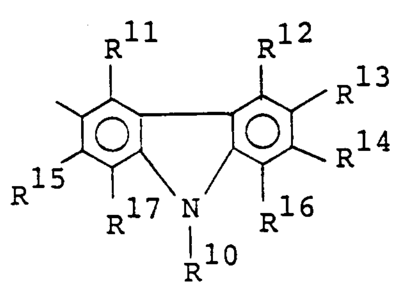

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups,

amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain alkyl groups having 1 to 9 carbon atoms, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$, are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{16}$ and $R^{17}$ are independently hydrogen, a straight-or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and

aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, and further $R^2$ may be a hydrogen atom, and $R^3$ and $R^4$ may together form a group of the formula:

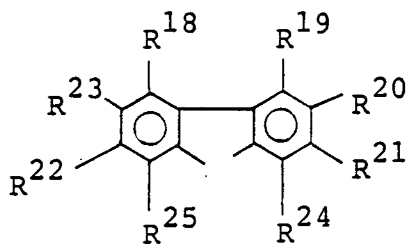

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group; and n is an integer of 1 or 2.

**4.** A process for producing an enamine derivative of the formula:

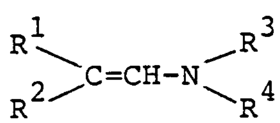

(I-1)

wherein

(a) $R^1$ is a group of the formula:

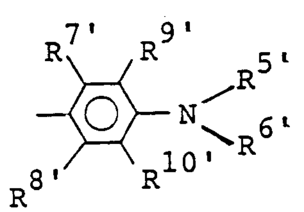

or

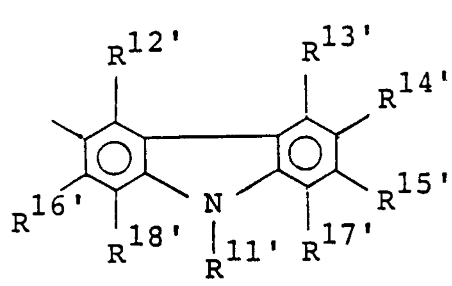

in which $R^{5'}$ and $R^{6'}$ are independently an alkyl group having 1 to 9 carbon atoms, an aryl group which may have on or more substituents, selected from one or more halogen atoms, hydroxyl groups, cyano groups, nitro groups, alkylamino groups, amino groups, alkyl groups, alkoxy groups,

acetyl groups, acetoxy groups, arylamino groups, aralkylamino groups, aryl groups, and aryloxy groups, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{7'}$ and $R^{8'}$ are independently hydrogen, an alkyl group having 1 to 9 carbon atoms, or an alkoxy group having 1 to 4 carbon atoms; $R^{9'}$ and $R^{10'}$ are independently hydrogen, a halogen atom, an alkyl group having 1 to 9 carbon atoms, an alkoxy group having 1 to 4 carbon atoms, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group, or a cyano group; $R^{11'}$ is an alkyl group having 1 to 9 carbon atoms, an aryl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; $R^{12'}$, $R^{13'}$, $R^{14'}$; $R^{15'}$ and $R^{16'}$ are independently hydrogen, an alkyl group having 1 to 9 carbon atoms, or an alkoxy group having 1 to 4 carbon atoms; $R^{17'}$ and $R^{18'}$ are independently hydrogen, a halogen atom, an alkyl group having 1 to 9 carbon atoms, an alkoxy group having 1 to 4 carbon atoms, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group; $R^2$, $R^3$ and $R^4$ are independently an alkyl group having 1 to 9 carbon atoms, an aryl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group, and further $R^2$ may be hydrogen, and $R^3$ and $R^4$ may together form a group of the formula:

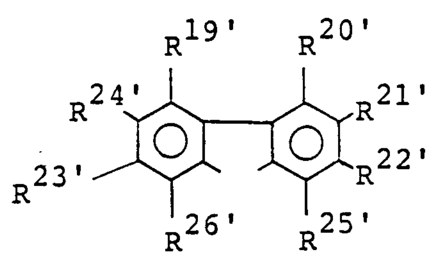

in which $R^{19'}$, $R^{20'}$, $R^{21'}$, $R^{22'}$, $R^{23'}$ and $R^{24'}$ are independently hydrogen, an alkyl group having 1 to 9 carbon atoms or an alkoxy group having 1 to 4 carbon atoms; $R^{25'}$ and $R^{26'}$ are independently hydrogen, a halogen atom, an alkyl group having 1 to 9 carbon atoms, an alkoxy group having 1 to 4 carbon atoms, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a hydroxyl group, a nitro group or a cyano group, or

(b) $R^1$ is a group of the formula:

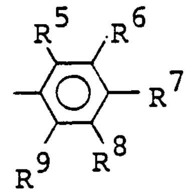

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain non-substituted or substituted alkyl groups having 1 to 9 carbon atoms, said substituted alkyl groups including alkyl groups substituted with one or more halogen atoms, hydroxyl groups, cyano groups, nitro groups, alkylamino groups, arylamino groups, aryl groups, and aryloxy groups, an amino group substituted with one or two non-substituted or substituted aralkyl groups, said substituted aralkyl groups including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two nonsubstituted or substituted heterocyclic ring groups, said substituted heterocyclic ring groups including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, a hydroxyl group, a nitro group or a cyano group; $R^2$ is hydrogen, an alkyl group having 1 to 9 carbon atoms, an aryl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an aralkyl group which may have one or more substituents, selected from one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a heterocyclic ring group; and $R^3$ and $R^4$ together form a group of the formula:

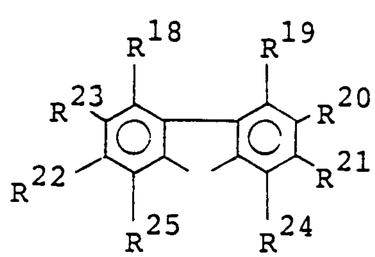

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group; which comprises reacting a compound of the formula:

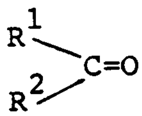

(III)

wherein $R^1$ and $R^2$ are as defined above, with a compound of the formula:

$$Ph_3\overset{+}{P}\overset{-}{Cl}CH_2OCH_3 \qquad \text{(IV-1)}$$

wherein Ph is a phenyl group, in a solvent in the presence of an alkoxide or an organometallic compound to yield a compound of the formula:

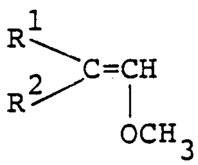

(V)

wherein $R^1$ and $R^2$ are as defined above, hydrolyzing the compound of the formula (V) with an acid, followed by alkali treatment to yield a compound of the formula:

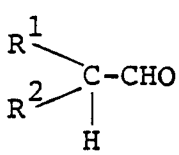

(VI)

wherein $R^1$ and $R^2$ are as defined above, and reacting the compound of the formula (VI) with a compound of the formula:

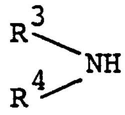

(VII)

wherein $R^3$ and $R^4$ are as defined above, in a solvent in the presence of a sulfonic acid.

**5.** A process for producing an enamine derivative of the formula:

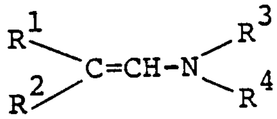

(I-1)

wherein $R^1$ through $R^4$ are as defined in claim 4, which comprises reducing a compound of the formula:

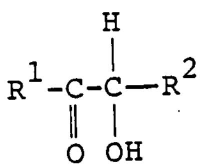

(VIII)

wherein $R^1$ and $R^2$ are as defined above, with a metal hydride, followed by an acid treatment and an alkali treatment to yield a compound of the formula:

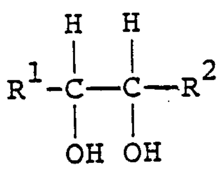

(IX)

wherein $R^1$ and $R^2$ are as defined above, reacting the compound of the formula (IX) with an acid to yield a compound of the formula:

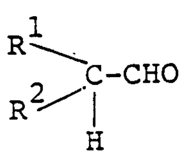

(VI)

wherein $R^1$ and $R^2$ are as defined above, and reacting the compound of the formula (VI) with a compound of the formula:

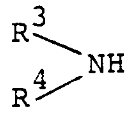

(VII)

wherein $R^3$ and $R^4$ are as defined above, in a solvent in the presence of a sulfonic acid.

**6.** A process for producing an enamine derivative of the formula:

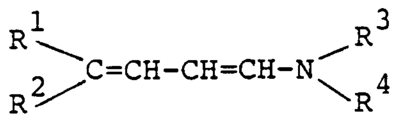

(I-2)

wherein $R^1$ is a group of the formula:

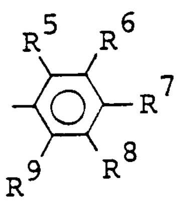

or

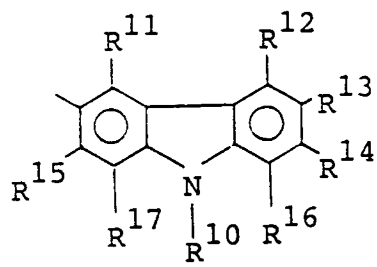

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain, non-substituted or substituted alkyl groups having 1 to 9 carbon atoms, said substituted alkyl groups including alkyl groups substituted with one or more halogen atoms, hydroxyl groups, cyano groups, nitro groups, alkylamino groups, arylamino groups, aryl groups, and aryloxy groups, an amino group substituted with one or two non-substituted or substituted aralkyl groups, said substituted aralkyl groups including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two non-substituted or substituted heterocyclic ring groups, said substituted heterocyclic ring groups including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and

aralkylamino groups, a hydroxyl group, a nitro group or a cyano group; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, an alkoxy group having 1 to 4 carbon atoms, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{16}$ and $R^{17}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, and further $R^2$ may be a hydrogen atom, and $R^3$ and $R^4$ may together form a group of the formula:

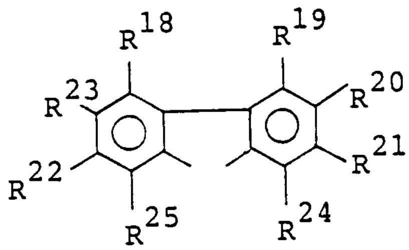

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; and $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group, which comprises reacting a compound of the formula:

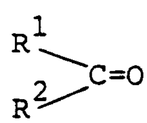

(III)

wherein $R^1$ and $R^2$ are as defined above, with a compound of the formula:

$$Ph_3\overset{+-}{PI}CH_3 \qquad \text{(IV-2)}$$

wherein Ph is a phenyl group, in a solvent in the presence of an alkoxide or an organometallic compound to yield a compound of the formula:

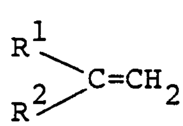

(X)

wherein $R^1$ and $R^2$ are as defined above, formylating the compound of formula (X) in dimethylformamide in the presence of $POCl_3$ to yield a compound of the formula:

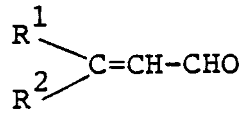

(XI)

wherein $R^1$ and $R^2$ are as defined above, reacting the compound of formula (XI) with a compound of the formula:

$$Ph_3\overset{+}{P}\overset{-}{Cl}CH_2OCH_3 \qquad (IV-1)$$

wherein Ph is a phenyl group, in a solvent in the presence of an alkoxide or an organometallic compound to yield a compound of the formula:

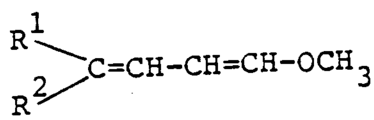

(XII)

wherein $R^1$ and $R^2$ are as defined above, hydrolyzing the compound of formula (XII) with an acid, followed by an alkali treatment to yield a compound of the formula:

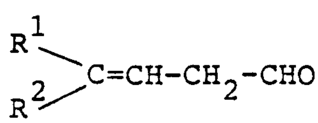

(XIII)

wherein $R^1$ and $R^2$ are as defined above, and reacting the compound of the formula (XIII) with a compound of the formula:

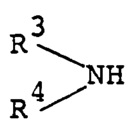

(VII)

wherein $R^3$ and $R^4$ are as defined above, in a solvent in the presence of a sulfonic acid.

7. A process for producing an enamine derivative of the formula:

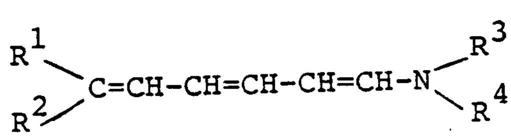

(I-3)

wherein $R^1$ is a group of the formula:

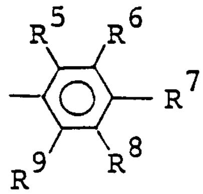

or

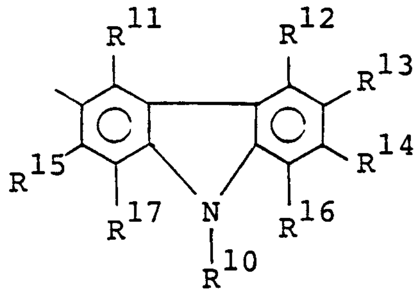

in which $R^5$, $R^6$, $R^7$, $R^8$ and $R^9$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, a straight- or branched-chain alkoxy group having 1 to 4 carbon atoms, a non-substituted or substituted aryloxy group, said substituted aryloxy group including aryloxy groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two straight- or branched-chain, non-substituted or substituted alkyl groups having 1 to 9 carbon atoms, said substituted alkyl groups including alkyl groups substituted with one or more halogen atoms, hydroxyl groups, cyano groups, nitro groups, alkylamino groups, arylamino groups, aryl groups, and aryloxy groups, an amino group substituted with one or two non-substituted or substituted aralkyl groups, said substituted aralkyl groups including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an amino group substituted with one or two non-substituted or substituted aryl groups, said substituted aryl groups including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, an amino group substituted with one or two non-substituted or substituted heterocyclic ring groups, said substituted heterocyclic ring groups including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups, a hydroxyl group, a nitro group or a cyano group; $R^{10}$ is a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{11}$, $R^{12}$, $R^{13}$, $R^{14}$ and $R^{15}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, an alkoxy group having 1 to 4 carbon atoms, a non-substituted or substituted aralkyl group, a non-substituted or substituted aryl group, said substituted aryl group including aryl groups substituted with one or more halogen atoms, hydroxyl groups, cyano groups, nitro groups, alkylamino groups, amino groups, alkyl groups, alkoxy groups, acetyl groups, acetoxy groups, arylamino groups, aralkylamino groups, aryl groups, and aryloxy groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; $R^{16}$ and $R^{17}$ are independently hydrogen, a halogen atom, a straight-or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group, or a cyano group; $R^2$, $R^3$ and $R^4$ are independently a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, or a non-substituted or substituted heterocyclic ring group, said substituted heterocyclic ring group including those substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups and aralkylamino groups; and further $R^2$ may be a hydrogen atom, and $R^3$ and $R^4$ may together form a group of the formula:

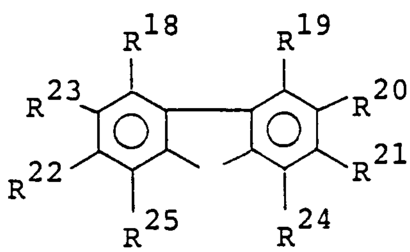

(II)

in which $R^{18}$, $R^{19}$, $R^{20}$, $R^{21}$, $R^{22}$ and $R^{23}$ are independently hydrogen, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, or an alkoxy group having 1 to 4 carbon atoms; and $R^{24}$ and $R^{25}$ are independently hydrogen, a halogen atom, a straight- or branched-chain, non-substituted or substituted alkyl group having 1 to 9 carbon atoms, said substituted alkyl group including alkyl groups substituted with one or more halogen atoms, hydroxyl groups, cyano groups, nitro groups, alkylamino groups, arylamino groups, aryl groups, and aryloxy groups, a non-substituted or substituted aralkyl group, said substituted aralkyl group including aralkyl groups substituted with one or more halogen atoms, hydroxyl groups, cyano groups, nitro groups, alkylamino groups, amino groups, alkyl groups, alkoxy groups, acetyl groups, acetoxy groups, aryl groups, aryloxy groups, arylamino groups, and aralkylamino groups, an alkoxy group having 1 to 4 carbon atoms, a hydroxyl group, a nitro group or a cyano group, which comprises reacting a compound of the formula:

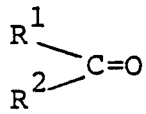

(III)

wherein $R^1$ and $R^2$ are as defined above, with a compound of the formula:

$$Ph_3\overset{+-}{PI}CH_3 \qquad (IV\text{-}2)$$

wherein Ph is a phenyl group, in a solvent in the presence of an alkoxide or an organometallic compound to yield a compound of the formula:

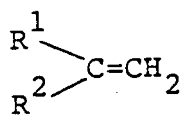

(X)

wherein $R^1$ and $R^2$ are as defined above, formylating the compound of the formula (X) in dimethylformamide in the presence of $POCl_3$ to yield a compound of the formula:

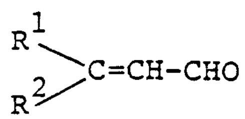

(XI)

wherein $R^1$ and $R^2$ are as defined above, reacting the compound of the formula (XI) with the compound of the formula (IV-2) in a solvent in the presence of an alkoxide or an organometallic compound to yield a compound of the formula:

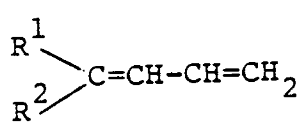

(XIV)

wherein $R^1$ and $R^2$ are as defined above, formylating the compound of the formula (XIV) in dimethylformamide in the presence of $POCl_3$ to yield a compound of the formula:

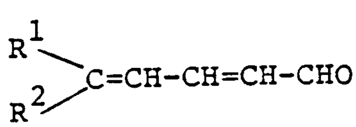

(XV)

wherein $R^1$ and $R^2$ are as defined above, reacting the compound of the formula (XV) with a compound of the formula:

$$Ph_3\overset{+}{P}\overset{-}{Cl}CH_2OCH_3 \qquad (IV-1)$$

wherein Ph is a phenyl group, in a solvent in the presence of an alkoxide or an organometallic compound to yield a compound of the formula:

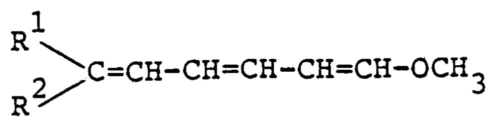

(XVI)

wherein $R^1$ and $R^2$ are as defined above, hydrolyzing the compound of the formula (XVI) with an acid, followed by an alkali treatment to yield a compound of the formula:

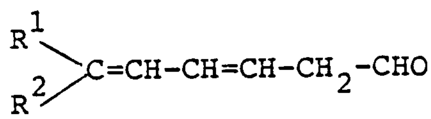

(XVII)

wherein $R^1$ and $R^2$ are as defined above, and reacting the compound of the formula (XVII) with a compound of the formula:

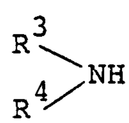

(VII)

wherein $R^3$ and $R^4$ are as defined above, in a so!vent in the presence of a sulfonic acid.

**8.** An electrophotographic plate, wherein the enamine derivative of claim 1 is used as a charge transport material.

**9.** An electrophotographic plate, wherein the enamine derivative of claim 2 is used as a charge transport material.

**10.** An electrophotographic plate, wherein the enamine derivative of claim 3 is used as a charge transport material.

## Patentansprüche

**1.** Enaminderivat der Formel:

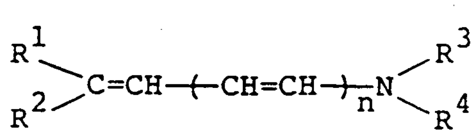

(I)

worin
(i) im Falle, wenn n Null bedeutet,

(a) $R^1$ eine Gruppe der Formel:

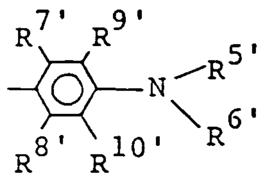

oder

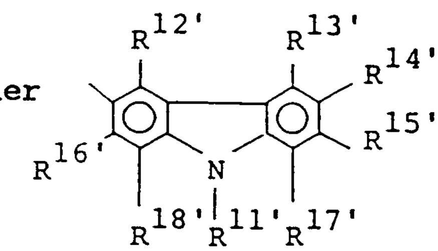

bedeutet, worin

$R^{5'}$ und $R^{6'}$ unabhängig eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeuten;

$R^{7'}$ und $R^{8'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{9'}$ und $R^{10'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten;

$R^{11'}$ eine Alkylgruppe mit 1 bis 9 Kohlenstoffatomen, eine Arylgruppe, die einen oder mehrere Sustituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeutet;

$R^{12'}$, $R^{13'}$, $R^{14'}$, $R^{15'}$ und $R^{16'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{117'}$ und $R^{18'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten;

$R^2$, $R^3$ und $R^4$ unabhängig eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeuten; und weiter $R^2$ Wasserstoff bedeuten kann und $R^3$ und $R^4$ zusammen eine Gruppe der Formel:

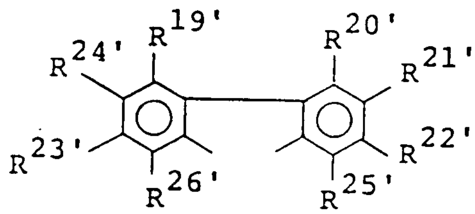

bilden können, worin $R^{19'}$, $R^{20'}$, $R^{21'}$, $R^{22'}$, $R^{23'}$ und $R^{24'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{25'}$ und $R^{26'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten; oder

(b) $R^1$ eine Gruppe der Formel:

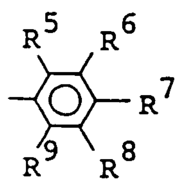

bedeutet, worin

$R^5$, $R^6$, $R^7$, $R^8$ und $R^9$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine geradkettige oder verzweigtkettige Alkoxygruppe mit 1 bis 4 Kohlenstoffatomen, eine nichtsubstituierte oder substituierte Aryloxygruppe, wobei die substituierte Alyloxygruppe Aryloxygruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine Aminogruppe, substituiert mit ein oder zwei geradkettigen oder verzweigtkettigen, nichtsubstituierten oder substituierten Alkylgruppen mit 1 bis 9 Kohlenstoffatomen, wobei die substituierten Alkylgruppen Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließen, eine Aminogruppe, substituiert mit ein oder zwei nichtsubstituierten oder substituierten Aralkylgruppen, wobei die substituierten Aralkylgruppen Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließen, eine Aminogruppe, substituiert mit ein oder zwei nichtsubstituierten oder substituierten Arylgruppen, wobei die

substituierten Arylgruppen Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließen, eine Aminogruppe, substituiert mit ein oder zwei nichtsubstituierten oder substituierten heterocyclischen Ringgruppen, wobei die substituierten heterocyclischen Ringgruppen solche einschließen, die mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen substituiert sind, eine Hydroxylgruppe, eine Nitrogruppe oder eine Cyanogruppe bedeuten;

$R^2$ Wasserstoff, eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylamlnogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeutet; und

$R^3$ und $R^4$ zusammen eine Gruppe der Formel:

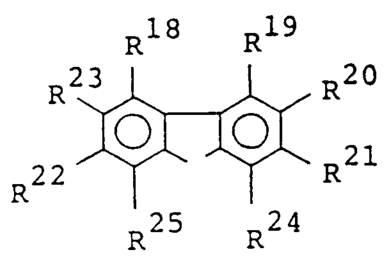

.(II)

bilden können, worin

$R^{18}$, $R^{19}$, $R^{20}$, $R^{21}$, $R^{22}$ und $R^{23}$ unabhängig Wasserstoff, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, oder eine Alkoxygruppe mit 1 bis 4 Kohlenstoffatomen bedeuten;

$R^{24}$ und $R^{25}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine Nitrogruppe oder eine Cyanogruppe bedeuten; und

(ii) im Falle, wenn n eine ganze Zahl von 1 oder 2 bedeutet, $R^1$ eine Gruppe der Formel:

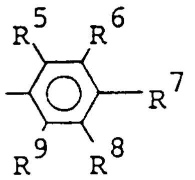

oder

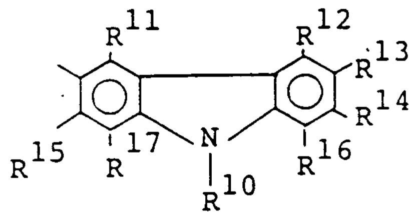

bedeutet, worin

$R^5$ bis $R^9$ die oben gegebenen Definitionen besitzen;

$R^{10}$ eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine nichtsubstituierte oder substituierte Aralkylgruppe, wobei die substituierte Aralkylgruppe Aralkylgruppen, srnbstituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, oder eine nichtsubstituierte oder substituierte heterocyclische Ringgruppe, wobei die substituierte heterocyclische Ringgruppe solche, die mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, substituiert sind, einschließt, bedeutet;

$R^{11}$, $R^{12}$, $R^{13}$, $R^{14}$ und $R^{15}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine Alkoxygruppe mit 1 bis 4 Kohlenstoffatomen, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten;

$R^{16}$ und $R^{17}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine

Nitrogruppe oder eine Cyanogruppe bedeuten;

$R^2$, $R^3$ und $R^4$ unabhängig eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten; und weiter $R^2$ ein Wasserstoffatom bedeuten kann und $R^3$ und $R^4$ zusammen eine Gruppe der Formel:

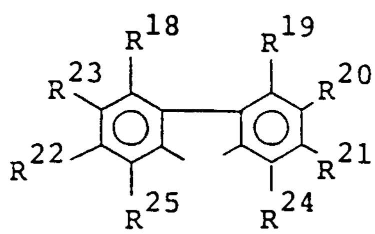

(II)

bilden können, worin $R^{18}$ bis $R^{25}$ die oben gegebenen Definitionen besitzen.

**2.** Enaminderivat nach Anspruch 1, dargestellt durch die Formel:

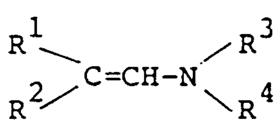

(I-1)

worin $R^1$ eine Gruppe der Formel:

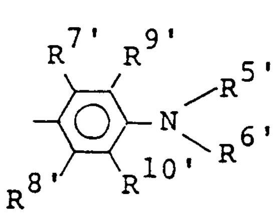

oder

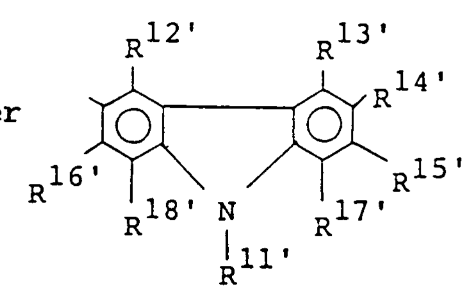

bedeutet, worin

$R^{5'}$ und $R^{6'}$ unabhängig eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen,

Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeuten;

$R^{7'}$ und $R^{8'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{9'}$ und $R^{10'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten;

$R^{11'}$ eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeutet;

$R^{12'}$, $R^{13'}$, $R^{14'}$ $R^{15'}$ und $R^{16'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{17'}$ und $R^{18'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten;

$R^2$, $R^3$ und $R^4$ unabhängig eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeuten; und weiter $R^2$ Wasserstoff bedeuten kann und $R^3$ und $R^4$ zusammen eine Gruppe der Formel:

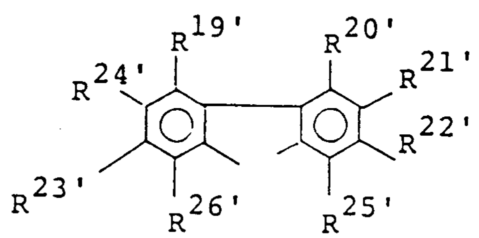

bilden können, worin

$R^{19'}$, $R^{20'}$, $R^{21'}$, $R^{22'}$, $R^{23'}$ und $R^{24'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{25'}$ und $R^{26'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten.

**3.** Enaminderivat nach Anspruch 1, dargestellt durch die Formel:

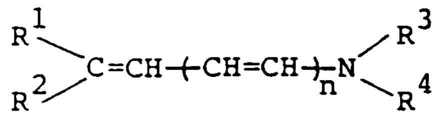

(I)

worin $R^1$ eine Gruppe der Formel:

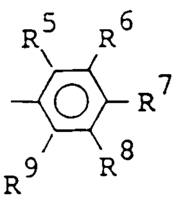

oder

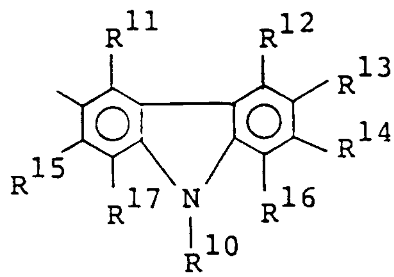

bedeutet, worin

$R^5$, $R^6$, $R^7$, $R^8$ und $R^9$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine geradkettige oder verzweigtkettige Alkoxygruppe mit 1 bis 4 Kohlenstoffatomen, eine nichtsubstituierte oder substituierte Aryloxygruppe, wobei die substituierte Aryloxygruppe Aryloxygruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine Aminogruppe, substituiert mit ein oder zwei geradkettigen oder verzweigtkettigen Alkylgruppen mit 1 bis 9 Kohlenstoffatomen, eine Aminogruppe, substituiert mit ein oder zwei nichtsubstituierten oder substituierten Arylgruppen, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine Hydroxylgruppe, eine Nitrogruppe oder eine Cyanogruppe bedeuten;

$R^{10}$ eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, oder eine nichtsubstituierte oder substituierte heterocyclische Ringgruppe, wobei die substituierte heterocyclische Ringgruppe solche, substituiert mit ein oder

mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeutet;

$R^{11}$, $R^{12}$, $R^{13}$, $R^{14}$ und $R^{15}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten;

$R^{16}$ und $R^{17}$ unabhängig Wasserstoff, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe eine Nitrogruppe oder eine Cyanogruppe bedeuten;

$R^2$, $R^3$ und $R^4$ unabhängig eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten; und weiter $R^2$ ein Wasserstoffatom bedeuten kann und $R^3$ und $R^4$ zusammen eine Gruppe der Formel:

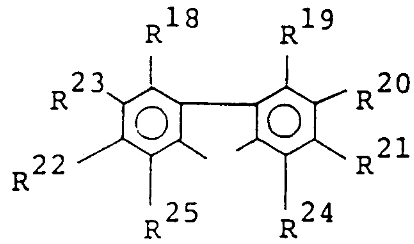

(II)

bilden können, worin

$R^{18}$, $R^{19}$, $R^{20}$, $R^{21}$, $R^{22}$ und $R^{23}$ unabhängig Wasserstoff, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, oder eine Alkoxygruppe mit 1 bis 4 Kohlenstoffatomen bedeuten;

$R^{24}$ und $R^{25}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine Nitrogruppe oder eine Cyanogruppe bedeuten; und

n eine ganze Zahl von 1 oder 2 bedeutet.

**4.** Verfahren zur Herstellung eines Enaminderivats der Formel:

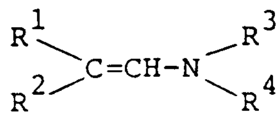

(I-1)

worin

(a) $R^1$ die Gruppe der Formel:

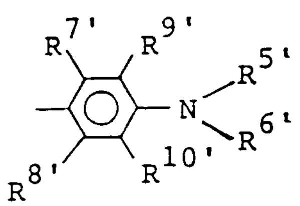

oder

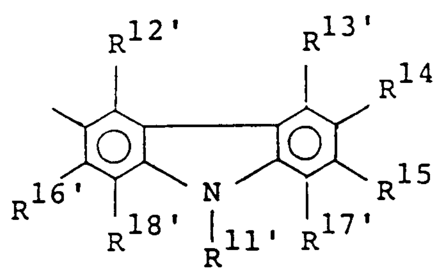

bedeutet, worin

$R^{5'}$ und $R^{6'}$ unabhängig eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeuten;

$R^{7'}$ und $R^{8'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{9'}$ und $R^{10'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten;

$R^{11'}$ eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeutet;

$R^{12'}$, $R^{13'}$, $R^{14'}$, $R^{15'}$ und $R^{16'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{17'}$ und $R^{18'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten;

$R^2$, $R^3$ und $R^4$ unabhängig eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeuten; und weiter $R^2$ Wasserstoff bedeuten kann und $R^3$ und $R^4$ zusammen eine Gruppe der Formel:

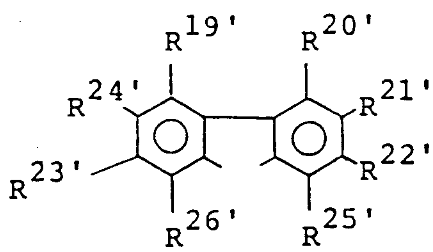

bilden können, worin

$R^{19'}$, $R^{20'}$, $R^{21'}$, $R^{22'}$, $R^{23'}$ und $R^{24'}$ unabhängig Wasserstoff, eine Alkylgruppe mit 1 bis 9 Kohlenstoffatomen oder eine Alkoxygruppe mit 1 bis 4 Kohlenstoffatomen bedeuten;

$R^{25'}$ und $R^{26'}$ unabhängig Wasserstoff, ein Halogenatom, eine Alkylgruppe mit 1 bis 9 Kohlenstoffatomen, eine Alkoxygruppe mit 1 bis 4 Kohlenstoffatomen, eine Aralkylgruppe, die einen oder mehrere

mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, eine Hydroxylgruppe, eine Nitrogruppe oder eine Cyanogruppe bedeuten; oder

(b) $R^1$ eine Gruppe der Formel:

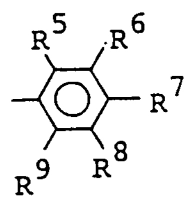

bedeutet, worin

$R^5$, $R^6$, $R^7$, $R^8$ und $R^9$ unabhängig Wasserstoff, ein Halogenatom, eine geradkettige oder verzweigtkettige, nichtsubstitrnierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine geradkettige oder verzweigtkettige Alkoxygruppe mit 1 bis 4 Kohlenstoffatomen, eine nichtsubstituierte oder substituierte Aryloxygruppe, wobei die substituierte Aryloxygruppe Aryloxygruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine Aminogruppe, substituiert mit ein oder zwei geradkettigen oder verzweigtkettigen, nichtsubstituierten oder substituierten Alkylgruppen mit 1 bis 9 Kohlenstoffatomen, wobei die substituierten Alkylgruppen Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließen, eine Aminogruppe, substituiert mit ein oder zwei nichtsubstituierten oder substituierten Aralkylgruppen, wobei die substituierten Aralkylgruppen Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließen, eine Aminogruppe, substituiert mit ein oder zwei nichtsubstituierten oder substituierten Arylgruppen, wobei die substituierten Arylgruppen Arylgruppen, substituiert mit ein oder mehreren Halogenatomen, Hydroxylgruppen, Cyanogruppen, Nitrogruppen, Alkylaminogruppen, aminogruppen, Alkylgruppen, Alkoxygruppen, Acetylgruppen, Acetoxygruppen, Arylaminogruppen, Aralkylaminogruppen, Arylgruppen und Aryloxygruppen, einschließen, eine Aminogruppe, substituiert mit ein oder zwei nichtsubstituierten oder substituierten heterocyclischen Ringgruppen, wobei die substituierten heterocyclischen Ringgruppen solche einschließen, die mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen substituiert sind, eine Hydroxylgruppe, eine Nitrogruppe oder eine Cyanogruppe bedeuten;

$R^2$ Wasserstoff, eine Alkylgruppe mit 1 bis 9 Kohlenstoffatomen, eine Arylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Aylgruppen und Aryloxygruppen, enthalten kann, eine Aralkylgruppe, die einen oder mehrere Substituenten, ausgewählt aus ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, enthalten kann, oder eine heterocyclische Ringgruppe bedeutet; und

$R^3$ und $R^4$ zusammen eine Gruppe der Formel:

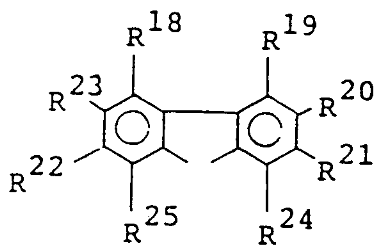

(II)

bilden können, worin

$R^{18}$, $R^{19}$, $R^{20}$, $R^{21}$, $R^{22}$ und $R^{23}$ unabhängig Wasserstoff, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, oder eine Alkoxygruppe mit 1 bis 4 Kohlenstoffatomen bedeuten;

$R^{24}$ und $R^{25}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine Nitrogruppe oder eine Cyanogruppe bedeuten, dadurch **gekennzeichnet,** daß eine Verbindung der Formel:

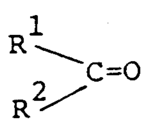

(III)

worin $R^1$ und $R^2$ die oben gegebenen Definitionen besitzen, mit einer Verbindung der Formel:

$$Ph_3\overset{+}{P}\overset{-}{Cl}CH_2OCH_3 \qquad (IV\text{-}1)$$

worin Ph eine Phenylgruppe bedeutet, in einem Lösungsmittel in Anwesenheit eines Alkoxids oder einer metallorganischen Verbindung unter Bildung einer Verbindung der Formel:

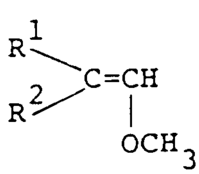

(V)

worin $R^1$ und $R^2$ die oben gegebenen Definitionen besitzen, umgesetzt wird, die Verbindung der Formel (V) mit einer Säure hydrolysiert wird, gefolgt von einer Alkalibehandlung, wobei eine

Verbindung der Formel:

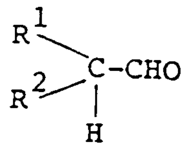

(VI)

gebildet wird, worin $R^1$ und $R^2$ die oben gegebenen Definitionen besitzen, und die Verbindung der Formel (VI) in eine Verbindung der Formel:

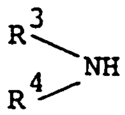

(VII)

worin $R^3$ und $R^4$ die oben gegebenen Definitionen besitzen, in einem Lösungsmittel in Anwesenheit einer Sulfonsäure umgesetzt wird.

**5.** Verfahren zur Herstellung eines Enaminderivats der Formel:

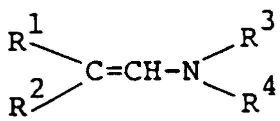

(I-1)

worin $R^1$ bis $R^4$ die in Anspruch 4 gegebenen Definitionen besitzen, dadurch **gekennzeichnet,** daß eine Verbindung der Formel:

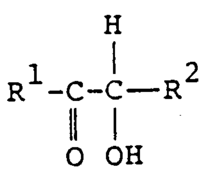

(VIII)

worin $R^1$ und $R^2$ die oben gegebenen Definitionen besitzen, mit einem Metallhydrid reduziert wird, anschließend eine Säurebehandlung und eine Alkalibehandlung folgen, wobei eine Verbindung der Formel:

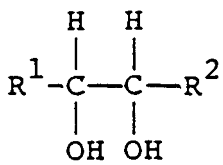

(IX)

gebildet wird, worin $R^1$ und $R^2$ die oben gegebenen Definitionen besitzen, die Verbindung der Formel (IX) mit einer Säure unter Bildung einer Verbindung der Formel:

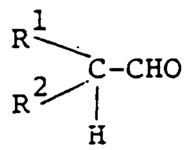

(VI)

umgesetzt wird, worin $R^1$ und $R^2$ die oben gegebenen Definitionen besitzen, und die Verbindung der Formel (VI) mit einer Verbindung der Formel:

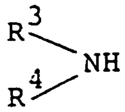

(VII)

worin $R^3$ und $R^4$ die oben gegebenen Definitionen besitzen, in einem Lösungsmittel in Anwesenheit einer Sulfonsäure umgesetzt wird.

**6.** Verfahren zur Herstellung eines Enaminderivats der Formel:

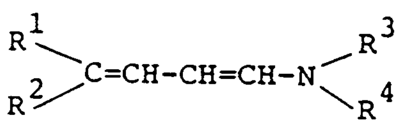

(I-2)

worin $R^1$ eine Gruppe der Formel:

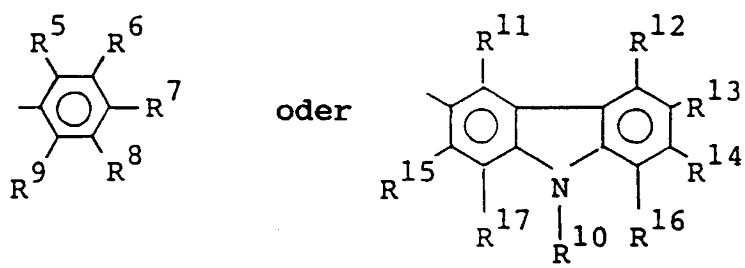

bedeutet, worin

$R^5$, $R^6$, $R^7$, $R^8$ und $R^9$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine geradkettige oder verzweigtkettige Alkoxygruppe mit 1 bis 4 Kohlenstoffatomen, eine nichtsubstituierte oder substituierte Aryloxygruppe, wobei die substituierte Aryloxygruppe Aryloxygruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine Aminogruppe, substituiert mit ein oder zwei geradkettigen oder verzweigtkettigen, nichtsubstituierten oder substituierten Alkylgruppen mit 1 bis 9 Kohlenstoffatomen, wobei die substituierte Alkylgruppe Alkylgruppen, substituiert mit ein oder mehreren Halogenatomen, Hydroxylgruppen, Cyanogruppen,

Nitrogruppen, Alkylaminogruppen, Arylaminogruppen, Arylgruppen und Aryloxygruppen, einschließt, eine Aminogruppe, substituiert mit ein oder zwei nichtsubstituierten oder substituierten Aralkylgruppen, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Aminogruppe, substituiert mit ein oder zwei nichtsubstituierten oder substituierten Arylgruppen, wobei die substituierten Arylgruppen Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließen, eine Aminogruppe, substituiert mit ein oder zwei nichtsubstituierten oder substituierten heterocyclischen Ringgruppen, wobei die substituierten heterocyclischen Ringgruppen solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließen, eine Hydroxylgruppe, eine Nitrogruppe oder eine Cyanogruppe bedeuten;

$R^{10}$ eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeutet;

$R^{11}$, $R^{12}$, $R^{13}$, $R^{14}$ und $R^{15}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine Alkoxygruppe mit 1 bis 4 Kohlenstoffatomen, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten;

$R^{16}$ und $R^{17}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe eine Nitrogruppe oder eine

Cyanogruppe bedeuten;

$R^2$, $R^3$ und $R^4$ unabhängig eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten; und weiter $R^2$ ein Wasserstoffatom bedeuten kann und $R^3$ und $R^4$ zusammen eine Gruppe der Formel:

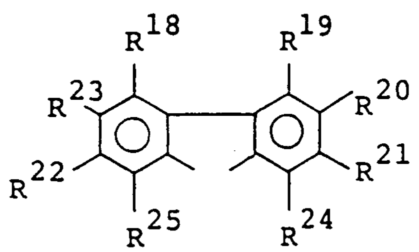

(II)

bilden können, worin

$R^{18}$, $R^{19}$, $R^{20}$, $R^{21}$, $R^{22}$ und $R^{23}$ unabhängig Wasserstoff, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, oder eine Alkoxygruppe mit 1 bis 4 Kohlenstoffatomen bedeuten; und

$R^{24}$ und $R^{25}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine Nitrogruppe oder eine Cyanogruppe bedeuten;

dadurch **gekennzeichnet,** daß eine Verbindung der Formel:

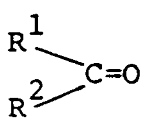

(III)

worin $R^1$ und $R^2$ die oben gegebenen Definitonen besitzen, mit einer Verbindung der Formel:

$$Ph_3\overset{+}{P}\overset{-}{I}CH_3 \qquad (IV\text{-}2)$$

worin Ph eine Phenylgruppe bedeutet, in einem Lösungsmittel in Anwesenheit eines Alkoxids oder einer metallorganischen Verbindung unter Bildung einer Verbindung der Formel:

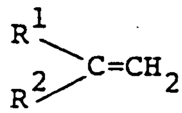

(X)

worin $R^1$ und $R^2$ die oben gegebenen Definitionen besitzen, umgesetzt wird, die Verbindung der Formel (X) in Dimethylformamid in Anwesenheit von $POCl_3$ unter Bildung einer Verbindung der Formel:

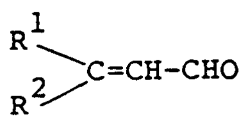

(XI)

worin $R^1$ und $R^2$ die oben gegebenen Definitionen besitzen, formyliert wird, die Verbindung der Formel (XI) mit einer Verbindung der Formel:

$$Ph_3\overset{+}{P}\overset{-}{Cl}CH_2OCH_3 \qquad (IV\text{-}1)$$

worin Ph eine Phenylgruppe bedeutet, in einem Lösungsmittel in Anwesenheit eines Alkoxids oder einer metallorganischen Verbindung unter Bildung einer Verbindung der Formel:

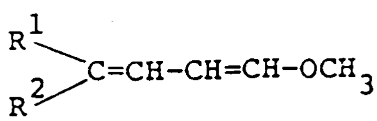

(XII)

worin $R^1$ und $R^2$ die oben gegebenen Definitionen besitzen, umgesetzt wird, die Verbindung der Formel (XII) mit einer Säure hydrolysiert wird, anschließend eine Alkalibehandlung unter Bildung einer Verbindung der Formel:

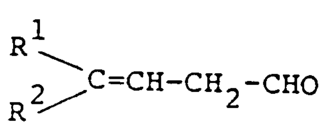

(XIII)

worin $R^1$ und $R^2$ die oben gegebenen Definitionen besitzen, folgt und die Verbindung der Formel (XIII) mit einer Verbindung der Formel:

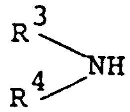

(VII)

worin $R^3$ und $R^4$ die oben gegebenen Definitionen besitzen, in einem Lösungsmittel in Anwesenheit einer Sulfonsäure umgesetzt wird.

7. Verfahren zur Herstellung eines Enaminderivats der Formel:

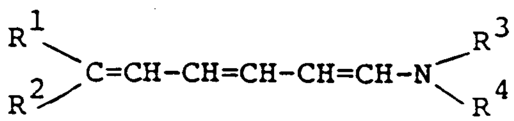

(I-3)

worin $R^1$ eine Gruppe der Formel:

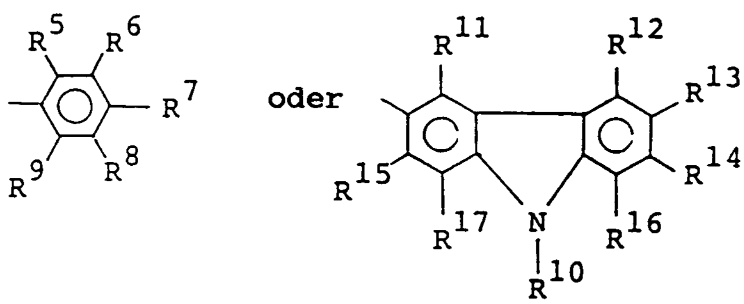

bedeutet, worin

$R^5$, $R^6$, $R^7$, $R^8$ und $R^9$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine geradkettige oder verzweigtkettige Alkoxygruppe mit 1 bis 4 Kohlenstoffatomen, eine nichtsubstituierte oder substituierte Aryloxygruppe, wobei die substituierte Aryloxygruppe Aryloxygruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine Aminogruppe, substituiert mit ein oder zwei geradkettigen oder verzweigtkettigen, nichtsubstituierten oder substituierten Alkylgruppen mit 1 bis 9 Kohlenstoffatomen, wobei die substituierten Alkylgruppen Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließen, eine Aminogruppe, substituiert mit ein oder zwei nichtsubstituierten oder substituierten Aralkylgruppen, wobei die substituierten Aralkylgruppen Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließen, eine Aminogruppe, substituiert mit ein oder zwei nichtsubstituierten oder substituierten Arylgruppen, wobei die substituierten Arylgruppen Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließen, eine Aminogruppe, substituiert mit ein oder zwei nichtsubstituierten oder substituierten heterocyclischen Ringgruppen, wobei die substituierten heterocyclischen Ringgruppen solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließen, eine Hydroxylgruppe, eine Nitrogruppe oder eine Cyanogruppe bedeuten;

$R^{10}$ eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Arylorygruppen, einschließt, eine nichtsubstituierte oder substituierte Arylgruppe, wobei die substituierte Arylgruppe Arrylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, oder eine nichtsubstituierte oder Substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeutet;

$R^{11}$, $R^{12}$, $R^{13}$, $R^{14}$ und $R^{15}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine Alkoxygruppe mit 1 bis 4 Kohlenstoffatomen, eine nichtsubstituierte oder substituierte Aralkylgruppe, eine nichtsubstituierte oder substituierte Arylgruppe, wobei die substituierte Arylgruppe Arylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylaminogruppen, Aralkylaminogruppen, Arylgruppen und Aryloxygruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten;

$R^{16}$ und $R^{17}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine Nitrogruppe oder eine Cyanogruppe bedeuten;

$R^2$, $R^3$ und $R^4$ unabhängig eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, oder eine nichtsubstituierte oder substituierte heterocyclische Ringgruppe, wobei die substituierte heterocyclische Ringgruppe solche, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, bedeuten; und weiter $R^2$ ein Wasserstoffatom bedeuten kann und $R^3$ und $R^4$ zusammen eine Gruppe der Formel:

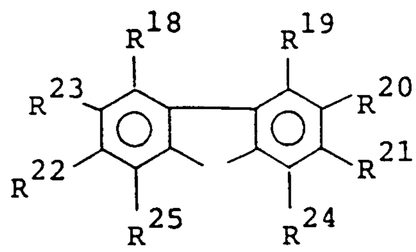

(II)

bilden können, worin

$R^{18}$, $R^{19}$, $R^{20}$, $R^{21}$, $R^{22}$ und $R^{23}$ unabhängig Wasserstoff, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, oder eine Alkoxygruppe mit 1 bis 4 Kohlenstoffatomen bedeuten; und

$R^{24}$ und $R^{25}$ unabhängig Wasserstoff, ein Halogenatom, eine geradkettige oder verzweigtkettige, nichtsubstituierte oder substituierte Alkylgruppe mit 1 bis 9 Kohlenstoffatomen, wobei die substituierte Alkylgruppe Alkylgruppen, substituiert mit ein oder mehreren Halogenatomen, Hydroxylgruppen, Cyanogruppen, Nitrogruppen, Alkylaminogruppen, Arylaminogruppen, Arylgruppen und Aryloxygruppen, einschließt, eine nichtsubstituierte oder substituierte Aralkylgruppe, wobei die substituierte Aralkylgruppe Aralkylgruppen, substituiert mit ein oder mehreren Halogenatomen, Hydroxylgruppen, Cyanogruppen, Nitrogruppen, Alkylaminogruppen, Aminogruppen, Alkylgruppen, Alkoxygruppen, Acetylgruppen, Acetoxygruppen, Arylgruppen, Aryloxygruppen, Arylaminogruppen und Aralkylaminogruppen, einschließt, eine Alkoxygruppe mit 1 bis 4 Kohlenstoffatomen, eine Hydroxylgruppe, eine Nitrogruppe oder eine Cyanogruppe bedeuten;

dadurch **gekennzeichnet,** daß eine Verbindung der Formel:

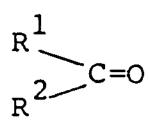

(III)

worin $R^1$ und $R^2$ die oben gegebenen Definitionen besitzen, mit einer Verbindung der Formel:

$$Ph_3\overset{+-}{PI}CH_3 \qquad (IV-2)$$

worin Ph eine Phenylgruppe bedeutet, in einem Lösungsmittel in Anwesenheit eines Alkoxids oder einer metallorganischen Verbindung unter Bildung einer Verbindung der Formel:

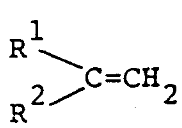

(X)

worin $R^1$ und $R^2$ die oben gegebenen Definitionen besitzen, umgesetzt wird, die Verbindung der Formel (X) in Dimethylformamid in Anwesenheit von $POCl_3$ unter Bildung einer Verbindung der Formel:

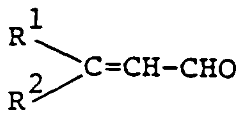

(XI)

worin $R^1$ und $R^2$ die oben gegebenen Definitionen besitzen, formyliert wird, die Verbindung der Formel (XI) mit der Verbindung der Formel (IV-2) in einem Lösungsmittel in Anwesenheit eines Alkoxids oder einer metallorganischen Verbindung unter Bildung einer Verbindung der Formel:

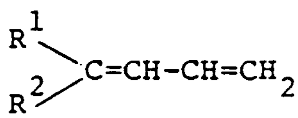

(XIV)

worin $R^1$ und $R^2$ die oben gegebenen Definitionen besitzen, umgesetzt wird, die Verbindung der Formel (XIV) in Dimethylformamid in Anwesenheit von $POCl_3$ unter Bildung einer Verbindung der Formel:

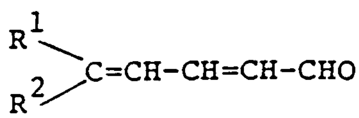

(XV)

worin $R^1$ und $R^2$ die oben gegebenen Definitionen besitzen, formyliert wird, die Verbindung der Formel (XV) mit einer Verbindung der Formel:

$$Ph_3\overset{+}{P}\overset{-}{Cl}CH_2OCH_3 \qquad (IV-1)$$

worin Ph eine Phenylgruppe bedeutet, in einem Lösungsmittel in Anwesenheit eines Alkoxids oder einer metallorganischen Verbindung unter Bildung einer Verbindung der Formel:

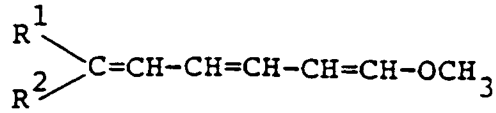

(XVI)

worin $R^1$ und $R^2$ die oben gegebenen Definitionen besitzen, umgesetzt wird, die Verbindung der Formel (XVI) mit einer Säure hydrolysiert wird, anschließend eine Alkalibehandlung unter Bildung einer Verbindung der Formel:

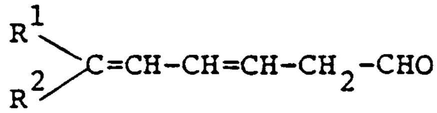

(XVII)

worin $R^1$ und $R^2$ die oben gegebenen Definitionen besitzen, durchgeführt wird, und die Verbindung der Formel (XVII) mit einer Verbindung der Formel:

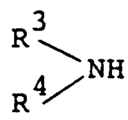

(VII)

worin $R^3$ und $R^4$ die oben gegebenen Definitionen besitzen, in einem Lösungsmittel in Anwesenheit einer Sulfonsäure umgesetzt wird.

**8.** Elektrophotographische Platte, in der das Enaminderivat von Anspruch 1 als Ladungstransportmaterial verwendet wird.

**9.** Elektrophotographische Platte, in der das Enaminderivat von Anspruch 2 als Ladungstransportmaterial verwendet wird.

**10.** Elektrophotographische Platte, in der das Enaminderivat von Anspruch 3 als Ladungstransportmaterial verwendet wird.

**Revendications**

**1.** Dérivé énamine de formule :

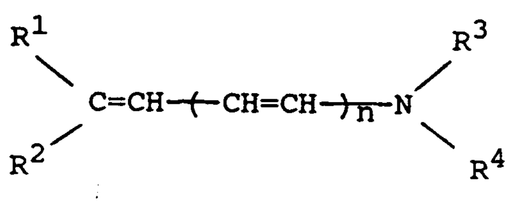

(I)

dans laquelle :

(i) dans le cas où n est égal à zéro :

(a) $R^1$ représente un groupe de formule :

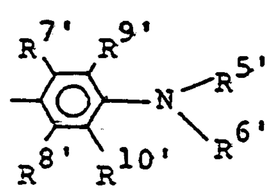

ou

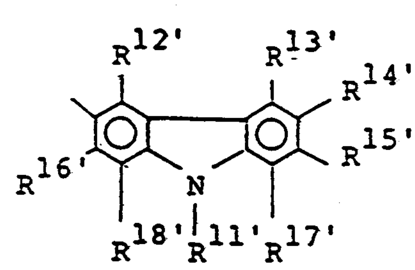

dans laquelle $R^{5'}$ et $R^{6'}$ représentent chacun, indépendamment, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

$R^{7'}$ et $R^{8'}$ représentent chacun, indépendamment, un atome d'hydrogène, un groupe alkyle ayant 1 à 9 atomes de carbone ou un groupe alcoxy ayant 1 à 4 atomes de carbone,

$R^{9'}$ et $R^{10'}$ représentent chacun, indépendamment, un atome d'hydrogène ou d'halogène, un groupe alkyle ayant 1 à 9 atomes de carbone, un groupe alcoxy ayant 1 à 4 atomes de carbone, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe hydroxyle, un groupe nitro ou un

groupe cyano,

$R^{11'}$ représente un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

$R^{12'}$, $R^{13'}$, $R^{14'}$, $R^{15'}$ et $R^{16'}$ représentent chacun, indépendamment, un atome d'hydrogène, un groupe alkyle ayant 1 à 9 atomes de carbone ou un groupe alcoxy ayant 1 à 4 atomes de carbone,

$R^{17'}$ et $R^{18'}$ représentent chacun, indépendamment, un atome d'hydrogène ou d'halogène, un groupe alkyle ayant 1 à 9 atomes de carbone, un groupe alcoxy ayant 1 à 4 atomes de carbone, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe hydroxyle, un groupe nitro ou un groupe cyano,

$R^2$, $R^3$ et $R^4$ représentent chacun, indépendamment, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

et en outre $R^2$ peut représenter un atome d'hydrogène, et $R^3$ et $R^4$ peuvent former ensemble un groupe de formule :

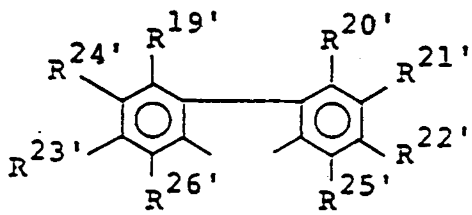

dans laquelle $R^{19'}$, $R^{20'}$, $R^{21'}$, $R^{22'}$, $R^{23'}$ et $R^{24'}$ représentent chacun, indépendamment, un atome d'hydrogène, un groupe alkyle ayant 1 à 9 atomes de carbone ou un groupe alcoxy ayant 1 à 4 atomes de carbone,

$R^{25'}$ et $R^{26'}$ représentent chacun, indépendamment, un atome d'hydrogène, un groupe alkyle ayant 1 à 9 atomes de carbone, un groupe alcoxy ayant 1 à 4 atomes de carbone, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe hydroxyle, un groupe nitro ou un groupe cyano, ou bien

(b) $R^1$ est un groupe de formule :

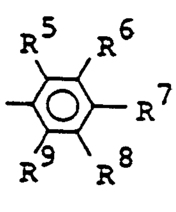

dans laquelle $R^5$ , $R^6$ , $R^7$, $R^8$ et $R^9$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes

hydroxyle, cyano, nitro, alkylamino, arylamino, aryle et aryloxy, un groupe aralkyle, non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy, à chaîne droite ou ramifiée, ayant 1 à 4 atomes de carbone, un groupe aryloxy non substitué ou substitué, ledit groupe aryloxy substitué comprenant les groupes aryloxy substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, un groupe amino substitué par un ou deux groupes alkyle ayant 1 à 9 atomes de carbone, à chaîne droite ou ramifiée, non substitués ou substitués, lesdits groupes alkyle substitués comprenant les groupes alkyle substitués par un ou plusieurs substituants pris parmi les atomes d'halogène et les groupes hydroxyle, cyano, nitro, alkylamino, arylamino, aryle et aryloxy, un groupe amino substitué par un ou deux groupes alkyle non substitués ou substitués, lesdits groupes aralkyle substitués comprenant les groupes aralkyle substitués par un ou plusieurs substituants choisis parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes aryle non substitués ou substitués, lesdits groupes aryle substitués comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, un groupe amino substitué par un ou deux groupes hétérocycliques non substitués ou substitués, lesdits groupes hétérocycliques substitués comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano,

$R^2$ représente un atome d'hydrogène, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

et $R^3$ et $R^4$ forment ensemble un groupe de formule :

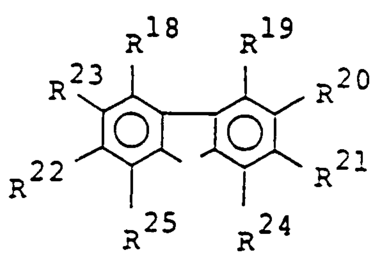

(II)

dans laquelle $R^{18}$, $R^{19}$, $R^{20}$, $R^{21}$, $R^{22}$ et $R^{23}$ représentent chacun, indépendamment, un atome d'hydrogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, ou un groupe alcoxy ayant 1 à 4 atomes de carbone,

$R^{24}$ et $R^{25}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs

substituants pris parmi les atomes d'halogène et les groupes hydroxyle, cyano, nitro, alkylamino, amino, alkyle, alcoxy, acétyle, acétoxy, aryle, aryloxy, arylamino, et aralkylamino, un groupe alcoxy ayant 1 à 4 atomes de carbone, un groupe hydroxyle, un groupe nitro ou un groupe cyano, et

(ii) dans le cas où n est un nombre entier égal à 1 ou 2, $R^1$ représente un groupe de formule :

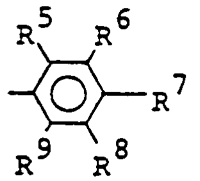

ou

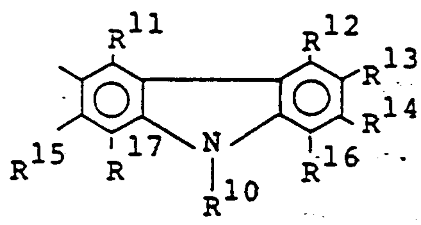

dans laquelle $R^5$ à $R^9$ sont tels que définis précédemment,

$R^{10}$ représente un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy,

$R^{11}$, $R^{12}$, $R^{13}$, $R^{14}$ et $R^{15}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe alcoxy ayant 1 à 4 atomes de carbone, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aryle non substitué ou substitué, ledit groupe aryle substitué comprenant les groupes aryle substitués par un ou plusieurs substituants pris parmi les atomes d'halogène et les groupe hydroxyle, cyano, nitro, alkylamino, amino, alkyle, alcoxy, acétyle, acétoxy, arylamino, aralkylamino, aryle et aryloxy, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy,

$R^{16}$ et $R^{17}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupe alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,

$R^2$, $R^3$ et $R^4$ représentent chacun, indépendamment, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes

d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aryle non substitué ou substitué, ledit groupe aryle substitue comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,
et en outre $R^2$ peut représenter un atome d'hydrogène, et $R^3$ et $R^4$ peuvent former ensemble un groupe de formule :

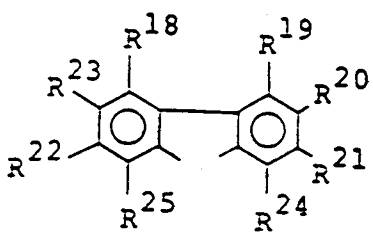

(II)

dans laquelle $R^{18}$ à $R^{25}$ sont tels que définis précédemment.

**2.** Dérivé énamine selon la revendication 1, qui est représenté par la formule :

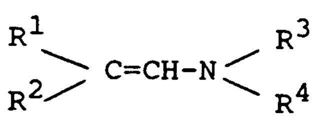

(I-1)

dans laquelle $R^1$ est un groupe de formule :

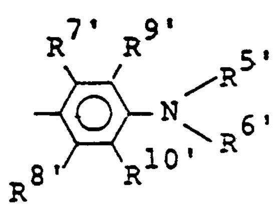

ou

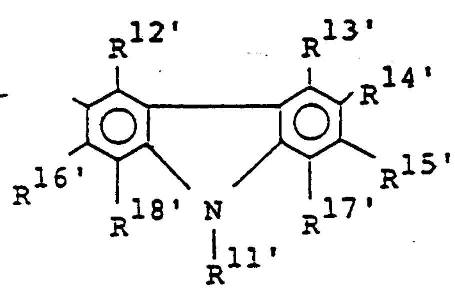

dans laquelle $R^{5'}$ et $R^{6'}$ représentent chacun, indépendamment, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique,
$R^{7'}$ et $R^{8'}$ représentent chacun, indépendamment, un atome d'hydrogène, un groupe alkyle ayant 1 à 9 atomes de carbone ou un groupe alcoxy ayant 1 à 4 atomes de carbone,
$R^{9'}$ et $R^{10'}$ représentent chacun, indépendamment, un atome d'hydrogène ou d'halogène, un groupe alkyle ayant 1 à 9 atomes de carbone, un groupe alcoxy ayant 1 à 4 atomes de carbone, un groupe aralkyle pouvant porter un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et

aralkylamino, un groupe hydroxyle, un groupe nitro ou un groupe cyano,

$R^{11'}$ représente un groupe alkyle ayant 1 à 9 atomes de carbone, un groupe aryle pouvant porter un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aralkyle pouvant porter un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique,

$R^{12'}$, $R^{13'}$, $R^{14'}$, $R^{15'}$ et $R^{16'}$ représentent chacun, indépendamment, un atome d'hydrogène, un groupe alkyle ayant 1 à 9 atomes de carbone, ou un groupe alcoxy ayant 1 à 4 atomes de carbone,

$R^{17'}$ et $R^{18'}$ représentent chacun, indépendamment, un atome d'hydrogène ou d'halogène, un groupe alkyle ayan't 1 à 9 atomes de carbone, un groupe alcoxy ayant 1 à 4 atomes de carbone, un groupe aralkyle pouvant porter un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano,

$R^2$, $R^3$ et $R^4$ représentent chacun, indépendamment, un groupe alkyle ayant 1 à 9 atomes de carbone, un groupe aryle pouvant porter un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aralkyle pouvant porter un ou plusieurs substituants pris parmi les atomes d'halogène et les groupes hydroxyie, cyano, nitro, alkylamino, amino, alkyle, alcoxy, acétyle, acétoxy, aryle, aryloxy, arylamino et aralkylamino, ou un groupe hétérocyclique,

et en outre $R^2$ peut être un atome d'hydrogène, et $R^3$ et $R^4$ peuvent former ensemble un groupe de formule :

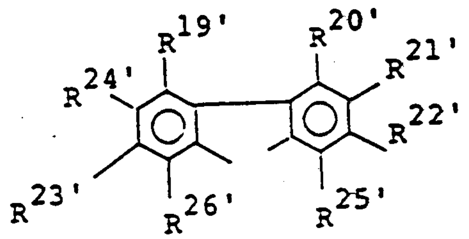

dans laquelle $R^{19'}$, $R^{20'}$, $R^{21'}$, $R^{22'}$, $R^{23'}$ et $R^{24'}$ représentent chacun, indépendamment, un atome d'hydrogène, un groupe alkyle ayant 1 à 9 atomes de carbone ou un groupe alcoxy ayant 1 à 4 atomes de carbone,

$R^{25'}$ et $R^{26'}$ représentent chacun, indépendamment, un atome d'hydrogène ou d'halogène, un groupe alkyle ayant 1 à 9 atomes de carbone, un groupe alcoxy ayant 1 à 4 atomes de carbone, un groupe aralkyle pouvant porter un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano.

**3.** Dérivé énamine selon la revendication 1, qui est représenté par la formule :

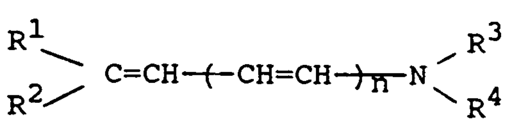

(I)

dans laquelle $R^1$ représente un groupe de formule :

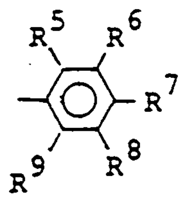

ou

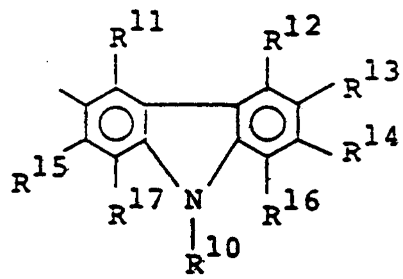

dans laquelle $R^5$, $R^6$, $R^7$, $R^8$ et $R^9$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitué par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitué par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy, à chaîne droite ou ramifiée, ayant 1 à 4 atomes de carbone, un groupe aryloxy non substitué ou substitué, ledit groupe aryloxy substitué comprenant les groupes aryloxy substitué par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes alkyle, à chaîne droite ou ramifiée, ayant 1 à 9 atomes de carbone, un groupe amino substitué par un ou deux groupes aryle non substitué ou substitué, lesdits groupes aryle substitués comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano,

$R^{10}$ représente un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy,

$R^{11}$, $R^{12}$, $R^{13}$, $R^{14}$ et $R^{15}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitué par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitué par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy,

$R^{16}$ et $R^{17}$ représente chacun, indépendamment, un atome d'hydrogène, un groupe alkyle ayant 1 à 9

atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle; cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,

$R^2$, $R^3$ et $R^4$ représente chacun, indépendamment, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

et en outre $R^2$ peut représenter un atome d'hydrogène, et $R^3$ et $R^4$ peuvent former ensemble un groupe de formule :

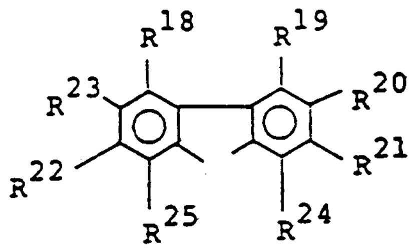

(II)

dans laquelle $R^{18}$, $R^{19}$, $R^{20}$, $R^{21}$, $R^{22}$ et $R^{23}$ représentent chacun, indépendamment, un atome d'hydrogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, ou un groupe alcoxy ayant 1 à 4 atomes de carbone,

$R^{24}$ et $R^{25}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,

et n est un nombre entier égal à 1 ou 2.

**4.** Procédé de préparation d'un dérivé énamine de formule :

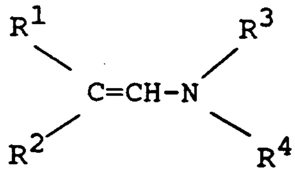

(I-1)

dans laquelle :

(a) $R^1$ représente un groupe de formule :

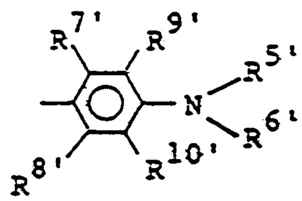

ou

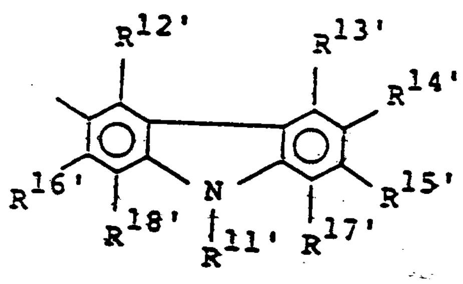

dans laquelle $R^{5'}$ et $R^{6'}$ représentent chacun, indépendamment, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

$R^{7'}$ et $R^{8'}$ représentent chacun, indépendamment, un atome d'hydrogène, un groupe alkyle ayant 1 à 9 atomes de carbone ou un groupe alcoxy ayant 1 à 4 atomes de carbone,

$R^{9'}$ et $R^{10'}$ représentent chacun, indépendamment, un atome d'hydrogène ou d'halogène, un groupe alkyle ayant 1 à 9 atomes de carbone, un groupe alcoxy ayant 1 à 4 atomes de carbone, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe hydroxyle, un groupe nitro ou un groupe cyano,

$R^{11'}$ représente un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

$R^{12'}$, $R^{13'}$, $R^{14'}$, $R^{15'}$ et $R^{16'}$ représentent chacun, indépendamment, un atome d'hydrogène, un groupe alkyle ayant 1 à 9 atomes de carbone ou un groupe alcoxy ayant 1 à 4 atomes de carbone,

$R^{17'}$ et $R^{18'}$ représentent chacun, indépendamment, un atome d'hydrogène ou d'halogène, un groupe alkyle ayant 1 à 9 atomes de carbone, un groupe alcoxy ayant 1 à 4 atomes de carbone, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe hydroxyle, un groupe nitro ou un groupe cyano,

$R^2$, $R^3$ et $R^4$ représentent chacun, indépendamment, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

et en outre $R^2$ peut représenter un atome d'hydrogène, et $R^3$ et $R^4$ peuvent former ensemble un groupe de formule :

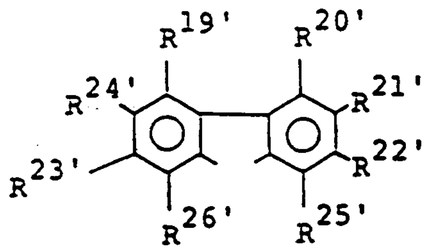

dans laquelle $R^{19'}$, $R^{20'}$, $R^{21'}$, $R^{22'}$, $R^{23'}$ et $R^{24'}$ représentent chacun, indépendamment, un atome d'hydrogène, un groupe alkyle ayant 1 à 9 atomes de carbone ou un groupe alcoxy ayant 1 à 4 atomes de carbone,

$R^{25'}$ et $R^{26'}$ représentent chacun, indépendamment, un atome d'hydrogène, un groupe alkyle ayant 1 à 9 atomes de carbone, un groupe alcoxy ayant 1 à 4 atomes de carbone, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe hydroxyle, un groupe nitro ou un groupe cyano, ou bien

(b) $R^1$ est un groupe de formule :

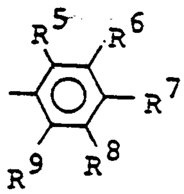

dans laquelle $R^5$ , $R^6$ , $R^7$, $R^8$ et $R^9$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle, non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy, à chaîne droite ou ramifiée, ayant 1 à 4 atomes de carbone, un groupe aryloxy non substitué ou substitué, ledit groupe aryloxy substitué comprenant les groupes aryloxy substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, un groupe amino substitué par un ou deux groupes alkyle ayant 1 à 9 atomes de carbone, à chaîne droite ou ramifiée, non substitués ou substitués, lesdits groupes alkyle substitués comprenant les groupes alkyle substitués par un ou plusieurs substituants pris parmi les atomes d'halogène et les groupes hydroxyle, cyano', nitro, alkylamino, arylamino, aryle et aryloxy, un groupe amino substitué par un ou deux groupes alkyle non substitués ou substitués, lesdits groupes aralkyle substitués comprenant les groupes aralkyle substitués par un ou plusieurs substituants choisis parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes aryle non substitués ou substitués, lesdits groupes aryle substitués comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, un

groupe amino substitué par un ou deux groupes hétérocycliques non substitués ou substitués, lesdits groupes hétérocycliques substitués comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano,

$R^2$ représente un atome d'hydrogène, un groupe alkyle ayant 1 à 9 atomes de carbone, un groupe aryle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, un groupe aralkyle pouvant porter un ou plusieurs substituants choisis parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique,

et $R^3$ et $R^4$ forment ensemble un groupe de formule :

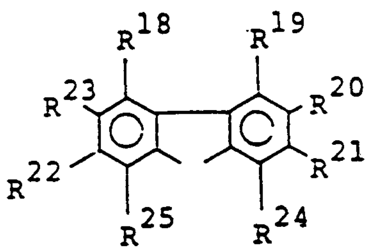

(II)

dans laquelle $R^{18}$, $R^{19}$, $R^{20}$, $R^{21}$, $R^{22}$ et $R^{23}$ représentent chacun, indépendamment, un atome d'hydrogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, ou un groupe alcoxy ayant 1 à 4 atomes de carbone,

$R^{24}$ et $R^{25}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino, et aralkylamino, un groupe alcoxy ayant 1 à 4 atomes de carbone, un groupe hydroxyle, un groupe nitro ou un groupe cyano,

procédé qui comprend les étapes consistant à faire réagir un composé de formule :

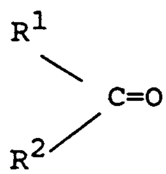

(III)

dans laquelle $R^1$ et $R^2$ sont tels que définis précédemment, avec un composé de formule :

$$\overset{+}{Ph_3P}\ \overset{-}{Cl}CH_2OCH_3 \qquad (IV\text{-}1)$$

dans laquelle Ph représente un groupe phényle, dans un solvant, en présence d'un alcoolate ou d'un dérivé organométallique, pour obtenir un composé de formule :

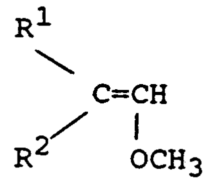

(V)

dans laquelle $R^1$ et $R^2$ sont tels que définis précédemment, à hydrolyser le composé de formule (V) avec un acide, puis à effectuer un traitement par un alcali, pour obtenir un composé de formule :

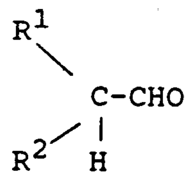

(VI)

dans laquelle $R^1$ et $R^2$ sont tels que définis précédemment, et à faire réagir le composé de formule (VI) avec un composé de formule :

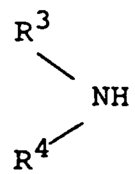

(VII)

dans laquelle $R^3$ et $R^4$ sont tels que définis précédemment, dans un solvant, en présence d'un acide sulfonique.

5. Procédé de préparation d'un dérivé énamine de formule :

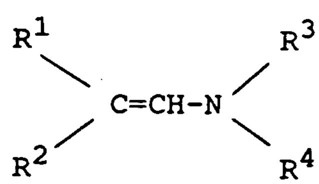

(I-1)

dans laquelle $R^1$ à $R^4$ sont tels que définis dans la revendication 4, qui comprend les étapes consistant à réduire un composé de formule :

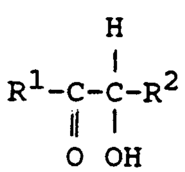

(VIII)

dans laquelle $R^1$ et $R^2$ sont tels que définis précédemment, avec un hydrure métallique, puis à effectuer un traitement par un acide et un traitement par un alcali, pour obtenir un composé de formule :

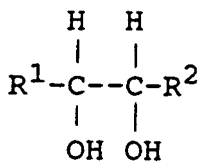

(IX)

dans laquelle $R^1$ et $R^2$ sont tels que définis précédemment, à faire réagir le composé de formule (IX) avec un acide pour obtenir un composé de formule :

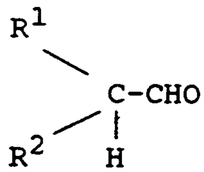

(VI)

dans laquelle $R^1$ et $R^2$ sont tels que définis précédemment, et à faire réagir le composé de formule (VI) avec un composé de formule :

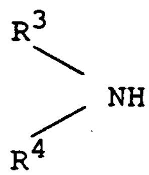

(VII)

dans laquelle $R^3$ et $R^4$ sont tels que définis précédemment, dans un solvant, en présence d'un acide sulfonique.

**6.** Procédé de préparation d'un dérivé énamine de formule :

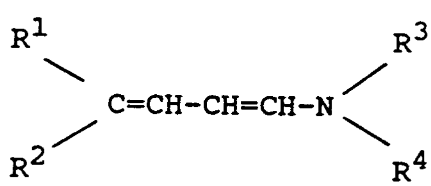

(I-2)

dans laquelle $R^1$ est un groupe de formule :

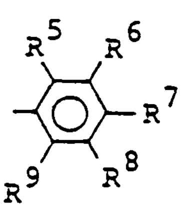

ou

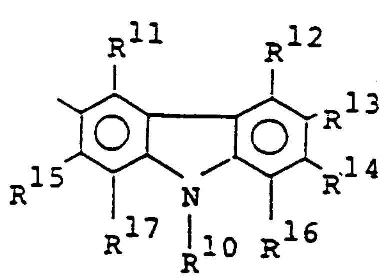

dans laquelle $R^5$, $R^6$, $R^7$, $R^8$ et $R^9$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino,

arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, à chaîne droite ou ramifiée, un groupe aryloxy non substitué ou substitué, ledit groupe aryloxy substitué comprenant les groupes aryloxy substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes alkyle ayant 1 à 9 atomes de carbone, à chaîne droite ou ramifiée, non substitués ou substitués, lesdits groupes alkyle substitués comprenant les groupes alkyle substitués par un ou plusieurs substituants pris parmi les atomes d'halogène et les groupes hydroxyle, cyano, nitro, alkylamino, arylamino, aryle et aryloxy, un groupe amino substitué par un ou deux groupes aralkyle non substitués ou substitués, lesdits groupes aralkyle substitués comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes aryle non substitués ou substitués, lesdits groupes aryle substitués comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes hétérocycliques non substitués ou substitués, lesdits groupes hétérocycliques substitués comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano,

$R^{10}$ représente un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle, et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

$R^{11}$, $R^{12}$, $R^{13}$, $R^{14}$ et $R^{15}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe alcoxy ayant 1 à 4 atomes de carbone, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aryle non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

$R^{16}$ et $R^{17}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les

groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,

$R^2$, $R^3$ et $R^4$ représentent chacun, indépendamment, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

et en outre $R^2$ peut être un atome d'hydrogène, et $R^3$ et $R^4$ peuvent former ensemble un groupe de formule :

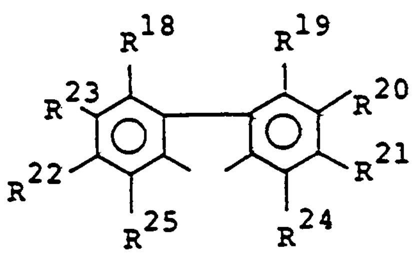

(II)

dans laquelle $R^{18}$, $R^{19}$, $R^{20}$, $R^{21}$, $R^{22}$ et $R^{23}$ représentent chacun, indépendamment, un atome d'hydrogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, ou un groupe alcoxy ayant 1 à 4 atomes de carbone,

et $R^{24}$ et $R^{25}$ représentent chacun, indépendamment, un atome d'hydrogène ou d'oxygène, un groupe alkyle ayant 1 à 9 atomes de carbone, à chaîne droite ou ramifiée, non substitué ou substitué, ledit groupe alkyle substitué comprenant les groupes alkyle substitues par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,

procédé qui comprend les étapes consistant à faire réagir un composé de formule :

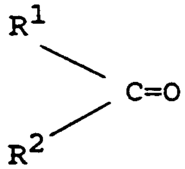

(III)

dans laquelle $R^1$ et $R^2$ sont tels que définis précédemment, avec un composé de formule :

$$Ph_3\overset{+}{P}\ \overset{-}{I}CH_3 \qquad (IV-2)$$

dans laquelle Ph représente un groupe phényle, dans un solvant, en présence d'un alcoolate ou d'un dérivé organométallique, pour obtenir un composé de formule :

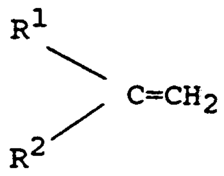

(X)

dans laquelle $R^1$ et $R^2$ sont tels que définis précédemment, à réaliser la formylation du composé de formule (X) dans le diméthylformamide, en présence de $POCl_3$, pour obtenir un composé de formule :

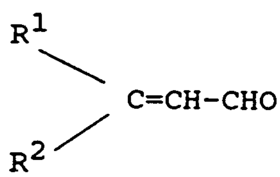

(XI)

dans laquelle $R^1$ et $R^2$ sont tels que définis précédemment, à faire réagir le composé de formule (XI) avec un composé de formule :

$$Ph_3\overset{+}{P}\ \overset{-}{Cl}CH_2OCH_3 \qquad (IV-1)$$

dans laquelle Ph représente un groupe phényle, dans un solvant, en présence d'un alcoolate ou d'un dérivé organométallique, pour obtenir un composé de formule :

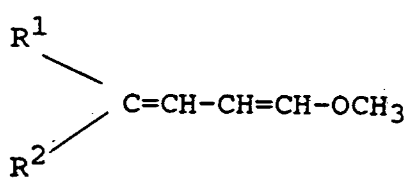

(XII)

dans laquelle $R^1$ et $R^2$ sont tels que définis précédemment, à hydrolyser le composé de formule (XII) avec un acide, puis à effectuer un traitement par un alcali, pour obtenir un composé de formule :

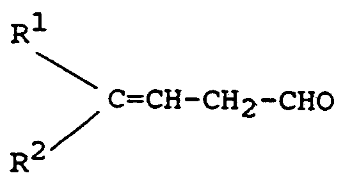

(XIII)

dans laquelle $R^1$ et $R^2$ sont tels que définis précédemment, et à faire réagir le composé de formule (XIII) avec un composé de formule :

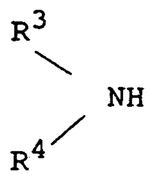

(VII)

dans laquelle $R^3$ et $R^4$ sont tels que définis précédemment, dans un solvant, en présence d'un acide sulfonique.

7. Procédé de préparation d'un dérivé énamine de formule :

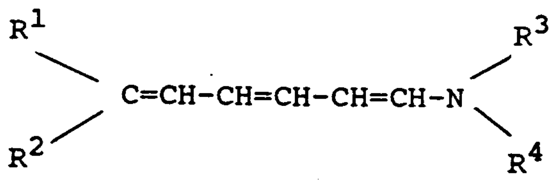

(I-3)

dans laquelle $R^1$ est un groupe de formule :

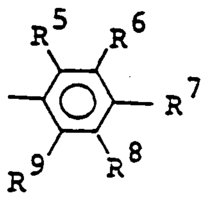

ou

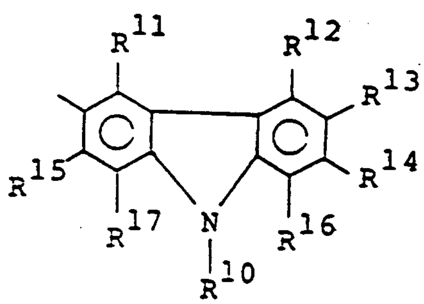

dans laquelle $R^5$, $R^6$, $R^7$, $R^8$ et $R^9$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, à chaîne droite ou ramifiée, un groupe aryloxy non substitué ou substitué, ledit groupe aryloxy substitué comprenant les groupes aryloxy substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes alkyle ayant 1 à 9 atomes de carbone, à chaîne droite ou ramifiée, non substitués ou substitués, lesdits groupes alkyle substitués comprenant les groupes alkyle substitués par un ou plusieurs substituants pris parmi les atomes d'halogène et les groupes hydroxyle, cyano, nitro, alkylamino, arylamino, aryle et aryloxy, un groupe amino substitué par un ou deux groupes aralkyle non substitués ou substitués, lesdits groupes aralkyle substitués comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes aryle non substitués ou substitués, lesdits groupes aryle substitués comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe amino substitué par un ou deux groupes hétérocycliques non substitués ou substitués, lesdits groupes hétérocycliques substitués comprenant les groupes hétérocycliques substitués par un ou plusieurs substituants pris parmi les atomes

d'halogène et les groupes hydroxyle, cyano, nitro, alkylamino, amino, alkyle, alcoxy, acétyle, acétoxy, aryle, aryloxy, arylamino et aralkylamino, un groupe hydroxyle, un groupe nitro ou un groupe cyano,

$R^{10}$ représente un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aryle non substitué ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle, et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

$R^{11}$, $R^{12}$, $R^{13}$, $R^{14}$ et $R^{15}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe alcoxy ayant 1 à 4 atomes de carbone, un groupe aralkyle non substitué ou substitué, un groupe aryle non substitué Ou substitué, ledit groupe aryle substitué comprenant les groupes aryle substitués par un ou plusieurs substituants pris parmi les atomes d'halogène et les groupes hydroxyle, cyano, nitro, alkylamino, amino, alkyle, alcoxy, acétyle, acétoxy, arylamino, aralkylamino, aryle et aryloxy, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

$R^{16}$ et $R^{17}$ représentent chacun, indépendamment, un atome d'hydrogène ou d'halogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,

$R^2$, $R^3$ et $R^4$ représentent chacun, indépendamment, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, ou un groupe hétérocyclique non substitué ou substitué, ledit groupe hétérocyclique substitué comprenant les groupes hétérocycliques substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino,

et en outre $R^2$ peut être un atome d'hydrogène, et $R^3$ et $R^4$ peuvent former ensemble un groupe de formule :

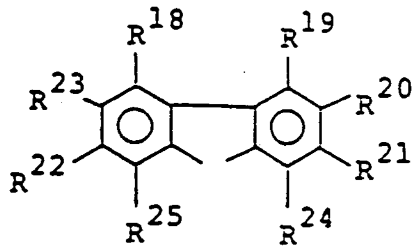

(II)

dans laquelle $R^{18}$, $R^{19}$, $R^{20}$, $R^{21}$, $R^{22}$ et $R^{23}$ représentent chacun, indépendamment, un atome d'hydrogène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, ou un groupe alcoxy ayant 1 à 4 atomes de carbone,
et $R^{24}$ et $R^{25}$ représentent chacun, indépendamment, un atome d'hydrogène ou d'oxygène, un groupe alkyle ayant 1 à 9 atomes de carbone, à chaîne droite ou ramifiée, non substitué ou substitué, ledit groupe alkyle substitué comprenant les groupes alkyle substitués par un ou plusieurs substituants pris parmi les atomes d'halogène et les groupes hydroxyle, cyano, nitro, alkylamino, arylamino, aryle et aryloxy, un groupe aralkyle non substitué ou substitué, ledit groupe aralkyle substitué comprenant les groupes aralkyle substitués par un ou plusieurs substituants pris parmi les atomes d'halogène et les groupes hydroxyle, cyano, nitro, alkylamino, amino, alkyle, alcoxy, acétyle, acétoxy, aryle, aryloxy, arylamino et aralkylamino, un groupe alcoxy ayant 1 à 4 atomes de carbone, un groupe hydroxyle, un groupe nitro ou un groupe cyano,
procédé qui comprend les étapes consistant à faire réagir un composé de formule :

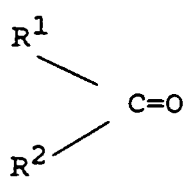

(III)

dans laquelle $R^1$ et $R^2$ sont tels que définis précédemment, avec un composé de formule :

$$Ph_3\overset{+}{P}\ \overset{-}{I}CH_3 \qquad \text{(IV-2)}$$

dans laquelle Ph représente un groupe phényle, dans un solvant, en présence d'un alcoolate ou d'un dérivé organométallique, pour obtenir un composé de formule :

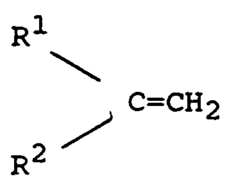

(X)

dans laquelle $R^1$ et $R^2$ sont tels que définis précédemment, à réaliser la formylation du composé de formule (X) dans le diméthylformamide, en présence de $POCl_3$, pour obtenir un composé de formule :

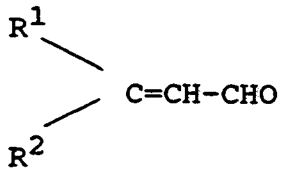

(XI)

dans laquelle $R^1$ et $R^2$ sont tels que définis précédemment, à faire réagir le composé de formule (XI) avec un composé de formule (IV-2), dans un solvant, en présence d'un alcoolate ou d'un dérivé organo-métallique, pour obtenir un composé de formule :

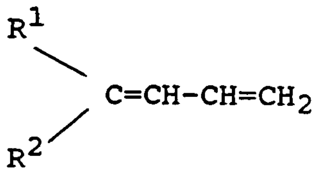

(XIV)

dans laquelle $R^1$ et $R^2$ sont tels que définis précédemment, à effectuer la formylation du composé de formule (XIV) dans du diméthylformamide, en présence de $POCl_3$, pour obtenir un composé de formule :

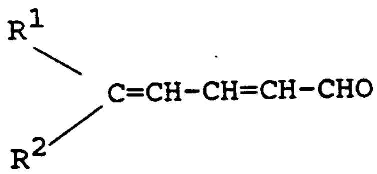

(XV)

dans laquelle $R^1$ et $R^2$ sont tels que définis précédemment, à faire réagir le composé de formule (XV) avec un composé de formule :

$$Ph_3\overset{+}{P}\ \overset{-}{Cl}CH_2OCH_3 \qquad (IV\text{-}1)$$

dans laquelle Ph représente un groupe phényle, dans un solvant, en présence d'un alcoolate ou d'un dérivé organométallique, pour obtenir un composé de formule :

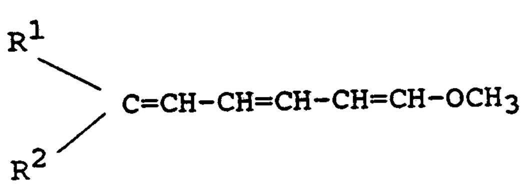

(XVI)

dans laquelle $R^1$ et $R^2$ sont tels que définis précédemment, à hydrolyser le composé de formule (XVI) avec un acide, puis à effectuer un traitement par un alcali, pour obtenir un composé de formule :

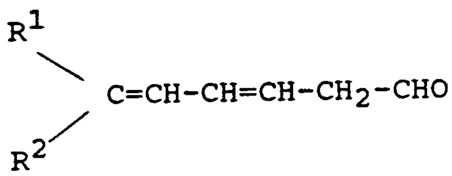

(XVII)

dans laquelle $R^1$ et $R^2$ sont tels que définis précédemment, et à faire réagir le composé de formule (XVII) avec un composé de formule :

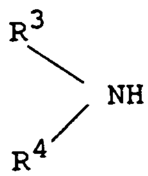

(VII)

dans laquelle $R^3$ et $R^4$ sont tels que définis précédemment, dans un solvant, en présence d'un acide sulfonique.

**8.** Plaque pour électrophotographie, dans laquelle le dérivé énamine selon la revendication 1 est utilisé en tant que produit de transport de charges.

**9.** Plaque pour électrophotographie, dans laquelle le dérivé énamine selon la revendication 2 est utilisé en tant que produit de transport de charges.

**10.** Plaque pour électrophotographie, dans laquelle le dérivé énamine selon la revendication 3 est utilisé en tant que produit de transport de charges.

FIG. 1

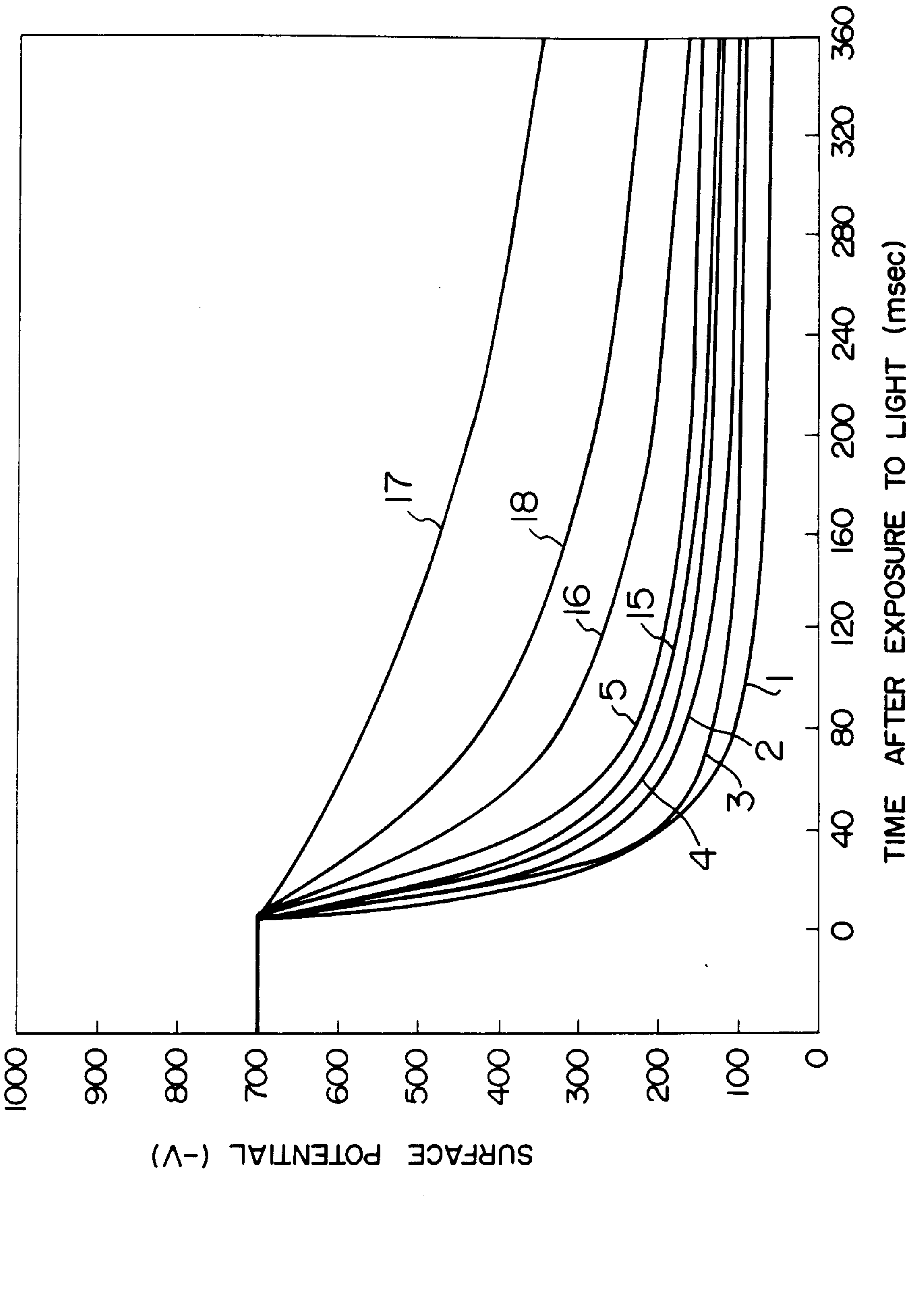
SURFACE POTENTIAL (-V)
1000
900
800
700
600
500
400
300
200
100
0
TIME AFTER EXPOSURE TO LIGHT (msec)
0
40
80
120
160
200
240
280
320
360
17
18
16
15
5
4
3
2
1

F I G. 2

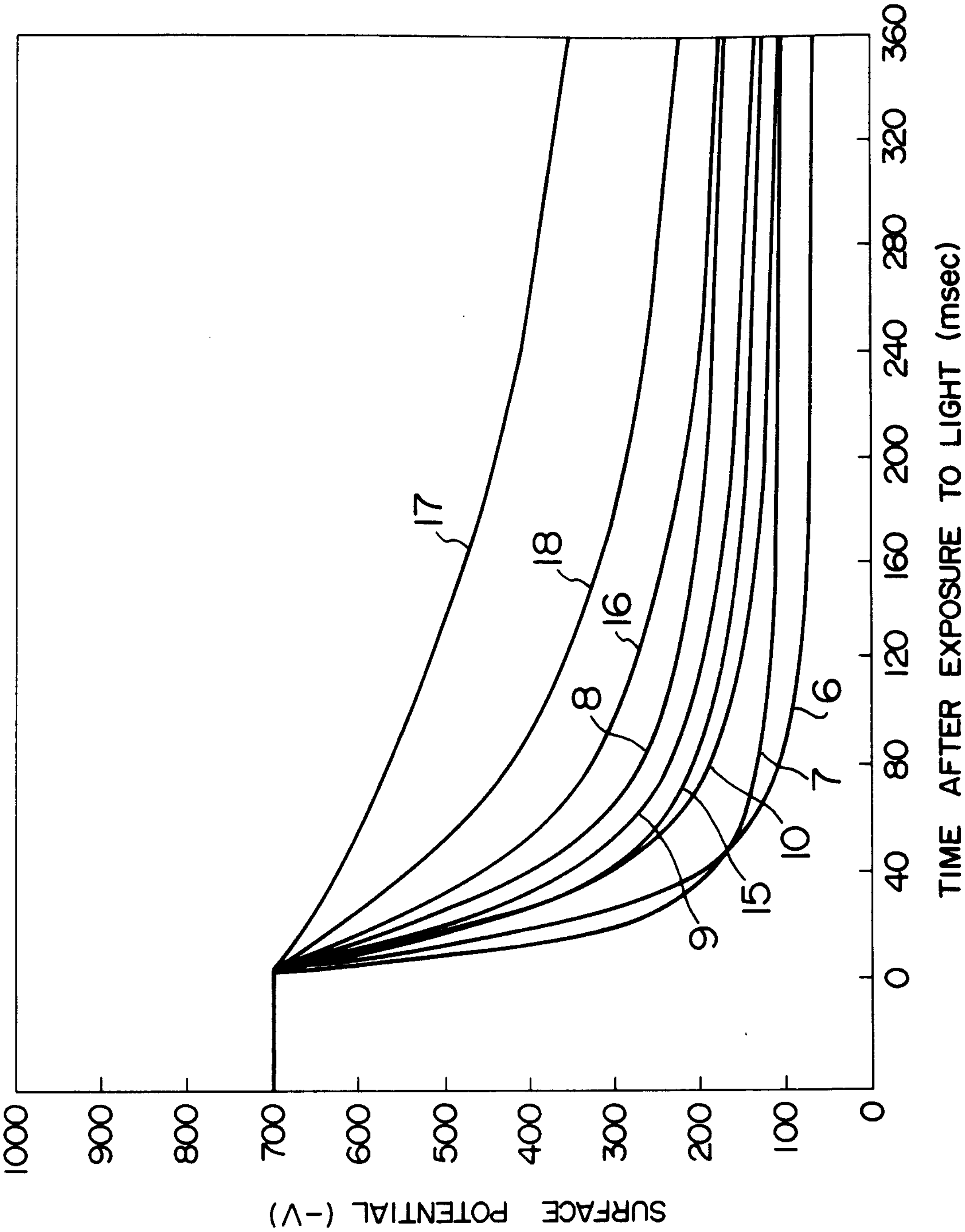
SURFACE POTENTIAL (-V)
1000
900
800
700
600
500
400
300
200
100
0
TIME AFTER EXPOSURE TO LIGHT (msec)
0
40
80
120
160
200
240
280
320
360
17
18
16
8
9
15
10
7
6

FIG. 3

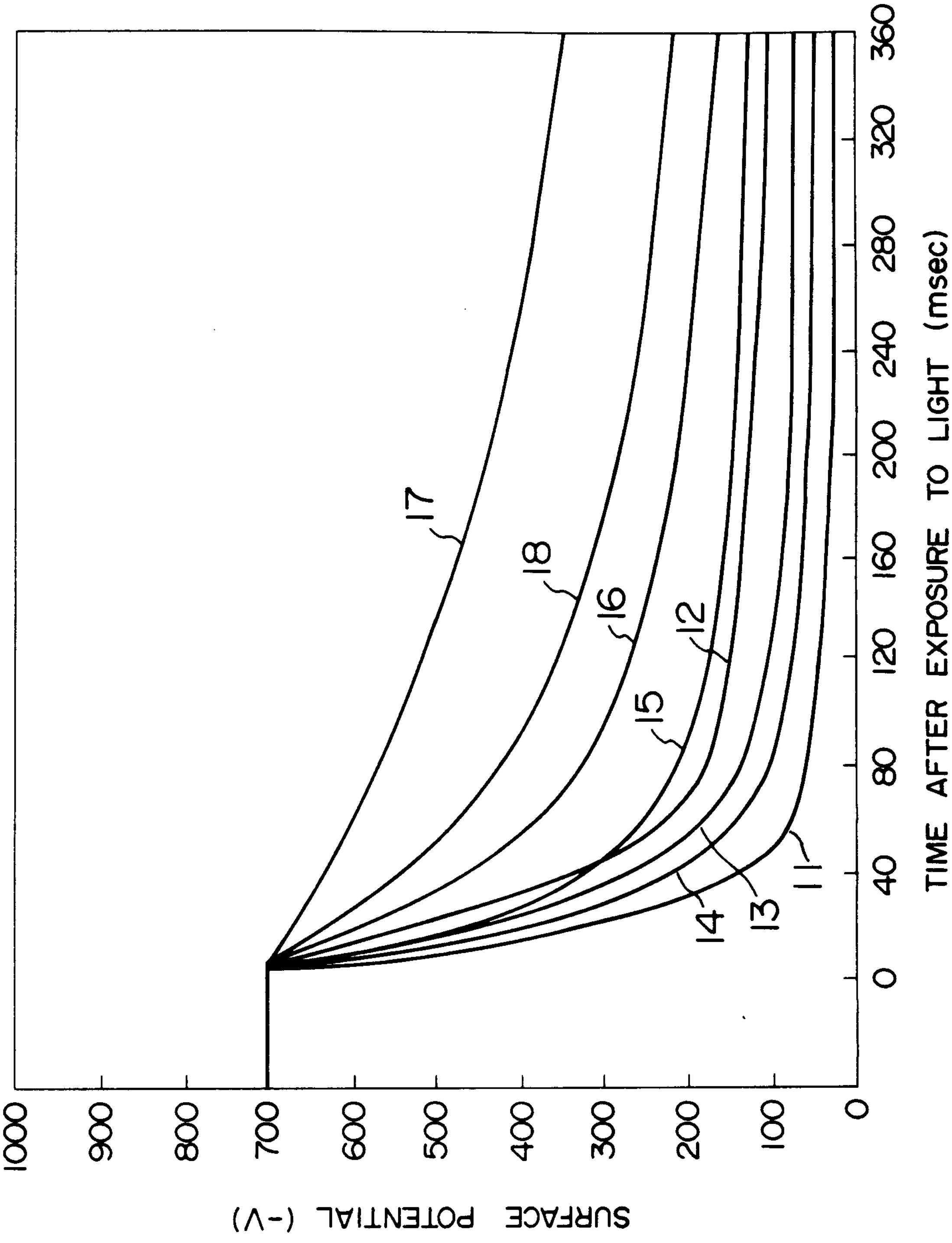
SURFACE POTENTIAL (-V)
1000
900
800
700
600
500
400
300
200
100
0
TIME AFTER EXPOSURE TO LIGHT (msec)
0
40
80
120
160
200
240
280
320
360
11
12
13
14
15
16
17
18

FIG. 4

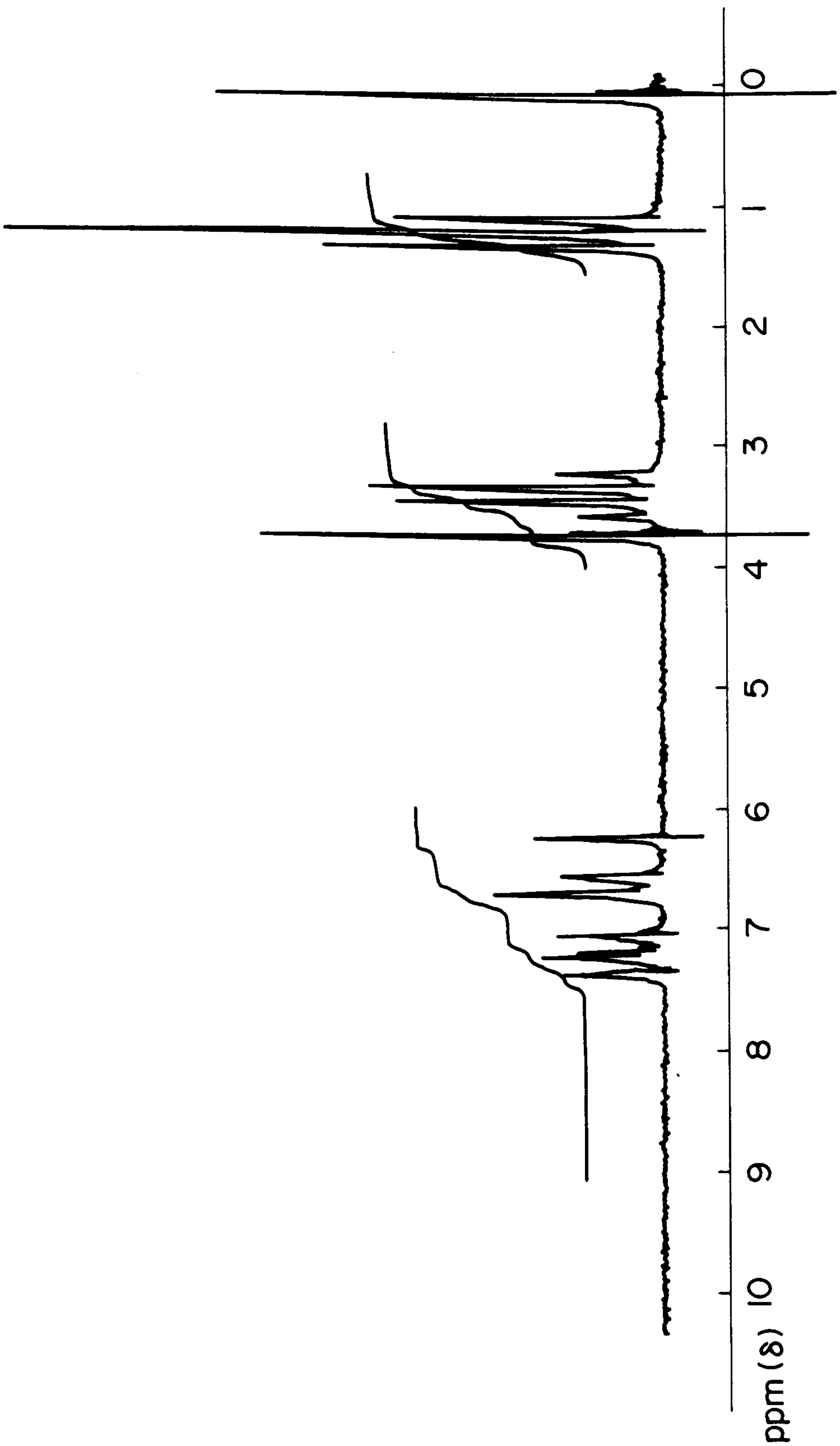

FIG. 5

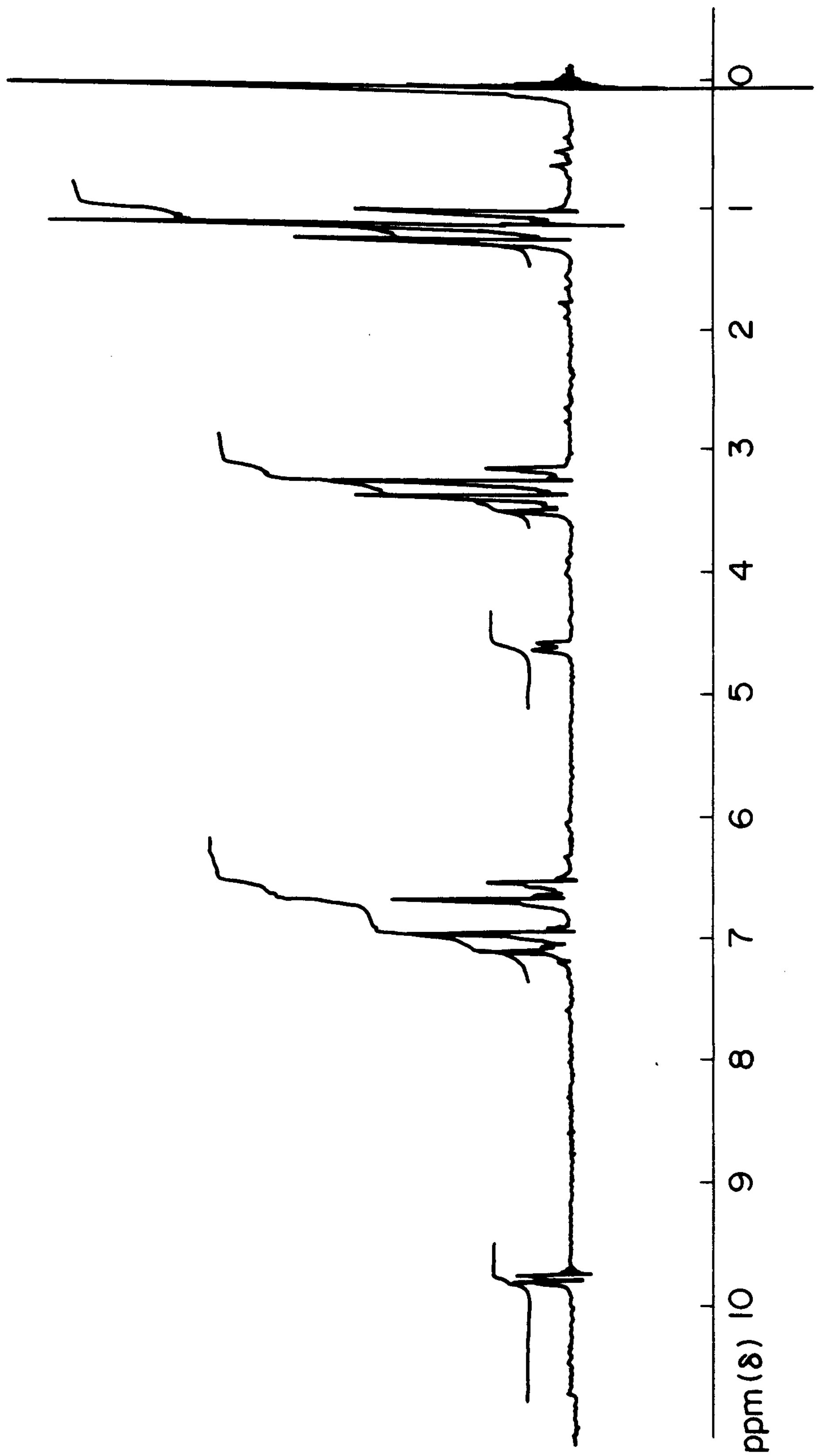

FIG. 6

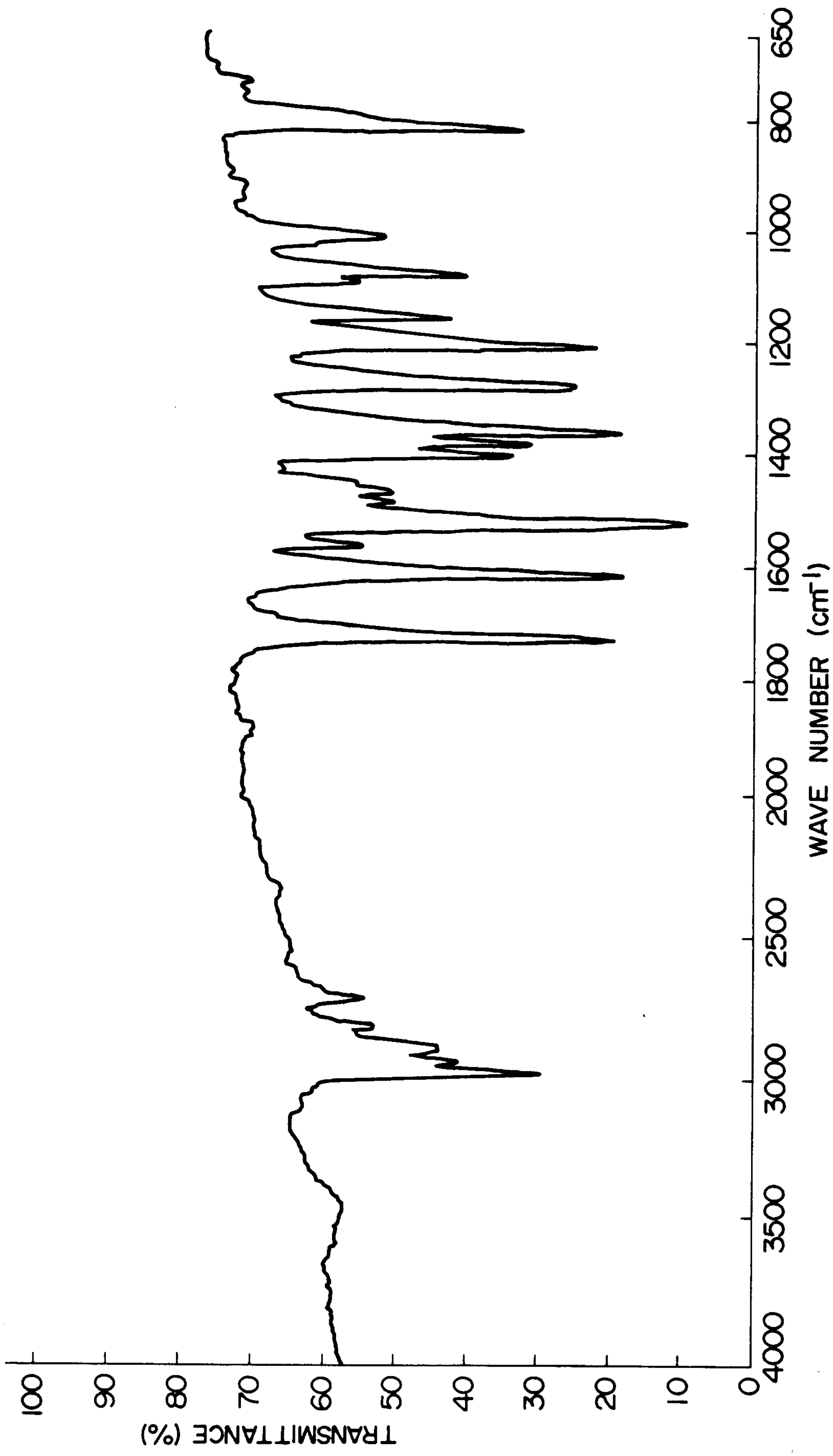
TRANSMITTANCE (%)
0
10
20
30
40
50
60
70
80
90
100
WAVE NUMBER ($cm^{-1}$)
4000
3500
3000
2500
2000
1800
1600
1400
1200
1000
800
650

FIG. 7

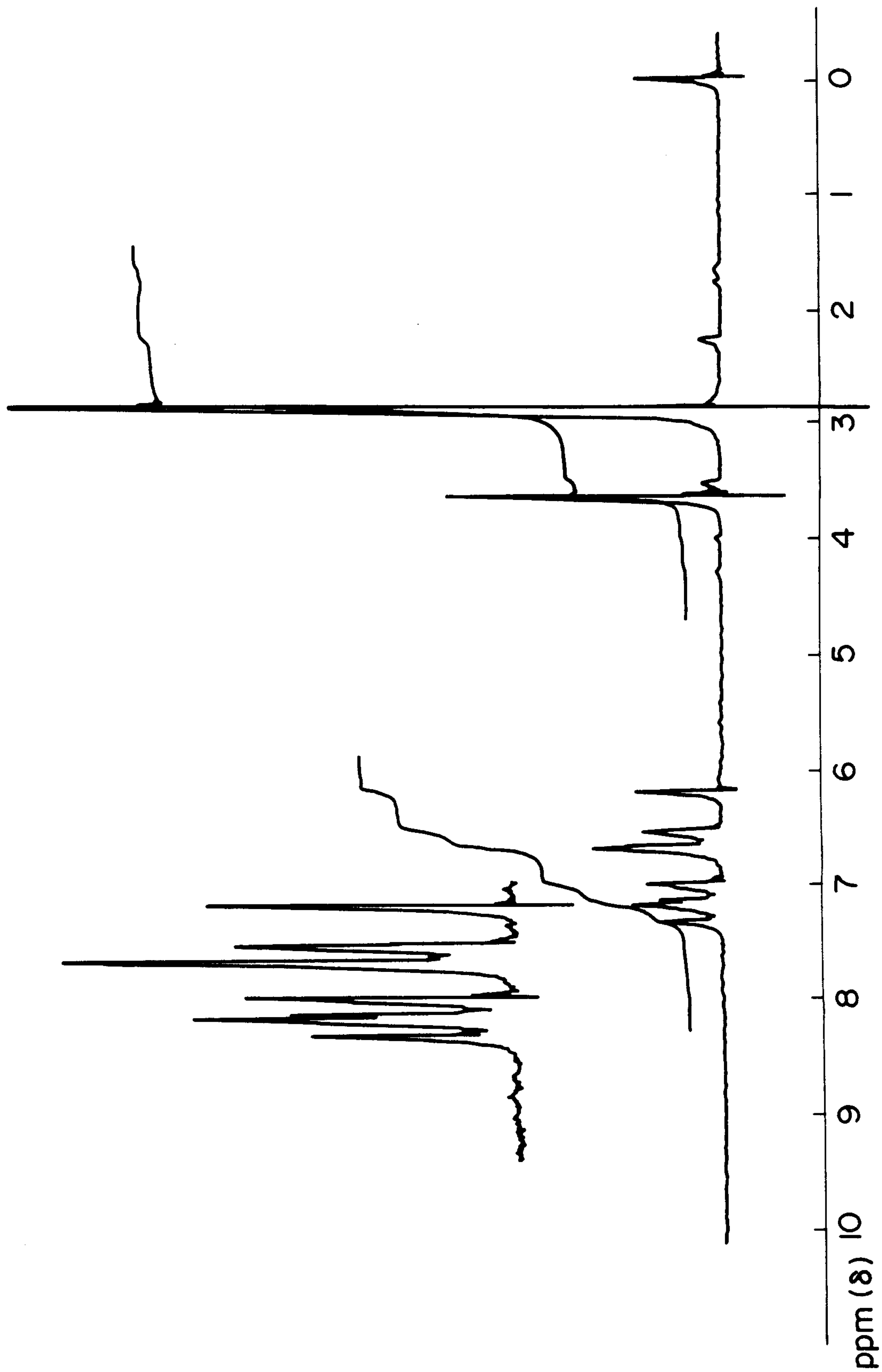

FIG. 8

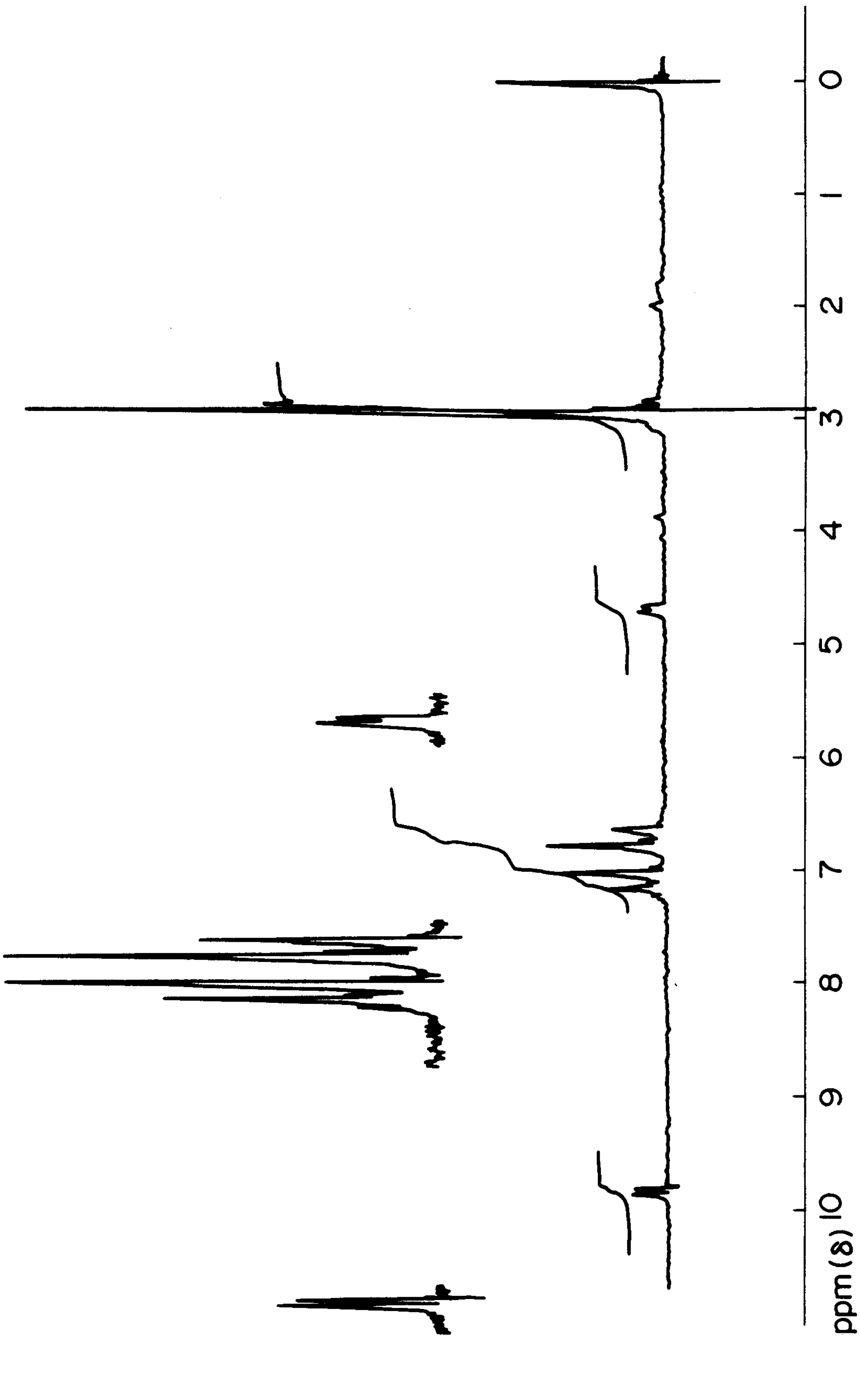

FIG. 9

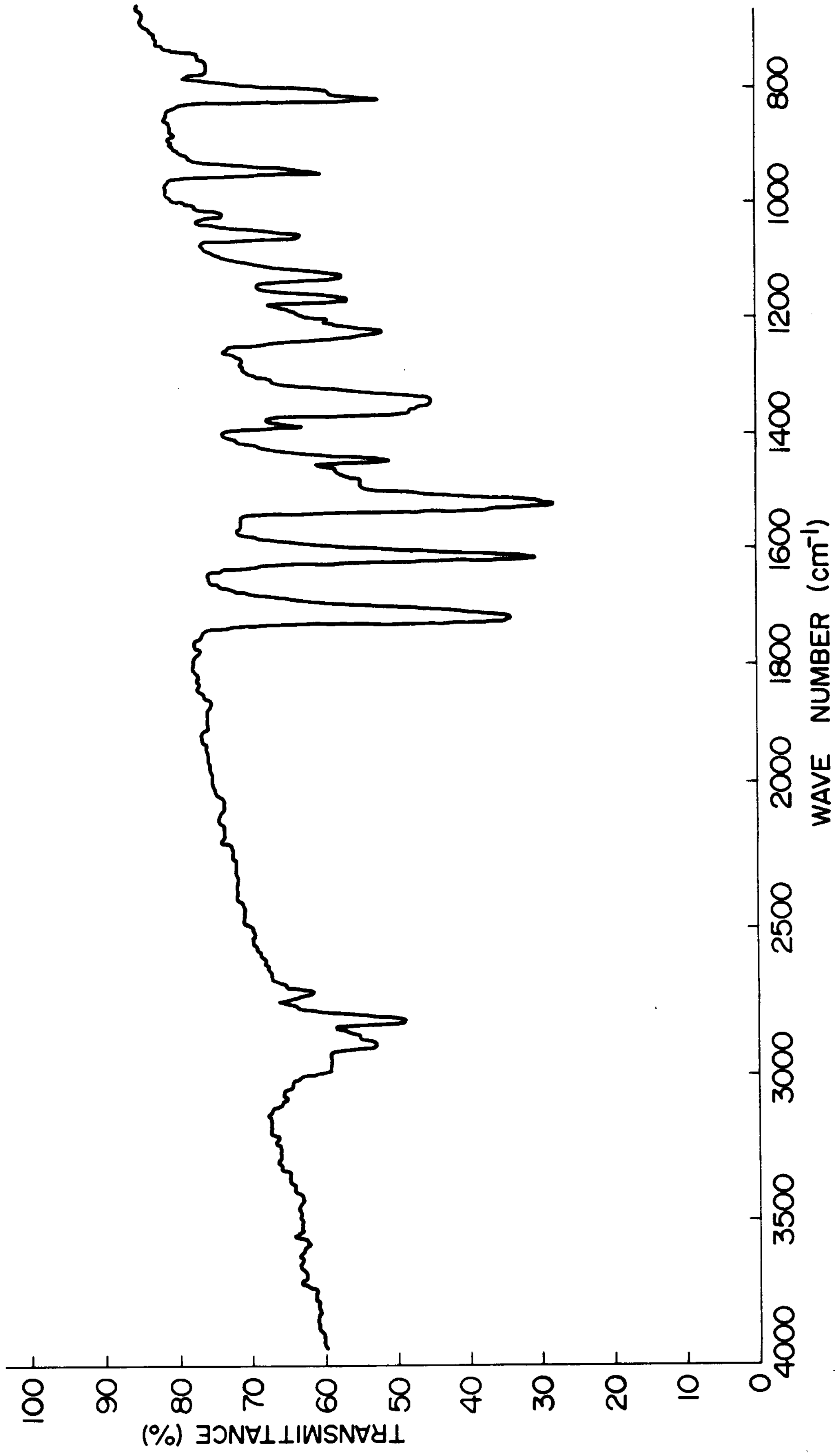
TRANSMITTANCE (%)
0
10
20
30
40
50
60
70
80
90
100
4000
3500
3000
2500
2000
1800
1600
1400
1200
1000
800
WAVE NUMBER (cm⁻¹)

FIG. 10

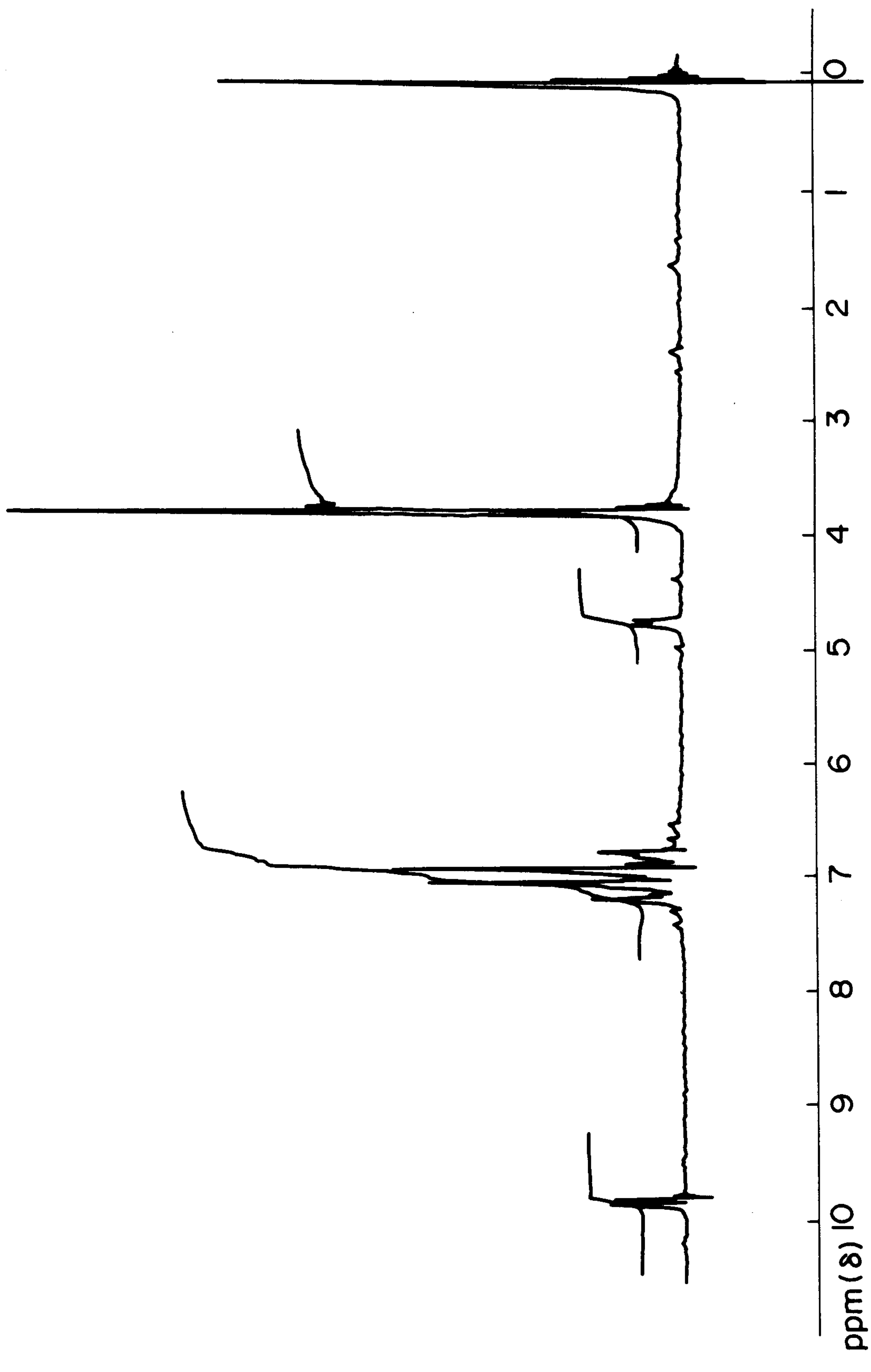

FIG. 11

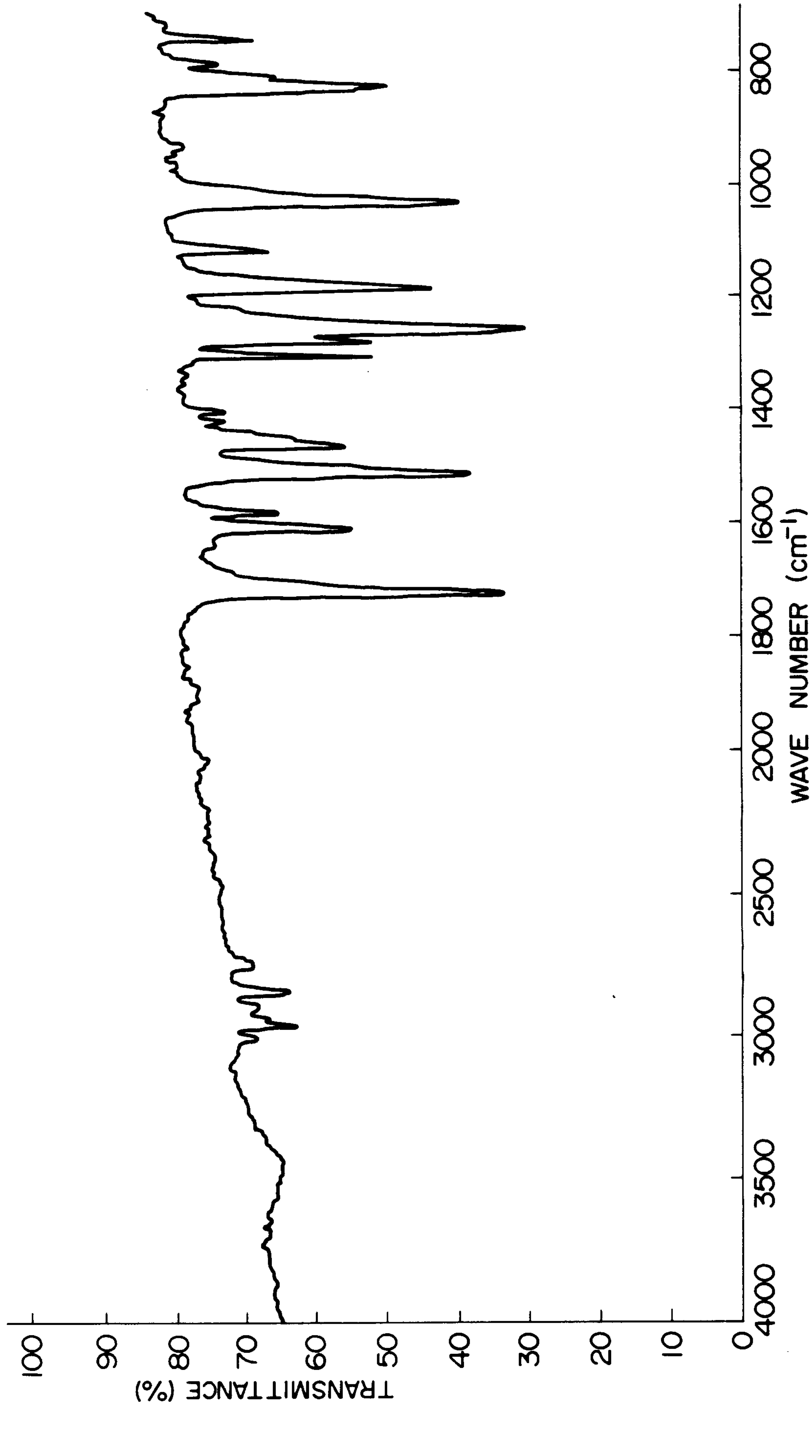
TRANSMITTANCE (%)
100
90
80
70
60
50
40
30
20
10
0
4000
3500
3000
2500
2000
1800
1600
1400
1200
1000
800
WAVE NUMBER (cm⁻¹)

FIG. 12

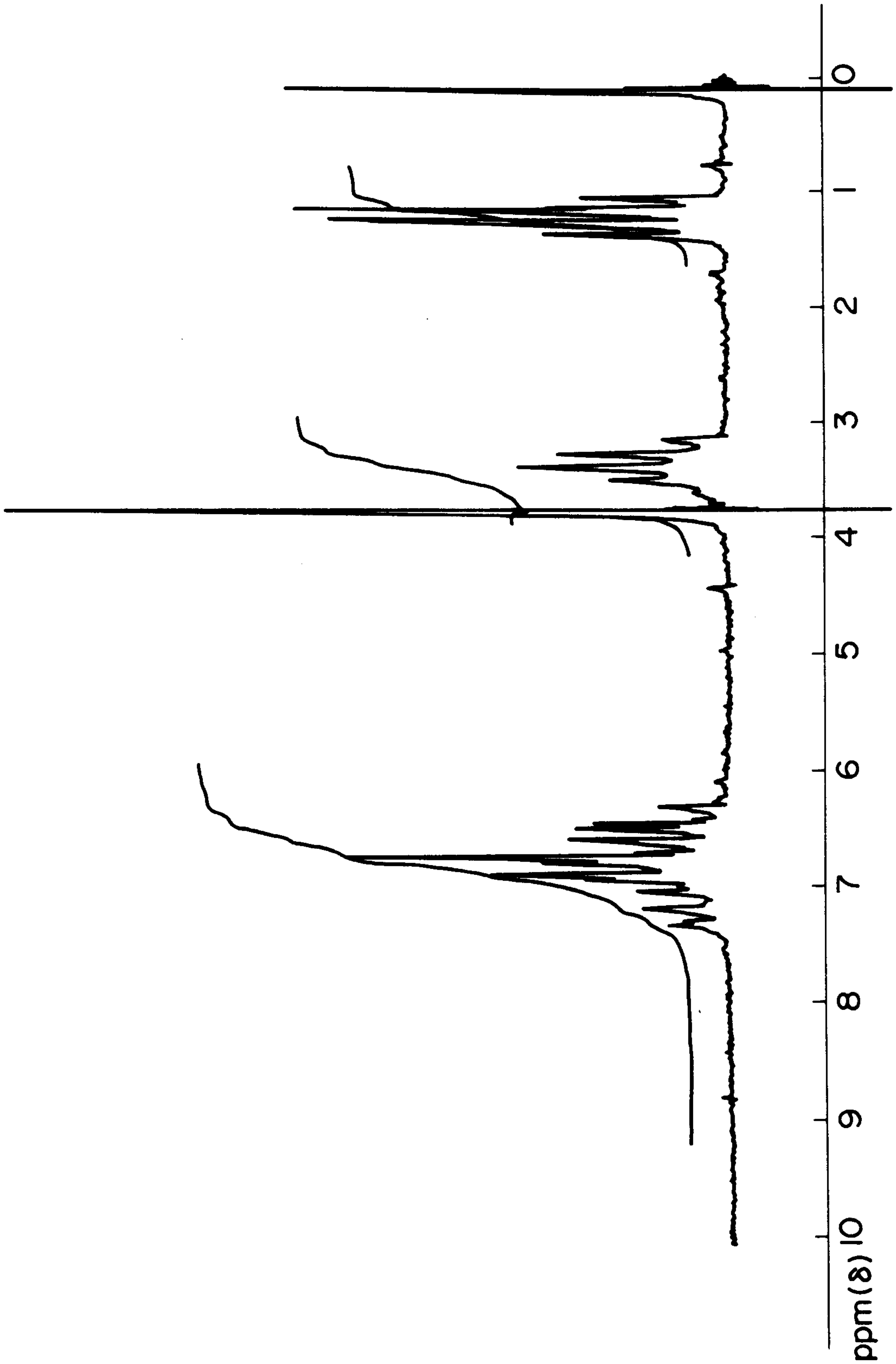

FIG. 13

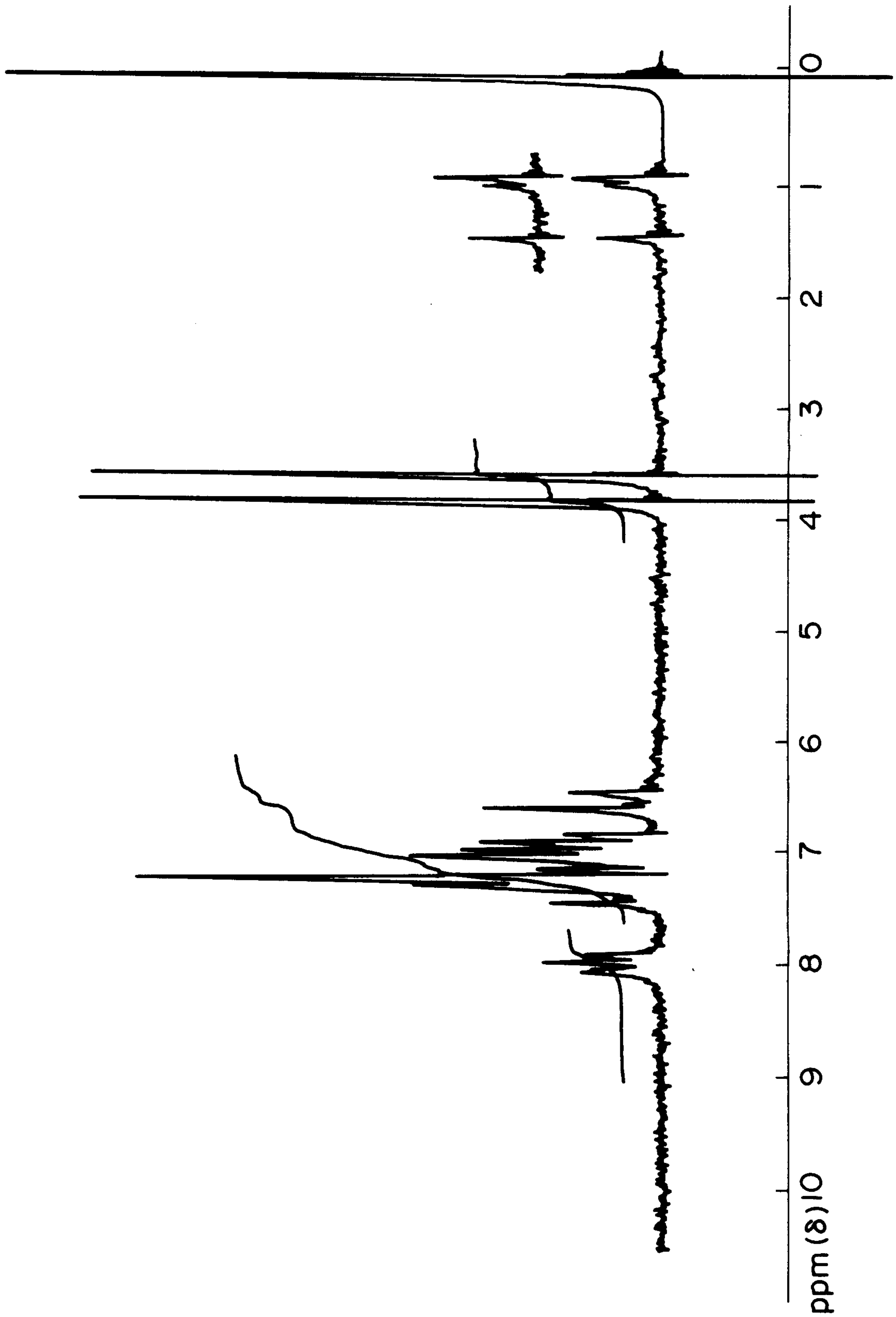

FIG. 14

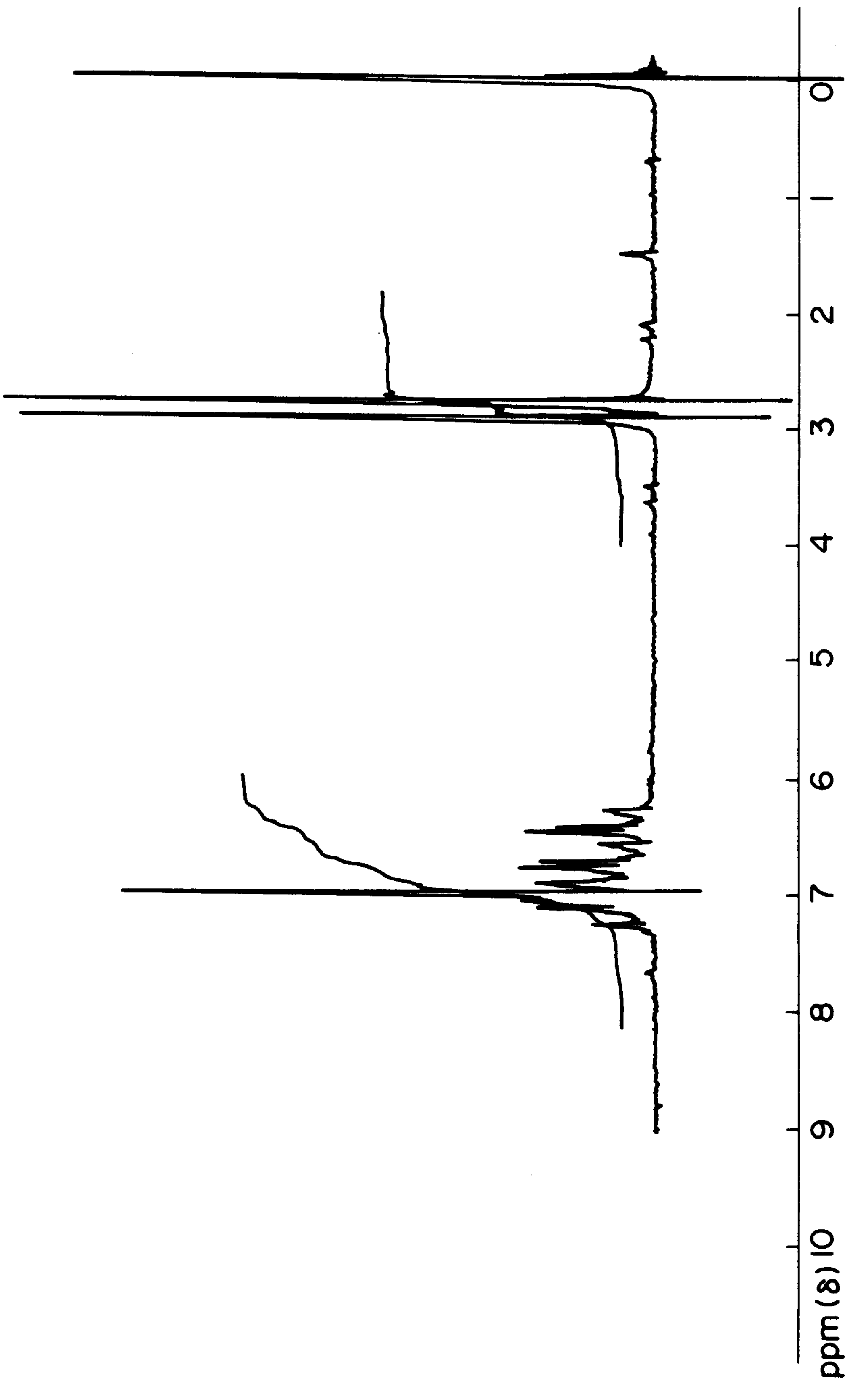

FIG. 15